(19) Europäisches Patentamt
European Patent Office
Office européen des brevets

(11) **EP 3 650 005 A1**

(12) **EUROPEAN PATENT APPLICATION**
published in accordance with Art. 153(4) EPC

(43) Date of publication:
**13.05.2020 Bulletin 2020/20**

(21) Application number: **18828760.1**

(22) Date of filing: **05.07.2018**

(51) Int Cl.:
***A61K 8/06*** *(2006.01)* ***A61K 8/81*** *(2006.01)*
***A61Q 1/00*** *(2006.01)* ***A61Q 19/00*** *(2006.01)*

(86) International application number:
**PCT/JP2018/025576**

(87) International publication number:
**WO 2019/009372 (10.01.2019 Gazette 2019/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.07.2017 US 201762529251 P**

(71) Applicant: **Shiseido Company, Ltd.**
**Tokyo 104-0061 (JP)**

(72) Inventors:
- **SATO, Tomoko**
  **Yokohama-shi**
  **Kanagawa 224-8558 (JP)**
- **RODRIGUEZ, Ana Maria Bago**
  **Hull HU6 7RX (GB)**
- **BINKS, Bernard. P.**
  **Hull HU6 7RX (GB)**

(74) Representative: **Santarelli**
**49, avenue des Champs-Elysées**
**75008 Paris (FR)**

(54) **COSMETIC COMPOSITION**

(57) It is to provide a cosmetic composition comprising a coacervate droplet which can form a metastable water-in-water type emulsion.

A cosmetic composition of the present invention comprises a water phase as a matrix and an oil phase dispersed therein, and stabilized by a coacervate droplet of an anionic polyelectrolyte and a cationic polyelectrolyte, the coacervate droplet being one which can form a metastable water-in-water type emulsion.

FIG. 20

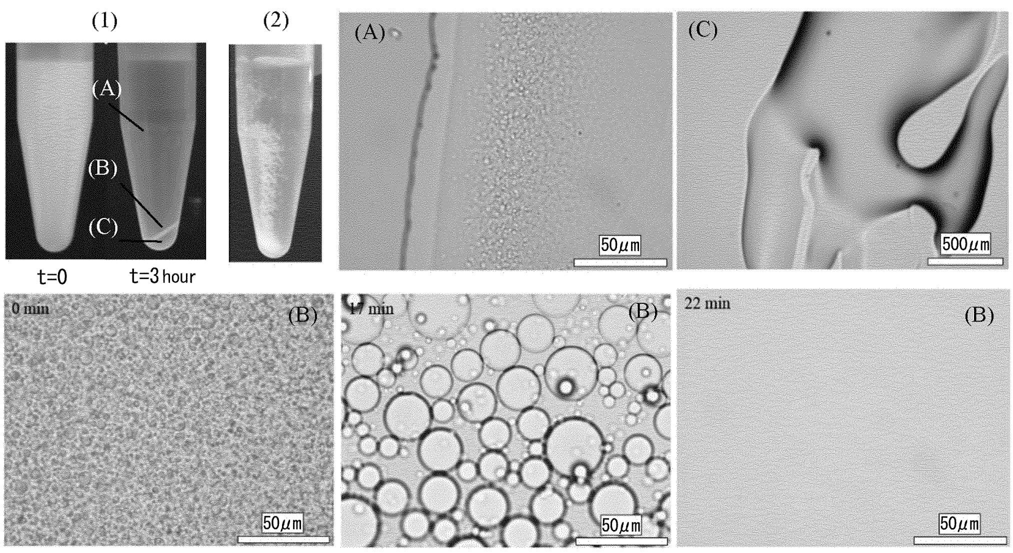


EP 3 650 005 A1

## Description

FIELD

The present invention relates to a cosmetic composition.

BACKGROUND

SECTION 1- LITERATURE SEARCH

1. PHASE SEPARATION IN POLYMER MIXTURES

A polymer can be defined as a macromolecule whose structure is composed by many repeating units (monomers) that chemically interact in a polymerization reaction. The mixtures between two or more polymers gained special attention many years ago in both academic and industrial laboratories due to its potential applications. When mixing two polymer solutions, different types of phase separation can occur which are depicted in the following chart.

If the polymers are incompatible (they repel each other), segregative phase separation takes place and each polymer is collected (predominantly) in a different phase. One example is the two phases formed by mixing dextran and methylcellulose in water (Figure 2) (NON-PTL 1). This is the most common scenario and it is consistent with the small entropy of mixing that results upon mixing two polymer solutions (NON-PTL 2). The second law of thermodynamics states that the entropy of an isolated system increases over time. As an amorphous polymer is already in its disordered state, mixing will not substantially increase the entropy of the system. Hence, mixing is disfavoured.

Complete miscibility occurs in exceptional cases and gives rise to a homogeneous solution. That is the case of the mixture between polystyrene and poly(vinyl methyl ether) (NON-PTL 3). For complete miscibility to occur, the following condition must be satisfied:

$$\Delta G_m = \Delta H_m - T\Delta S_m < 0 \qquad \cdots \text{ equation 1}$$

where $\Delta G_m$, $\Delta H_m$ and $\Delta S_m$ are the Gibb's free energy, the enthalpy and entropy of mixing at temperature T, respectively. As the value of $T\Delta S_m$ is always > 0 due to an increase of the entropy of mixing, this condition only fulfilles if the entropic contribution exceeds the enthalpic one.

Finally, if the polymers show net attraction, usually through electrostatic interactions, associative phase separation occurs. In this case, the two polymers are collected in one phase while the other phase consists almost entirely of solvent. This interaction can lead either to the formation of a complex coacervate (liquid-liquid type of phase separation) or a precipitate (solid-liquid type of phase separation). Both complexation mechanisms are driven by an increase of entropy due to the release of the counter-ions initially bound to the PEL backbone chain. Although the factors that dictate the nature of each type of associative phase separation are not fully elucidated, it is generally assumed that strong interactions between PELs originate precipitates while in coacervation the interactions are relatively weak (NON-PTLs 4 and 5). Other factors that play an important role in the phase separation of oppositely charged macromolecules are the ionic strength (salt concentration), pH, temperature, stoichiometry, molecular weight, charge density, chain flexibility and polyion concentration (NON-PTL 6).

The distinction between these two types of associative phase separation is not clear in the literature and generally the term polyelectrolyte complex (PEC) is adopted to describe both scenarios. However, optical microscopy is a simple and useful technique to distinguish between precipitation (amorphous solid particles) and complex coacervation (micro size droplets) (NON-PTL 4).

1.1. Coacervation: definition and applications

(a) Definition

The coacervation phenomena was first observed in 1911 by Tiebackx (NON-PTL 7), followed by Bungenberg de Jong (NON-PTL 8) who first systematically investigated the phase behaviour of the natural polymers gelatin (weakly positively charged protein) and gum Arabic (weakly negatively charged polysaccharide). In 1977, Oparin, a Russian Biochemist, popularized coacervates outside the colloid area as he proposed that life was first formed in coacervate droplets (NON-PTL 9). However, this idea has been already displaced by modern theories, particularly after the discovery of the DNA.

The term coacervation was coined by Bungenberg de Jong and Kruyt in 1929 and derives from Latin "co" (together) and "acerv" (a heap or aggregate) (NON-PTL 8). In coacervation a dense polymer-rich phase (coacervate phase) and a very dilute polymer-deficient phase (aqueous phase) coexist (NON-PTL 10). Coacervates are subdivided into "simple" and "complex" depending on the number of polymers that take part on the associative phase separation phenomena.

In simple coacervation one macromolecule is present and the associative process is induced by the addition of a dehydrating agent such as salts, alcohols or a change in the temperature or the pH of the medium. One example is the mixture of polyethylene glycol, potassium phosphate and water, in which the bottom phase is rich in salt and the top phase is rich in the polymer (NON-PTL 1).

On the other hand, in complex systems, two oppositely charged species are involved in an associative phase separation. As a result, two immiscible (and hence incompatible) liquid phases arise. The upper phase (supernatant) consists almost entirely of solvent and the bottom phase embody a dense clear liquid phase (coacervate) which is concentrated in macromolecules. Before reaching this steady state, a biphasic system formed by a metastable suspension of macroion-rich droplets (coacervate suspension) is present (NON-PTL 10). Coacervates hold higher amounts of water compared to precipitates. Jha et al. determined the weight fraction of water in the coacervate system containing poly(acrylic acid) (PAA) as the polyacid and poly(N,N-dimethylaminoethyl methacrylate) (PDMAEMA) or poly(diallyldimethyl ammonium chloride) (PDADMAC) as the polybases (NON-PTL 11). The weight fraction of water in absence of salt varies from 0.30 to 0.80 in both systems. As the coacervate droplets contain water, the metastable suspension can also be understood as a w/w emulsion. The dispersed phase in this system are the coacervate droplets which are dispersed in a continuous phase consistent almost entirely of water. Another special feature of the coacervate phase is that it displays a much higher viscosity compared to the initial polyelectrolyte solutions. Liu and co-workers reported an increase by 3 orders of magnitude in the viscosity of the coacervate phase between PAA and PDADMAC compared to the individual PEL solutions (NON-PTL 5). Finally, ultra-low interfacial tensions ($\gamma$) (around 1 mN $m^{-1}$ or lower) between the coacervate phase and the coexistent supernatant phase have been constantly reported by different authors (NON-PTL 12 to 15). De Ruiter and Bungenberg de Jong were the first who measured $\gamma$ between the coacervate phase and its equilibrium aqueous phase using the capillary rise method (NON-PTL 15). They pointed out the inaccuracy of the results for several reasons. The main drawback is that the capillaries need to be very narrow for those low interfacial tensions to be measured. Hence, frequent obstruction by small particles or aggregates can occur. More recently, the colloidal probe AFM (NON-PTL 12) and the surface force apparatus (SFA) (NON-PTL 13) have confirmed these findings of extremely low $\gamma$.

(b) Applications of complex coacervation

One of the most important applications of complex coacervation is microencapsulation. In this process, a substance (also called core material or internal phase) which can be solid or liquid in nature, is entrapped within a carrier material (also known as wall, shell or coating). The microcapsules are widely used in many industries such as printing, food, agriculture, cosmetics and specially pharmaceuticals (NON-PTL 16). The microcapsules can display different shapes (spherical, oval or irregular) (NON-PTL 17) and typically have either mononuclear or polynuclear structure (Figure 3) (NON-PTL 18). In the mononuclear case, the core material is surrounded by the wall material while in the second case, individual entities of core material are embedded in a matrix of the wall material.

In 1929, Bungenberg de Jong and Kurt first recognised the tendency of coacervate drops to absorb solid particles and organic liquids. Since then, many microencapsulation examples have been reported with both simple and complex coacervation. One example of encapsulation of lipophilic materials by simple coacervation was reported by Keipert and Melegari with gelatin (NON-PTL 19). A typical encapsulation process by simple coacervation consists first on emulsifying the polymer solution with the core material, followed by the addition of a coacervation agent and a final dehydration step. On the other hand, the process of encapsulation by complex coacervation consists of three basic steps: (a) formation of three immiscible phases: core material, coating material (complex between two oppositely charged polyelectrolytes) and dispersed media; (b) deposition of the coacervate phase around the core material; (c) solidification of the wall material by crosslinking, desolvation or thermal treatment to form the microcapsule shell. Microencapsulation by complex coacervation has been extensively studied for the electrostatic protein-polysaccharide complexes and specially for the system gelatin-gum Arabic. Two slightly different experimental set ups are described in the literature (NON-PTL 20). The first methodology consists of the addition of an aqueous solution containing the protein-polysaccharide complexes followed by homogenisation. This are the so-called mixed emulsions. Another approach involves first the formation of a primary emulsion stabilised by a protein, followed by the addition of a polysaccharide which adsorbs onto the protein layer, forming a bilayer. This method is known as bilayer or layer-by-layer emulsions. The complex at the emulsion droplet surface is found to enhance emulsion stability compared to emulsions of protein alone. Generally, in both cases a final step is required after the formation of the microcapsules. This consists of the addition of a crosslinking agent to harden the coacervate layer. Different crosslinking agents have been reported in the literature, such as glutaraldehyde, formaldehyde and epoxy compounds (NON-PTL 16).

For the encapsulation to be successful the complex adsorption and wettability are key parameters to consider. The complex coacervate has to spontaneously spread over the surface of dispersed liquid droplets or particles and coat them to form the capsule (NON-PTL 21). This ability can be assessed by interfacial tension measurements. Indeed, by measuring the interfacial tensions between the three phases (coacervate, core material and supernatant), three spreading coefficients (S) can be calculated. Depending on the sign combinations of the three S, different equilibrium morphologies (complete engulfing, partial engulfing or non-engulfing) can be predicted, as first described by Torza and Mason (NON-PTL 22).

The three-phase system under study is depicted in Figure 4. Phase 1 is the drop of core material, Phase 2 is the continuous phase and Phase 3 is the coacervate phase.

The spreading coefficient (S) measures the ability of one liquid to spontaneously spread across another and it is defined as the difference between the work of adhesion ($W_{adhesion}$) and the work of cohesion ($W_{cohesion}$) per unit area (equation 2).

$$S = W_{adhesion} - W_{cohesion} \qquad \cdots \text{ equation 2}$$

By considering the system in Figure 4, when Phase 3 wets Phase 1 the two previously existing surfaces (3-2) and (1-2) disappear. As a result, energy is released due to the respective interfacial tensions. At the same time, work, referred to as interfacial tension, must be done in order to form the new interface (3-1). Therefore, the work of adhesion (per unit area) for the previously defined three-phase system for the spreading of the coacervate phase onto the core material is given by equation 3. This concept was first introduced by Harkins (NON-PTL 23).

$$W_{adhesion,3} = \gamma_{32} + \gamma_{12} - \gamma_{31} \qquad \cdots \text{ equation 3}$$

On the other hand, the work of cohesion (per unit area) is the work necessary to bring two surfaces together and can be defined for our system as follows,

$$W_{cohesion,3} = \gamma_{32} + \gamma_{32} - 0 = 2\gamma_{32} \qquad \cdots \text{ equation 4}$$

Therefore, by subtracting equation 4 from equation 3, the following expression for the spreading coefficient of phase 3 can be written:

$$S_3 = \gamma_{12} - (\gamma_{13} + \gamma_{32}) \qquad \cdots \text{ equation 5}$$

The first term in equation 5 ($\gamma_{12}$) represents the energy per unit area of the original system, before the spreading of phase 3. The negative term ($\gamma_{13} + \gamma_{32}$) denotes the energy per unit area of the new system, where two new interfaces have been created. Therefore, if S< 0, the new surface energy is higher than the initial one and partial wetting occurs. On the other hand, if S > 0, the coacervate covers the total surface forming a film (total wetting). Hence, for the system under study, one equation for each phase can be written down following the same derivation but considering the spreading of different phases:

$$S_1 = \gamma_{23} - (\gamma_{12} + \gamma_{13}) \qquad \cdots \text{ equation 6}$$

$$S_2 = \gamma_{13} - (\gamma_{12} + \gamma_{23}) \qquad \cdots \text{ equation 7}$$

$$S_3 = \gamma_{12} - (\gamma_{13} + \gamma_{32}) \qquad \cdots \text{ equation 8}$$

Assuming that the interfacial tension between the continuous phase and the coacervate phase is lower than the one between the core material with the continuous phase ($\gamma_{23} < \gamma_{12}$), $S_1$ is always negative. This means no spontaneous

spreading of phase 1 over phases 2 or 3. Therefore, with this presumption three possible combinations of spreading coefficients arise, which correspond to three specific microcapsule morphologies shown in Figure 5.

Several studies have determined the various spreading coefficients to evaluate the different capsule morphologies (NON-PTL 24 and 25). Loxley and Vincent worked with a three-phase system composed by oil, water and poly(methylmethacrylate) and spreading coefficients were calculated to account for the morphologies observed (NON-PTL 24). They measured the interfacial tensions between the core-oils and the aqueous solutions with a tensiometer and the Du Nouy ring method. The interfacial tensions between the polymer and the different liquid phases (oil and aqueous solutions) were measured from the contact angle that each liquid made against a dry film of the polymer deposited on a clean glass slide. Then, from those values and using Young-Dupre equation, the interfacial tensions between the polymer and the various liquids were calculated. This procedure can be applied to our three-phase system (oil, water, coacervate phase) depicted in Figure 4. $\gamma_{12}$ can be measured with the tensiometer and the Du Nouy ring method while $\gamma_{13}$ and $\gamma_{23}$ can be found by applying Young's equation and determining the contact angle that each liquid (oil and water) make against a dry film of the coacervate deposited on a glass slide. To fully understand the use of Young's equation to elucidate the unknown interfacial tensions, a brief explanation of the related theory is given.

When a drop of liquid is placed on a solid surface in the presence of another fluid (gas or liquid) (Figure 6), the equilibrium three-phase contact angle ($\theta$) that arise is determined as a function of the three interfacial tensions as stated by Young's equation:

$$\gamma_{SG} = \gamma_{SL} + \gamma_{LG}\cos\theta \quad \cdots \text{ equation 9}$$

where $\gamma$ is the interfacial tension and the subscripts S, G and L refer to the solid, gas and liquid phases, respectively. The contact angle ($\theta$) is the angle formed between the solid surface and the tangent to the liquid surface at the line of contact with the solid. This equation is valid only for smooth, chemically homogeneous, impermeable and nondeformable surfaces (NON-PTL 26).

To understand the origin of the contact angle, two force balances have to be taken into account. These are the cohesive forces between the molecules in the liquid and the adhesive forces between the liquid molecules and the solid surface. If the liquid under study is water and the solid surface is made up by polar groups, strong adhesive forces will take place and low contact angles will be displayed. In this case, the surface is hydrophilic. Conversely, if the surface is mainly composed by non-polar groups (polymeric surfaces or organic layers), low adhesive forces and consequently large contact angles will be exhibited. This surfaces are hydrophobic (NON-PTL 26).

The equilibrium contact angle has been widely used to explain two close-related phenomena, such as wetting and spreading. If $\theta < 90°$ the liquid wets (spreads over) the solid while if $\theta > 90°$ the liquid does not wet the solid. If $\theta = 0°$ complete wetting occurs.

Therefore, two equations that can be written to work out the interfacial tensions between the coacervate and the oil and the coacervate and the aqueous solution ($\gamma_{CO}$ and $\gamma_{CW}$, respectively) are shown in Figure 7 together with the three-phases contact angle schemes re-labelled for these systems.

From the equations included in Figure 7, $\gamma_{CW}$ and $\gamma_{CO}$ are the unknowns. $\gamma_{WG}$ and $\gamma_{OG}$ are the water and the oil surface tensions, which can be measured with the Tensiometer and the Du Nouy ring method. Finally, $\gamma_{CG}$ is the surface energy of the coacervate and can be found in the literature for specific systems. Otherwise, the surface tension of the coacervate needs to be estimated by indirect methods. These methods are based on the nature of the interaction between the liquids and the solid surface. The surface energy ($\gamma$) is commonly decomposed into its polar ($\gamma^p$) and dispersive ($\gamma^d$) components (equation 10) (NON-PTL 27). The polar component comprises interactions such as hydrogen bonding whilst the dispersive component comes from van der Waals forces between the molecules of the material.

$$\gamma = \gamma^p + \gamma^d \quad \cdots \text{ equation 10}$$

The interfacial tension between a solid (S) and a liquid (L) phase ($\gamma_{SL}$) can be expressed in terms of the two components (dispersive and polar) for each phase as given by equation 11.

$$\gamma_{SL} = \gamma_{SG} + \gamma_{LG} - 2\left(\sqrt{\gamma_{SG}^{d}\gamma_{LG}^{d}} + \sqrt{\gamma_{SG}^{p}\gamma_{LG}^{p}}\right) \quad \cdots \text{ equation 11}$$

By combining equation 11 with Young's (equation 9), the following relationship is encountered:

$$\gamma_{SG} - \gamma_{SL}\cos\theta_{LG} = \gamma_{SG} + \gamma_{LG} - 2\left(\sqrt{\gamma_{SG}^{d}\gamma_{LG}^{d}} + \sqrt{\gamma_{SG}^{p}\gamma_{LG}^{p}}\right) \quad \text{... equation 12}$$

After re-arranging equation 12, a final expression is found (equation 14).

$$-(\gamma_{SL} + \gamma_{LG\cos\theta_{LG}}) = -2\left(\sqrt{\gamma_{SG}^{d}\gamma_{LG}^{d}} + \sqrt{\gamma_{SG}^{p}\gamma_{LG}^{p}}\right) \quad \text{... equation 13}$$

$$\frac{1}{2}\gamma_{LG}(1 + \cos\theta_{LG}) = \sqrt{\gamma_{SG}^{d}\gamma_{LG}^{d}} + \sqrt{\gamma_{SG}^{p}\gamma_{LG}^{p}} \quad \text{... equation 14}$$

The two unknowns in this modified equation are $\gamma^{d}_{SG}$ and $\gamma^{p}_{SG}$ and can be determined by solving simultaneously two versions of equation 14 with two oils of different polarity. However, it is advisable to consider more than two liquids (NON-PTL 28). After solving all the possible combinations of equations, a least-squares calculation is carried out to determine the best combination of $\gamma^{d}_{SG}$ and $\gamma^{p}_{SG}$ that fit all the oils simultaneously. A 3-D surface energy diagram can be obtained by plotting the goodness of fit to contact angle set against a matrix of possible values of $\gamma^{d}_{SG}$ and $\gamma^{p}_{SG}$. From the coordinates that define the position of the peak in the chart, the values of $\gamma^{d}_{SG}$ and $\gamma^{p}_{SG}$ that best fit all the contact angles can be read (NON-PTL 29).

The microencapsulation process can be understood as an emulsion system where the surface active material that stabilises both immiscible phases is the coacervate complex. In fact, many examples of emulsions stabilised by coacervate complexes prepared from the interaction of proteins and polysaccharides are found in the literature (NON-PTLs 20, 30 and 31).

2. EMULSIONS

An emulsion can be defined as a turbid, heterogeneous system of two immiscible liquid phases in which one of the phases (dispersed phase) is dispersed into the other (continuous phase) as drops of microscopic or colloidal size (NON-PTLs 32 and 33). The boundary between these phases is called the interface. Depending on which phase contains the drops, two types of simple emulsions can be distinguished. Oil droplets dispersed in water form the so-called oil-in-water emulsions (o/w) while water droplets dispersed in oil constitute the water-in-oil (w/o) emulsions. The simplest way to distinguish between o/w and w/o emulsions is the drop test. This consists in checking the miscibility of the emulsion with water and oil. If a small volume of the emulsion mixes readily with water, the continuous phase is aqueous whereas, w/o emulsions mix easily in oil. Electrical conductivity measurements can be used as well to determine the emulsion type by taking into account that an aqueous continuous phase displays higher conductivity values than an oil continuous phase (NON-PTLs 26 and 33). Emulsions are high energy states. Hence, they are thermodynamically unstable and nature rapidly wants to bring them back to the steady state (phase separated). Therefore, in order to protect the formed drops from re-coalescence, a surface-active material (emulsifier) should be added (NON-PTL 32).

2.1. Emulsion thermodynamics

Emulsions are inherently non-equilibrium systems because of their interfacial free energy, which would be released during the emulsion breaking. Based on equation 15, the change in the Gibbs free energy of the system ($\Delta G$) can be expressed by:

$$\Delta G = \Delta H - T \cdot \Delta S + \sum n_i d\mu_i \quad \text{... equation 15}$$

where $\Delta S$ is the change in entropy, $\Delta H$ is the change in enthalpy, ni is the number of moles of the component i and $\mu_i$ is the chemical potential of the component i. At constant composition, the Gibbs model can be reduced to equation 16.

$$\Delta G = \Delta H - T \cdot \Delta S \quad \text{... equation 16}$$

ΔS is a measure of the disorder in a system and in this case measures the size reduction of the dispersed phase. During the emulsification process the disorder increases which gives a ΔS > 0 contributing to stability. ΔH can be considered as the energy input needed to achieve a certain average droplet size and is equal to:

$$\Delta H = \Delta U + P\Delta V \qquad \cdots \text{ equation 17}$$

where ΔU is the change in the internal energy, P the pressure and ΔV the change in volume.

If a volume change during the emulsification is neglected, the enthalpy corresponds to the internal energy. The internal energy in this case is the sum of the work required to increase the interfacial area (ΔW) and an amount of heat, which results from wasting part of the energy input. The work required to increase the interfacial area can be used to measure the thermodynamic instability of an emulsion and is defined as stated in equation 18.

$$\Delta W = \gamma \cdot \Delta A \qquad \cdots \text{ equation 18}$$

where $\gamma$ is the interfacial tension and ΔA the change in the interfacial area. This suggests that the emulsion stability is enhanced if ΔW is low. This is due to either a low interfacial tension or bigger droplets (lower surface area) (NON-PTLs 34 and 35). During the emulsification process (Figure 8), an increase in the interfacial area is achieved due to the formation of small droplets by the input of considerable amount of mechanical energy. This leads to an increase in ΔW. Therefore, the system will evolve to recover its stability by the so-called breaking of the emulsion.

Despite emulsions being thermodynamically unstable, kinetic stability can be achieved by the absorption of an emulsifier at the liquid-liquid interface. These compounds are surface-active materials that decrease the interfacial tension, help to make small drops and reduce the thermodynamic driving force towards coalescence) (NON-PTLs 26 and 34).

2.2. Emulsion stability

Emulsions destabilise via a number of processes which may occur simultaneously or consecutively. The process by which an emulsion separates into oil and water phases can be originated by four different droplet loss mechanisms shown in Figure 9. They are known as: creaming (or sedimentation), flocculation, coalescence and Ostwald ripening (or disproportionation) (NON-PTL 32).

(a) Creaming: Creaming is a process by which the initial emulsion separates into a concentrated layer at the top of the sample (cream) which is richer in the dispersed phase than the bottom part (depleted serum). The formation of the cream is due to the vertical movement of the oil drops under gravity. Moreover, no change in drop size distribution is detected. Creaming is the principal process by which the disperse phase separates from an emulsion and is typically the precursor to coalescence. The equivalent phenomenon in a w/o emulsion is called sedimentation. The creaming rate (v) of an isolated, spherical drop can be estimated from Stokes' Law (equation 19).

$$v = 2a^2(\rho_0 - \rho)\, g / 9\eta \qquad \cdots \text{ equation 19}$$

where a is the radius of the drop, $\rho_0$ is the density of the continuous phase, $\rho$ is the density of the dispersed phase, $\eta$ is the viscosity of the continuous phase and g is the local acceleration due to gravity. Stokes' equation indicates that creaming in a dilute emulsion is inhibited by reducing the droplet radius, by increasing the viscosity of the continuous phase or by decreasing the density differences between the oil and water phases) (NON-PTL 32).

(b) Flocculation: Flocculation consists of the aggregation of droplets (net attraction, van der Waals) without merging. This means that all droplets remain as totally separate entities as there is no rupture of the stabilising layer at the interface. Flocculation usually leads to enhanced creaming as flocs rise faster due to the larger effective radius compared with individual drops (NON-PTL 32).

(c) Coalescence: Coalescence is an irreversible, first order rate process induced by the rupture of the film between two droplets which leads them to merge in a single larger drop with a subsequent reduction of the surface free energy (NON-PTLs 32 and 34).

(d) Ostwald ripening (disproportionation): Ostwald ripening is a process which involves the diffusion of disperse phase molecules from smaller to larger droplets through the continuous phase (NON-PTL 32). The driving force is due to the increased solubility (or chemical potential) of a substance bounded by a curved interface.

[CITATION LIST]

[NON-PATENT LITERATURE]

[NON-PTL 1] P.A. Albertsson, Partition of cell particles and macromolecules: distribution and fractionation of cells, mitochondria, chlorophasts, viruses, proteins, nucleic acids, and antigen-antibody complexes in aqueous polymer two-phase systems, Wiley-Interscience, New York, 1971, pp. 18-21.

[NON-PTL 2] K.R. Sharma, Polymer thermodynamics: Blends, copolymers and reversible polymerisation, CRC Press, Boca Roton, 2012, pp. 7.

[NON-PTL 3] O. Olabis, L.M. Robeson and M.T. Shaw. Polymer-polymer miscibility, Academic Press INC., New York, 1979, pp. 4.

[NON-PTL 4] F. Comert, A.J. Malanowski, F. Azarikia and P.L. Dubin, Coacervation and precipitation in polysaccharide-protein systems, Soft Matter, 2016, 12, 4154-4161.

[NON-PTL 5] X. Liu, M. Haddou, I. Grillo, Z. Mana, J. Chapel and C. Schatz, Early stage kinetics of polyelectrolyte complex coacervation monitored through stopped-flow light scattering, Soft Matter, 2016, 12, 9030-9038.

[NON-PTL 6] D. Priftis and M. Tirrell, Phase behaviour and complex coacervation of aqueous polypeptide solutions, Soft Matter, 2012, 8, 9396-9405.

[NON-PTL 7] F.W. Tiebackx, Gleichzeitige Ausflockung zweier Kolloide, Z. Chem. Ind. Kolloide, 1911, 8, 198.

[NON-PTL 8] H.G. Bungenberg de Jong and H.R. Kruyt, Coacervation (Partial miscibility in colloid systems), Proc. koninkl. Med. Akad. Wetershap., 1929, 32, 849-856.

[NON-PTL 9] A.I. Oparin, K.L. Gladilin, D.B. Kirpotin, G.V. Chertibrim, A.F. Orlovsky, Dokl. Acad. Nauk. SSSR, 1977, 232, 485.

[NON-PTL 10] E. Kizilay, A.B. Kayitmazer and P.L. Dubin, Complexation and coacervation of polyelectrolytes with oppositely charged colloids, Adv. Colloid Interface Sci., 2011, 167, 24-37.

[NON-PTL 11] P.K. Jha, P.S. Desai, J. Li and R.G. Larson, pH and salt effects on the associative phase separation of oppositely charged polyelectrolytes, Polymers, 2014, 6, 1414-1436.

[NON-PTL 12] E. Spruijt, J. Sprakel, M.A. Cohen Stuart and J. van der Gucht, Interfacial tension between a complex coacervate phase and its coexisting aqueous phase, Soft Matter, 2010, 6, 172-178.

[NON-PTL 13] D. Priftis, R. Farina and M. Tirrell, Interfacial energy of polypeptide complex coacervates measured via capillary adhesion, Langmuir, 2012, 28, 8721-8729.

[NON-PTL 14] J. Quin, D. Priftis, R. Farina, S.L. Perry, L. Leon, J. Whitmer, K. Hoffmann, M. Tirrell and J.J de Pablo, Interfacial tension of polyelectrolyte complex coacervate phases, ACS Macro Lett., 2014, 3, 565-568.

[NON-PTL 15] L. de Ruiter and H. G. Bungenberg de Jong, Proc. KNAW, 1947, 50, 836-848.

[NON-PTL 16] N. Devi, M. Sarmah, B. Khatun and T.K. Maji, Encapsulation of active ingredients in polysaccharide-protein complex coacervates, Adv. Colloid Interface Sci., in press.

[NON-PTL 17] A. Madene, M. Jacquot, J. Scher and S. Desobry, Flavour encapsulation and controlled release - a review, Int. J. Food Sci. Technol., 2006, 41, 1-21.

[NON-PTL 18] S. Liu, MSc Dissertation, Encapsulation of flax oil by complex coacervation, University of Saskatchewan, Canada, 2009.

[NON-PTL 19] S. Keipert and P. Melegari, Preparation and characterization of oil containing microparticles, Drug Dev. Ind. Pharm., 1993, 19, 603-621.

[NON-PTL 20] M. Evans, I. Ratcliffe and P.A. Williams, Emulsion stabilisation using polysaccharide-protein complexes, Curr. Opin. Colloid Interface Sci., 2013, 18, 272-282.

[NON-PTL 21] Encapsulation and controlled release technologies in food systems, ed. J.M. Lakkis, Wiley Blackwell, 2nd edition, 2007, ch. 7.

[NON-PTL 22] S. Torza and S.G. Mason, Three-phase interactions in shear and electrical fields, J. Colloid Interface Sci., 1970, 33 (1), 67- 83.

[NON-PTL 23] W.D. Harkins, Z Phys. Chem., 1928, 139, 647.

[NON-PTL 24] A. Loxley and B. Vincent, Preparation of poly(methylmethacrylate) microcapsules with liquid cores, J. Colloid Interface Sci., 1998, 208, 49-62.

[NON-PTL 25] A.L. Tasker, J.P. Hitchcock, L. He, E.A. Baxter, S. Biggs and O.J. Cayre. The effect of surfactant chain length on the morphology of poly(methylmethacrylate) microcapsules for fragrance oil encapsulation, J. Colloid Interface Sci., 2016, 484, 10-16.

[NON-PTL 26] G.T. Barnes and I.R. Gentle, Interfacial Science: An introduction, Oxford University Press Inc., New York, 2005, pp.8-15.

[NON-PTL 27] C. J. Van Oss, Interfacial Forces in Aqueous Media, Marcel Dekker, New York, 1994, ch.3.

[NON-PTL 28] C.J. Van Oss and R.F. Giese, The hydrophilicity and hydrophobicity of clay minerals, Clays Clay Miner., 1995, 43, 474-477.

[NON-PTL 29] B.P. Binks and A.T. Tyowua, Influence of the degree of fluorination on the behaviour of silica particles at air-oil surfaces, Soft Matter, 2013, 9, 834-845.

[NON-PTL 30] B. Yin, W. Deng, K. Xu, L. Huang and P. Yao, Stable nano-sized emulsions produced from soy protein and soy polysaccharide complexes, J. Colloid Interface Sci., 2012, 380,51-59.

[NON-PTL 31]A.R. Patel, E. Drost, J. Seijen ten Hoorn and K.P. Velikov, Fabrication and characterisation of emulsions with pH responsible switchable behaviour, Soft Matter, 2013, 9, 6747-6751.

[NON-PTL 32] B.P. Binks, Modern Aspects of Emulsion Science, Royal Society of Chemistry, Cambridge, 1998, ch. 1, pp.1-48.

[NON-PTL 33] I.D. Morrison and S. Ross, Colloidal Dispersions. Suspensions, Emulsions, and Foams, Wiley, New York, 2002.

[NON-PTL 34] P. Becher, Encyclopedia of Emulsion Technology, Marcel Dekker, New York, 1996, Volume 4, pp.1-37.

[NON-PTL 35] T.F. Tadros, Emulsion Formation, Stability, and Rheology, Wiley-VCH, Weinheim, 2013, pp.1-47.

[NON-PTL 36] H. Dautzenberg and B. Philipp, Polyelectrolytes: Formation, Application and Characterisation, Hanser Publishers, New York, 1994, pp. 1-45.

[NON-PTL 37] P.M. Visakh, O. Bayraktar, G. Pico, Polyelectrolytes. Thermodynamics and Rheology, Springer International Publishing, Switzerland, 2014, pp. 1-2.

[NON-PTL 38] G.S. Manning. Limiting laws and counterion condensation in polyelectrolyte solutions I. Colligative properties, J. Chem. Phys., 1969, 51, 924-933.

[NON-PTL 39] Polymeric Systems, ed. I. Prigogine and S.A. Rice, John Wiley & Sons, INC., 1997, pp. 12-14.

[NON-PTL 40] R.N. Goldberg, N. Kishore and R.M. Lennen, Thermodynamic quantities for the ionization reactions of buffers, J. Phys. Chem. Ref. Data, 2002, 31, 231-370.

[NON-PTL 41] J. Choi and M.F. Rubner, Influence of the degree of ionisation on weak polyelectrolyte multilayer assembly, Macromolecules, 2005, 38, 116-124.

[NON-PTL 42] S.E. Burke and C.J. Barrett, Acid-Base equlilibria of weak polyelectrolytes in multilayer thin films, Langmuir, 2003, 19, 3297-3303.

[NON-PTL 43] B.N. Dickhaus, R. Priefer, Determination of polyelectrolyte pKa values using surface-to-air tension measurements, Colloids Surf., A, 2016, 488, 15-19.

[NON-PTL 44] J. Koetz and S. Kosmella, Polyelectrolytes and Nanoparticles, Springer-Verlag Berlin Heidelberg, New York, 2007, pp. 1-4, 36-45.

[NON-PTL 45] A.V. Il'ina and V.P. Varlamov, Appl. Biochem. Micro., 2005, 41, 5-11.

[NON-PTL 46] T. Alonso, J. Irigoyen, J.J. Iturri, I.L Iarena and S.E. Moya, Soft Matter, 2013, 9, 1920-1928.

[NON-PTL 47] Modern Aspects of Emulsion Science, ed. B.P. Binks, Royal Society of Chemistry, Cambridge, 1998, pp. 1-48.

[NON-PTL 48] I.D. Morrison and S. Ross, Colloidal Dispersions. Suspensions, Emulsions, and Foams, Wiley, New York, 2002.

[NON-PTL 49] A.M. Bago Rodriguez, B.P. Binks and T. Sekine, Novel stabilisation of emulsions by soft particles: polyelectrolyte complexes, Faraday Discuss., 2016, 191, 255-285.

[NON-PTL 50] D.E. Koppel, J. Chem. Phys., 1972, 57, 4814-4820.

[NON-PTL 51] M. Smoluchowski, in Handbuch der Electrizitat und des Magnetismus, Leipzig, 1921, Vol. 2, pp. 366.

[NON-PTL 52] W.D. Harkins and H.F. Jordan, J. Am. Chem. Soc., 1930, 52, 1751-1772.

[NON-PTL 53] L. Vitorazi, N. Ould-Moussa, S. Sekar, J. Fresnais, W. Loh, J.-P. Chapel and J.-H. Berret. Evidence of two-step process and pathway dependency in the thermodynamics of poly(diallyldimethylammonium chloride)/poly(sodium acrylate) complexation, Soft Matter, 2014, 10, 9496-9505.

[NON-PTL 54] E.A. Litmanovich, E.V. Chernikova, G.V. Stoychev and S.O. Zakharchenko. Unusual behaviour of the mixture of poly(acrylic acid) and poly(diallyldimethylammonium chloride) in acidic media, Macromolecules, 2010, 43, 6871-6876.

[NON-PTL 55] T. Alonso, J. Irigoyen, J.J. Iturri, I.L. Iarena and S.E. Moya, Study of the multilayer assembly and complex formation of poly(diallyldimethylammonium chloride) (PDADMAC) and poly(acrylic acid) (PAA) as a function of pH, Soft Matter, 2013, 9, 1920-1928.

[NON-PTL 56] J. Koetz and S. Kosmella, Interactions between poly(diallyldimethylammonium chloride) and poly(acrylic acid) in dependence on polymer concentration, presented in part at the International Conference on Scaling concepts and complex fluids, Italy, 1994.

[NON-PTL 57] Y. Ishimuro and K. Ueberreiter. The surface tension of poly(acrylic acid) in aqueous solution. Colloid Polym. Sci., 1980, 258, 928-931.

[NON-PTL 58] A.S. Prata and C.R.F. Grosso, Influence of the oil phase on the microencapsulation by complex coacervation, J. Am. Oil Chem. Soc., 2015, 92, 1063-1072.

[NON-PTL 59] A. Goebel and K. Lunkenheimer. Interfacial tension of the water/n-alkane interface, Langmuir, 1997, 13, 369-372.
[NON-PTL 60] N.R. Pallas, Y. Harrison. An automated drop shape apparatus and the surface tension of pure water, Colloids and surfaces, 1990, 43, 169-194.
[NON-PTL 61] A. Mulero, I. Cachadina and M.I. Parra. Recommended correlations for the surface tensions of common fluids, J. Phys. Chem. Ref. Data, 2012, 41, 043105.
[NON-PTL 62] J.H. Clint, Adhesion and components of solid surface energies, Curr. Opin. Colloid Interface Sci., 2001, 6, 28-33.
[NON-PTL 63] B.P. Binks, T. Sekine and A.T. Tyowua, Dry oil powders and oil foams stabilised by fluorinated clay platelet particles, Soft Matter, 2014, 10, 578-589.
[NON-PTL 64] J.H. Clint and A.C. Wicks, Adhesion under water: surface energy considerations, Int. J. Adhes. Adhes., 2001, 21, 267-273.
[NON-PTL 65] B. P. Binks and J. H. Clint, Solid wettability from surface energy components: Relevance to pickering emulsions, Langmuir, 2002, 18, 1270-1273.

SUMMARY

[TECHNICAL PROBLEM]

The subject matter of the present invention is to provide a cosmetic composition comprising a coacervate droplet which can form a metastable water-in-water type emulsion.

[SOLUTION TO PROBLEM]

[Embodiment 1]

A cosmetic composition, comprising a water phase as a matrix, and an oil phase dispersed therein and stabilized by a coacervate droplet of an anionic polyelectrolyte and a cationic polyelectrolyte, the coacervate droplet being one which can form a metastable water-in-water type emulsion.

[Embodiment 2]

The cosmetic composition of embodiment 1 above, wherein each polyelectrolyte is not surface active by its own.

[Embodiment 3]

The cosmetic composition of embodiment 1 or 2 above, wherein the anionic polyelectrolyte is a strong anionic polyelectrolyte having a pKa of 1 or less, and the cationic polyelectrolyte is a strong cationic polyelectrolyte having a pKb of 1 or less.

[Embodiment 4]

The cosmetic composition of embodiment 3 above, wherein the strong anionic polyelectrolyte is one or more selected from a group consisting of poly(aryl sulfonic acid) and salts thereof, and the strong cationic polyelectrolyte is one or more selected from a group consisting of poly(quaternary ammonium salts).

[Embodiment 5]

The cosmetic composition of embodiment 4 above, wherein the strong anionic polyelectrolyte is poly(styrene sulfonate) sodium salt, and the strong cationic polyelectrolyte is poly(diallyldimethyl ammonium chloride).

[Embodiment 6]

The cosmetic composition of embodiment 1 or 2 above, wherein the anionic polyelectrolyte is a weak anionic polyelectrolyte having a pKa of more than 1, and the cationic polyelectrolyte is a strong cationic polyelectrolyte having a pKb of 1 or less.

[Embodiment 7]

The cosmetic composition of embodiment 6 above, wherein the weak anionic polyelectrolyte is one or more selected from a group consisting of poly(carboxylic acid), poly(allyl sulfonic acid), and salts thereof; and the strong cationic polyelectrolyte is one or more selected from a group consisting of poly(quaternary ammonium salts).

[Embodiment 8]

The cosmetic composition of embodiment 7 above, wherein the weak anionic polyelectrolyte is poly(acrylic acid) sodium salt, alginic acid, or poly(vinyl sulfate) potassium salt, and the strong cationic polyelectrolyte is poly(diallyldimethyl ammonium chloride).

[Embodiment 9]

The cosmetic composition of embodiment 1 or 2 above, wherein the anionic polyelectrolyte is a strong anionic polyelectrolyte having a pKa of 1 or less, and the cationic polyelectrolyte is a weak cationic polyelectrolyte having a pKb of more than 1.

[Embodiment 10]

The cosmetic composition of embodiment 9 above, wherein the strong anionic polyelectrolyte is one or more selected from a group consisting of poly(aryl sulfonic acid) and salts thereof; and the weak cationic polyelectrolyte is one or more selected from a group consisting of poly(primary ammonium), poly(secondary ammonium), and salts thereof.

[Embodiment 11]

The cosmetic composition of embodiment 10 above, wherein the strong anionic polyelectrolyte is poly(styrene sulfonate) sodium salt, and the weak cationic polyelectrolyte is poly(allylamine hydrochloride) or poly(ethylene imine).

[Embodiment 12]

The cosmetic composition of embodiment 1 or 2 above, wherein the anionic polyelectrolyte is a weak anionic polyelectrolyte having a pKa of more than 1, and the cationic polyelectrolyte is a weak cationic polyelectrolyte having a pKb of more than 1.

[Embodiment 13]

The cosmetic composition of embodiment 12 above, wherein the weak anionic polyelectrolyte is one or more selected from a group consisting of poly(carboxylic acid), poly(allyl sulfonic acid), and the salts thereof; and the weak cationic polyelectrolyte is one or more selected from a group consisting of poly(primary ammonium), poly(secondary ammonium), and salts thereof.

[Embodiment 14]

The cosmetic composition of embodiment 13 above, wherein the weak anionic polyelectrolyte is poly(acrylic acid) sodium salt, alginic acid, or poly(vinyl sulfate) potassium salt; and the weak cationic polyelectrolyte is poly(allylamine hydrochloride) or poly(ethylene imine).

[Embodiment 15]

A method for applying the cosmetic composition of any one of embodiments 1 to 14 above to a human skin.

[Embodiment 16]

The cosmetic composition of any one of embodiments 1 to 14 above, or the method of embodiment 15 above, wherein the cosmetic composition contains one or more the powder components listed below.

[Embodiment 17]

The cosmetic composition of any one of embodiments 1 to 14 above, or the method of embodiment 15 above, wherein the oil phase contains one or more the oil phase components listed below.

[Embodiment 18]

The cosmetic composition of any one of embodiments 1 to 14 above, or the method of embodiment 15 above, wherein the water phase contains one or more the water phase components listed below.

[ADVANTAGEOUS EFFECTS OF INVENTION]

According to the invention it is possible to provide a cosmetic composition comprising a coacervate droplet which can form a metastable water-in-water type emulsion.

BRIEF DESCRIPTION OF DRAWINGS

[FIG. 1] Types of phase separation upon mixing aqueous solutions of polymers. Classification taken from NON-PTL 1.
[FIG. 2] Composition of the two phases formed by a mixture of 1.1 % dextran and 0.36 % methylcellulose in water. Re-draw from NON-PTL 1.
[FIG. 3] Microcapsule with a mononuclear (A) and a polynuclear structure (B). Re-draw from NON-PTL 18.
[FIG. 4] Schematic representation of the three-phase system: core material (1), continuous phase (2) and coacervate phase (3).
[FIG. 5] Schematic diagram showing the three possible configurations: (a) complete engulfing, (b) partial engulfing and (c) non-engulfing, corresponding to the three sets of relations for S (given). Phase (1) is the core material, Phase (2) represents the continuous phase and Phase (3) the coacervate phase. Re-draw from NON-PTL 22.
[FIG. 6] Three-phases contact line of a drop of liquid wetted to a solid surface in a gas phase.
[FIG. 7] Three-phases contact angle of the system gas-water-coacervate (GWC) and gas-oil-coacervate (GOC). The adapted Young's equations for each system are included on the bottom of the figure.
[FIG. 8] Thermodynamics of the emulsification process.
[FIG. 9] Schematic representation of different ways by which an emulsion can become unstable.
[FIG. 10] Chemical structure of poly(acrylic acid) sodium salt (PAANa) (left) and poly(diallyldimethyl ammonium chloride) (PDADMAC) (right).
[FIG. 11] Dipole formed due to the presence of the carboxylic acid moiety.
[FIG. 12] On the left hand side, schematic representation of an intermolecular H-bonding between two carboxylic acids. On the right hand side, schematic representation of the H-bonding between a carboxylic acid and water molecules.
[FIG. 13] Potentiometric titration of a 1 g $L^{-1}$ PAANa solution against HCl (0.1 M). The pH of the initial PAANa solution was increased up to pH ≈ 12 with NaOH to evaluate the full pH range. Inset plot: curve of the degree of ionisation against the pH obtained from the titration curve.
[FIG. 14] Appearance of the freshly prepared aqueous PEC dispersions from the 0.1 g $L^{-1}$ individual PEL solutions at different $x_{PAANa}$ and pHs (given). Scale bar = 1 cm.
[FIG. 15] Variation of the average particle diameter with the $x_{PAANa}$ for the aqueous PEC dispersions prepared from the 0.1 g $L^{-1}$ individual PEL solutions at different pHs (given).
[FIG. 16] Variation of the zeta potential with the $x_{PAANa}$ for the aqueous PEC dispersions prepared from the 0.1 g $L^{-1}$ individual PEL solutions at different pHs (given).
[FIG. 17] Variation of the $x_{PAANa}$ (at zeta potential = 0) with the pH for the aqueous PEC dispersions prepared from the 0.1 g $L^{-1}$ individual PEL solutions.
[FIG. 18] Appearance of the aqueous PEC dispersions prepared from the 5 g $L^{-1}$ PEL solutions at (a) pH = 10 and (b) pH = 4 at different $x_{PAANa}$ (given). Scale bars = 1 cm. (c) Optical microscope image of a drop of the white dispersion ($x_{PAANa}$ = 0.57, [PAANa] = [PDADMAC] = 5 g $L^{-1}$) at pH = 10. (d) Optical microscope image of a drop of the dispersion ($x_{PAANa}$ = 0.13, [PAANa] = [PDADMAC] = 5 g $L^{-1}$) at pH = 4.
[FIG. 19] Appearance of the aqueous PEC dispersions prepared from the 5 g $L^{-1}$ PEL solutions at (a) pH = 2 and (b) pH = 6 at different $x_{PAANa}$ (given). Scale bars = 1 cm. (c) Optical microscope image of a drop of the white dispersion ($x_{PAANa}$ = 0.57, [PAANa] = [PDADMAC] = 5 g $L^{-1}$) at pH = 2. (d) Optical microscope image of a drop of the dispersion ($x_{PAANa}$ = 0.57, [PAANa] = [PDADMAC] = 5 g $L^{-1}$) at pH = 6.

[FIG. 20] (1) Appearance of 1 mL of the PEC aqueous dispersion ([PEL] = 10 g $L^{-1}$, pH = 10, $x_{PAANa}$ = 0.5) before and after centrifugation. The three different phases separated after centrifugation are appointed as: supernatant (Phase A), interface (Phase B) and coacervate phase (Phase C). Optical microscope images of the different phases are attached. For Phase B, images from the same drop have been taken along the time (given). (2) Appearance of 1 mL of the PEC aqueous dispersion ([PEL] = 5 g $L^{-1}$, pH = 4, $x_{PAANa}$ = 0.5) after centrifugation.

[FIG. 21] Evolution of the stability of the aqueous PEC dispersions prepared from the 5 g $L^{-1}$ individual PEL solutions ($x_{PAANa} \approx 0.50$) at different pHs (given) along the time. Scale bar = 1 cm.

[FIG. 22] Emulsions prepared with n-dodecane and a aqueous PEC dispersion ([PEL] = 5 g $L^{-1}$, pH = 10, $x_{PAANa}$ = 0.5). (a) addition of oil in one step ($\phi_o$ = 0.20), (b) addition of oil stepwise. The fraction of oil after each addition is given. Scale bar = 1 cm.

[FIG. 23] Appearance of the emulsions prepared with n-dodecane ($\phi_o$ = 0.20) stabilised with a PAANa solution at pH = 2 and different concentrations (given in g $L^{-1}$) along the time. Scale bar equals to 1 cm.

[FIG. 24] (a) Optical microscope images of the freshly prepared emulsion with n-dodecane ($\phi_o$ = 0.05) and the aqueous PEC dispersion ([PEL] = 5 g $L^{-1}$, pH = 10, $x_{PAANa}$ = 0.5) at different magnifications (given). (b) Cryo-SEM images of the freshly prepared emulsion with n-dodecane ($\phi_o$ = 0.20) and the aqueous PEC dispersion ([PEL] = 5 g $L^{-1}$, pH = 10, $x_{PAANa}$ = 0.5) at different magnifications (given).

[FIG. 25] (a) Appearance aqueous PEC dispersion prepared from the 30 g $L^{-1}$ PEL solutions at pH = 10 ($x_{PAANa}$ = 0.5). A white dispersion coexists with a viscous gelatinous phase as seen on the upside-down vial. (b) Appearance emulsion immediately after homogenisation ($\phi_o$ = 0.20). The second vial corresponds to the same vial after the removal of the gelatinous phase on the top. Scale bar = 1 cm. (c) Optical microscope image of the white dispersion of the vial in (a). (d) Optical microscope image of the gelatinous phase of the vial in (a). (e) Optical microscope image of the aqueous dispersion separated after homogenisation, vial (b).

[FIG. 26] Appearance of the emulsion with (a) isopropyl myristate and (b) squalane ($\phi_o$ = 0.20) and the aqueous PEC dispersion ([PEL] = 5 g $L^{-1}$, pH = 10, $x_{PAANa}$ = 0.5) along the time. Scale bars = 1 cm.

[FIG. 27] (a) Appearance of the emulsion with PDMS ($\phi_o$ = 0.20) and the aqueous PEC dispersion ([PEL] = 5 g $L^{-1}$, pH = 10, $x_{PAANa}$ = 0.5) along the time. Scale bar = 1 cm. (b) Optical microscope images of the above emulsion once creaming stopped.

[FIG. 28] (a) Appearance of the emulsion with toluene ($\phi_o$ = 0.20) and the aqueous PEC dispersion ([PEL] = 5 g $L^{-1}$, pH = 10, $x_{PAANa}$ = 0.5) along the time. Scale bar = 1 cm. (b) Optical microscope images of the above emulsion once creaming stopped.

[FIG. 29] 3-D surface energy plots for the coacervate phase as a function of the possible values of $\gamma^d_{CG}$ and $\gamma^p_{CG}$. The ordinate represents the goodness of fit to contact angles.

[FIG. 30] Appearance of the emulsions with n-dodecane ($\phi_o$ = 0.20) and the aqueous PEC dispersions ([PEL] = 5 g $L^{-1}$, $x_{PAANa} \approx 0.50$) at different pHs (given). The appearance of the emulsions 11 days after preparation is also shown. Scale bar = 1 cm.

[FIG. 31] Structural formula of linear poly(ethylenimine) (PEI).

[FIG. 32] Structural formula and pH dependence of branched poly(ethylenimine) (PEI).

[FIG. 33] Various combinations of strong and weak polyelectrolytes.

[FIG. 34A] Appearance of aqueous PEC dispersions of PAH and PSSNa. 5 g $L^{-1}$, pH = natural. pH = 2, precipitate/flocks. pH = natural, precipitate/flocks. All the particles migrate to the cream.

[FIG. 34B] Appearance of emulsions of PAH and PSSNa. 5 g $L^{-1}$, pH = natural. Similar results to pH = 2. Stable emulsion at specific $x_{PAANa}$. All the particles migrate to the cream.

[FIG. 34C] Appearance of aqueous PEC dispersions of PAH and PSSNa. 1 g $L^{-1}$, pH = 10. pH = 10, cloudy dispersions coacervation.

[FIG. 34D] Appearance of emulsions of PAH and PSSNa. 1 g $L^{-1}$, pH = 10. Stable emulsion at specific $x_{PAANa}$.

[FIG. 35A] Appearance of emulsions of PAH and PSSNa due to influence fraction of oil ($\phi_o$). Emulsion pH = 2, 5 g $L^{-1}$, $x_{PSSNa}$ = 0.83. No catastrophic phase inversion is achieved. Formation of high internal phase emulsions (HIPEs) at high $\phi_o$.

[FIG. 35B] Optical micrographs of emulsions of PAH and PSSNa due to influence fraction of oil ($\phi_o$). Emulsion pH = 2, 5 g $L^{-1}$, $x_{PSSNa}$ = 0.83. Rheology measurements have been carried out for all the emulsions.

[FIG. 36A] Appearance of aqueous PEC dispersions of PAH and PAANa. 5 g $L^{-1}$, pH = 4. pH = 4, precipitate/flocks, in general quite a lot of aggregation. All the particles migrate to the cream.

[FIG. 36B] Appearance of emulsions of PAH and PAANa. 5 g $L^{-1}$, pH = 4. pH = 4, precipitate/flocks, in general quite a lot of aggregation. All the particles migrate to the cream. However in some cases the emulsion is not stable at long term, probably due to the relatively big aggregates.

[FIG. 36C] Appearance of aqueous PEC dispersions of PAH and PAANa. 1 g $L^{-1}$, pH = 7. pH = 7 and 10, cloudy dispersions coacervation.

[FIG. 36D] Appearance of emulsions of PAH and PAANa. 1 g $L^{-1}$, pH = 7. No stable emulsions so far.

[FIG. 37] Types of emulsifiers and mechanism of emulsion stabilization.
[FIG. 38] Types of phase separation upon mixing aqueous solutions of polymers.
[FIG. 39] Emulsions stabilized by polyelectrolyte complexes (PEC) prepared from the mixture of two oppositely charged polyelectrolytes.
[FIG. 40] Materials and methods regarding strong PELs, pure standards, narrow molecular weight distribution.
[FIG. 41A] Results of appearances and a SEM image of PEC aqueous dispersions (1). Effect of $x_{PSSNa}$: [PEL] = 0.1 g $L^{-1}$ and different $x_{PSSNa}$ (given). Average diameter PEC particles ≈ 150nm. Size in agreement with SEM. Particle charge changes from + to - by varying $x_{PSSNa}$.
[FIG. 41B] Results of dynamic light scattering of PEC aqueous dispersions (1). Effect of $x_{PSSNa}$:[PEL] = 0.1 g $L^{-1}$ and different $x_{PSSNa}$ (given). Average diameter PEC particles ≈ 150nm. Size in agreement with SEM. Particle charge changes from + to - by varying $x_{PSSNa}$.
[FIG. 42A] Results of appearances of PEC aqueous dispersions (2). Effect of [PEL] : $x_{PSSNa}$ ≈ 0.5 and different [PEL] (given).
[FIG. 42B] Results regarding an average diameter and the [PEL] of PEC aqueous dispersions (2). Effect of [PEL] : $x_{PSSNa}$ ≈ 0.5 and different [PEL] (given).
[FIG. 43A] Results of appearances and an average droplet diameter of emulsions (1). Effect of $x_{PSSNa}$ : [PEL] = 20 g $L^{-1}$, $\phi_o$ = 0.2 and different $x_{PSSNa}$ (given).
[FIG. 43B] Results of optical micrographs of emulsions (1). Effect of $x_{PSSNa}$ : [PEL] = 20 g $L^{-1}$, $\phi_o$ = 0.2 and different $x_{PSSNa}$ (given).
[FIG. 44A] Results of appearances and an average droplet diameter of emulsions (2). Effect of [PEL] : $x_{PSSNa}$ ≈ 0.5, $\phi_o$ = 0.2 and different [PEL] (given/ g $L^{-1}$).
[FIG. 44B] Results of SEM images of emulsions (2) at [PEL] = 20. Effect of [PEL] : $x_{PSSNa}$ ≈ 0.5, $\phi_o$ = 0.2 and different [PEL] (given/ g $L^{-1}$). Densely packed PEC at the oil-water interface. More than one particle layer exists at the droplet interface. Excess particles form a network in the continuous aqueous phase.
[FIG. 45A] Results of appearances of emulsions (3). Effect $\phi_o$ : $x_{PSSNa}$ ≈ 0.5, [PEL] = 8 g $L^{-1}$ and different $\phi_o$ (given). Reduction of droplet size with increasing $\phi_o$. No catastrophic phase inversion.
[FIG. 45B] Results of appearances of emulsions (3) and an optical micrograph of an emulsion prepared with a squalane. Effect of oil type: $x_{PSSNa}$ ≈ 0.5, [PEL] = 50 g $L^{-1}$, $\phi_o$ = 0.2 and different oils (given). Regarding (1) squalane, (2) isopropyl myristate, (3) toluene, (4) 50 cS PDMS and (5) liquid paraffin, oil type does not affect emulsion stability.
[FIG. 46A] Results of appearances of emulsions (4). Effect of salt concentration: $x_{PSSNa}$ ≈ 0.5, [PEL] = 1 g $L^{-1}$, $\phi_o$ = 0.2 and different [NaCl] (given / M).
[FIG. 46B] Results of optical micrographs corresponding to the appearance results of emulsions (4).
[FIG. 46C] Results of optical micrographs corresponding to the appearance results of emulsions (4).
[FIG. 47] Results regarding an average droplet diameter and the [PEL] of emulsions (5). Limited coalescence model.
[FIG. 48] Results of interfacial tension of emulsions (6). Interfacial tension measurements of PEC aqueous dispersions. Dodecane-water interfacial PEC sol. ([PEL] = 1 g $L^{-1}$). No substantial reduction of the interfacial tension. The operative mechanism in emulsion stabilization is not the reduction of the interfacial tension.

DESCRIPTION OF EMBODIMENTS

Hereinafter, embodiments of the present invention will be described in detail. The present invention is not limited to the following embodiments, but may be variously modified within the scope of the present invention.

ABSTRACT

This application is divided into three sections. SECTION 1 described above constitutes a literature review about the different types of phase separation upon mixing polymer solutions. Special attention will be given to the description and applications of one type of associative phase separation, the so called complex coacervation. This introductory section will be followed by a brief summary on emulsion science and some important remarks to consider about the two polyelectrolytes that will be discussed along this report. SECTION 2 described below contains a draft of a paper (Evaluation of emulsion stability by complex coacervation of two synthetic polyelectrolytes) summarising all the important findings about the studied system. Finally, some ideas for future experiments are posed.
Complex coacervation is a liquid-liquid type of associative phase separation obtained by the interaction between two oppositely charged species. As a result, two immiscible (and hence incompatible) liquid phases arise. The upper phase (supernatant) consists almost entirely of solvent and the bottom phase embody a dense clear liquid phase (coacervate) which is concentrated in macromolecules. One of the most important applications of complex coacervation is microencapsulation. In microencapsulation, a substance (also called core material) which can be solid or liquid in nature, is entrapped within a carrier material. For the encapsulation to be successful, the complex adsorption and wettability

are key parameters to consider. The complex coacervate has to spontaneously spread over the surface of dispersed liquid droplets or particles and coat them to form a capsule.

Following up the idea developed during the first year with the PEC system prepared from the mixture of two strong polyelectrolytes (PSSNa and PDADMAC), the investigation has been directed towards the study of the PEC formed between a strong (PDADMAC) and a weak (PAANa) polyelectrolyte. Therefore, with this new system, the influence of pH is an important variable to consider. The selected PEC system was first characterised through dynamic light scattering and optical microscopy. Then, emulsion stability was investigated. Parameters such as the mole fraction of the individual polyelectrolytes, the polyelectrolyte concentration or the oil type were assessed. Finally, and linked to the microencapsulation area, the determination of the spreading coefficients was carried out to predict the morphology of the obtained capsules.

3. IMPORTANT REMARKS OF THE STUDIED PELs

A polyelectrolyte (PEL, also called polyion) can be defined as a polymer system consisting of a macromolecule bearing numerous ionisable groups (either cationic or anionic) and low molecular weight counterions to ensure electroneutrality (NON-PTL 36). Due to the charges along the polymer chain, they are hydrophilic and water-soluble. Depending on the nature of the bound ions, polyions can be classified in polycations (positive charged groups along the polymer chain), polyanions (negative charged groups) and polyampholytes (the main backbone chain contains both cationic and anionic groups). Moreover, in terms of the degree of ionization and how it is affected by pH-changes, PELs can be classed as strong or weak. Strong polyelectrolytes, such as poly(styrene sulfonate) sodium salt (PSSNa) or PDADMAC, dissociate completely in the entire pH range. Therefore, the total charge is solely given by the polymer structure. On the other hand, weak polyelectrolytes (polyacids and polybases), e.g. poly(acrylic acid), PAA, have a degree of dissociation that depends on the pH and the ionic strength (NON-PTLs 36 and 37). Therefore, at high pH values the charge of PAA increases with carboxylate dissociation whereas at low pH, PAA is not ionized. The chemical structures of a strong and a weak polyelectrolyte are depicted in Figure 10.

The behaviour of weak polyelectrolytes upon increasing or decreasing the charge density is not comparable to the case of simple electrolytes. In a polyelectrolyte not all counterions will dissociate away from the polymer chain. As the magnitude of the charge on the chain increases, it becomes progressively more difficult to remove the next ion due to the close proximity of charged groups. This is known as counterion condensation and is described by Manning's theory (NON-PTL 38). When the polyelectrolyte is strongly charged, some of the counterions remain bound to the polymer structure due to the large electrostatic potential on the chain, thereby reducing the effective charge of the polyion (NON-PTL 39). This is not the case for simple electrolytes as they are present in solution as individual entities separated one from each other by water molecules. As a result, while the pKa of a simple electrolyte such as acetic acid is 4.76 (NON-PTL 40), the literature values for the pKa of a polyelectrolyte with the same charged group (PAA) in absence of salt range from 5.5 to 6.79 (NON-PTLs 41 and 42).

Another parameter that influences de degree of ionisation is the ionic strength. When salt is added to a polyelectrolyte solution, the oppositely charged ions screen the electrostatic repulsion of the charges along the polymer chain and makes it easier to remove the proton in the case of a weak polyacid. Therefore, it reduces the pKa value (NON-PTL 43). For PAA, the pKa value is reported to be 4.68 in presence of 1.0 M of NaCl (NON-PTL 42), which is close to the value of acetic acid.

Moreover, in the case of poly(acrylic acid), the carboxylic acid moiety is a highly polar organic functional group due to the strongly polarized carbonyl and hydroxyl groups. Oxygen (which is a relatively electronegative atom) form a strong permanent dipole with the hydrogen and the carbon atoms to which it is covalently bond (Figure 11). This is originated from the large differences in electronegativity between the two atoms (electronegativities: H = 2.2; C = 2.55, O = 3.44).

As a result, these dipoles generated in the carboxylic acid moieties can interact through hydrogen bonding with other carboxylic acids or water molecules as depicted in Figure 12. This explains the widespread use of poly(acrylic acid) as superabsorbent in many applications, such as baby nappies.

As stated in section 1 of this introduction, it is known that oppositely charged polyelectrolytes can form complexes by electrostatic interaction between their charged domains. However, this associative phase separation can also be mediated through additional inter-macromolecular interactions, such as van der Waals, hydrogen bonding and hydrophobic and dipole interactions (NON-PTLs 44 and 45).

Regarding the polyelectrolyte system between PDADMAC and PAANa, which will be further discussed in this report, an interesting feature related to the ability of hydrogen bonding formation is remarkable. Alonso et al. studied the multilayer assemble and complex formation between PDADMAC and PAANa as a function of pH (NON-PTL 46). As stated above, PDADMAC is a strong polyelectrolyte while PAANa is a weak polyelectrolyte. Therefore, from this PEL combination one would expect to achieve complexes once the PAANa starts to get ionised as both polyelectrolytes could interact through electrostatic interactions. Furthermore, by increasing the pH, as the charge of PAA increases and the

electrostatic attraction should be stronger, the assemble would be enhanced. However, with their experiments they proved that the interaction between these two polyelectrolytes cannot be ruled out by electrostatics alone. Aggregates were detected at pH = 3 were PAA is not ionised. Their formation was explained by the formation of H-bonds between different chains of PAA. The authors also pointed out the possibility that PDADMAC could be forming complexes not with the whole aggregate of PAA formed at pH = 3, but with single chains or smaller aggregates of PDADMAC. Evaluating the situation at high pH, no complex was detected at pH = 13. PAA is more ionised at higher pH values and this leads to an increase in the charge. Therefore, there is an increase in the amount of water linked to the polymer through electrostatic interactions and hydrogen bonding. Conversely, the charges in PDADMAC are given by a quaternary amine surrounded by an organic environment, which makes it less favourable for structured water. At intermediate pHs these quaternary amines can destroy the water shell that surrounds the carboxylic acid groups of PAA and the electrostatic interaction takes place. However, with increasing the pH, the number of water molecules associated with the carboxylates increases and more unfavourable becomes the breaking of the water shell with the quaternary amines. As a result, no complexation is achieved. The role of hydrogen bonding in preventing the electrostatic complexation with PDADMAC was confirmed with urea, due to its ability as a hydrogen bonding breaker. Under these conditions complexation took place.

## 4. AIMS OF THE PROJECT

The aim of this thesis is to investigate whether polyelectrolyte complexes, prepared from the interaction between two non-surface active polyelectrolytes, can stabilize emulsions and foams.

From a previous study between the PEC system formed between two strong polyelectrolytes (PDADMAC and PSSNa), emulsion stabilisation was successfully achieved. In order to evaluate the generality of this mechanism, a new PEC system has been selected which consists of the mixture between a strong (PDADMAC) and a weak (PAANa) polyelectrolyte. The study of the aqueous PEC dispersions was first evaluated at different pHs followed by the study of emulsion stability. Polyelectrolytes with a similar Mw and relatively low polydispersity index have been selected for the study. Moreover, pure specimens were used to ensure that emulsion stabilisation do not come from impurities on the polyelectrolyte.

## SECTION 2- EVALUATION OF EMULSION STABILITY BY COMPLEX COACERVATION OF TWO SYNTHETIC POLYELECTROLYTES

## 1. INTRODUCTION

When mixing two polymer solutions, different types of phase separation can occur. If the polymers are incompatible (they repel each other), segregative phase separation takes place and each polymer is collected (predominantly) in a different phase. One example is the two phases formed by mixing dextran and methylcellulose in water (NON-PTL 1). Complete miscibility appears in exceptional cases and gives rise to a homogeneous solution as the mixture between polystyrene and poly(vinyl methyl ether) (NON-PTL 3). Finally, if the polymers show net attraction, usually through electrostatic interactions, associative phase separation is achieved. In this case, the two polymers are collected in one phase while the other phase consists almost entirely of solvent. This interaction can lead either to the formation of a complex coacervate (liquid-liquid type of phase separation) or a precipitate (solid-liquid type of phase separation). Both complexation mechanisms are driven by an increase of entropy due to the release of the counter-ions initially bound to the PEL backbone chain. Although the factors that dictate the nature of each type of associative phase separation are not fully elucidated, it is generally assumed that strong interactions between PELs originate precipitates, while in coacervation the interactions are relatively weak (NON-PTLs 4 and 5). The distinction between these two types of associative phase separation is not clear in the literature and generally the term polyelectrolyte complex (PEC) is adopted for both scenarios. However, optical microscopy is a simple and useful technique to distinguish between precipitation (amorphous solid particles) and complex coacervation (micro size droplets) (NON-PTL 4).

The term coacervation was coined by Bungenberg de Jong and Kruyt in 1929 and derives from Latin "co" (together) and "acerv" (a heap or aggregate) (NON-PTL 8). In coacervation a dense polymer-rich phase (coacervate phase) and a very dilute polymer-deficient phase (aqueous phase) coexist (NON-PTL 10). Coacervates are subdivided into simple and complex. In simple coacervation one macromolecule is present and the associative process is induced by the addition of a dehydrating agent such as salts, alcohols or a change in the temperature or the pH of the medium. One example is the mixture of polyethylene glycol, potassium phosphate and water, in which the bottom phase is rich in salt and the top phase is rich in the polymer (NON-PTL 1). On the other hand, in complex systems, two oppositely charged species are involved in an associative phase separation. As a result, two immiscible (and hence incompatible) liquid phases arise. The upper phase (supernatant) consists almost entirely of solvent and the bottom phase embody a dense clear liquid phase (coacervate) which is concentrated in macromolecules. Before reaching this steady state, a biphasic system formed by a metastable suspension of macroion-rich droplets (coacervate suspension) is present (NON-PTL 10). Coacervates hold higher amounts of water compared to precipitates. Jha et al. determined the weight fraction of water in the coacervate system containing poly(acrylic acid) (PAA) as the polyacid and poly(N,N-dimethylaminoethyl methacrylate) (PDMAEMA) or poly(diallyldimethyl ammonium chloride) (PDADMAC) as the polybases (NON-PTL 11). The weight fraction of water in absence of salt varies from 0.30 to 0.80 in both systems. Another special feature of the coacervate phase is that it displays a much higher viscosity compared to the initial polyelectrolyte solutions. Liu and co-workers reported an increase by 3 orders of magnitude in the viscosity of the coacervate phase between PAA and PDADMAC compared to the individual PEL solutions (NON-PTL 5). Finally, ultra-low interfacial tensions ($\gamma$) (around 1 mN $m^{-1}$ or lower) between the coacervate phase and the coexistent supernatant phase are measured (NON-PTLs 12 to 14).

One of the most important applications of complex coacervation is microencapsulation, as first recognised by Bungenberg de Jong in 1929 (NON-PTL 8). He pointed out the tendency of coacervate drops to absorb solid particles. In microencapsulation, a substance (also called core material) which can be solid or liquid in nature, is entrapped within a carrier material (NON-PTL 16). For the encapsulation to be successful the complex adsorption and wettability are key parameters to consider. The complex coacervate has to spontaneously spread over the surface of dispersed liquid droplets or particles and coat them to form a capsule (NON-PTL 21). This ability can be assessed by interfacial tension measurements. Indeed, by measuring the interfacial tensions between the three phases (coacervate, core material and supernatant), three spreading coefficients (S) can be calculated.

$$S_1 = \gamma_{23} - (\gamma_{12} + \gamma_{13}) \quad \text{... equation 20}$$

$$S_2 = \gamma_{13} - (\gamma_{12} + \gamma_{23}) \quad \text{... equation 21}$$

$$S_3 = \gamma_{12} - (\gamma_{13} + \gamma_{23}) \quad \text{... equation 22}$$

where the subscripts 1, 2 and 3 refers to each phase (oil, aqueous phase and coacervate, respectively). If we assume that $\gamma_{23} < \gamma_{12}$, then $S_1$ is always negative and only three possible combinations of spreading coefficients arise (equations 23 to 25).

$$S_1 < 0;\ S_2 < 0;\ S_3 > 0 \quad \text{... equation 23}$$

$$S_1 < 0;\ S_2 < 0;\ S_3 < 0 \quad \text{... equation 24}$$

$$S_1 < 0;\ S_2 > 0;\ S_3 < 0 \quad \text{... equation 25}$$

Depending on the sign combinations of the three S, different equilibrium configurations (complete engulfing, partial engulfing or non-engulfing) can be predicted, as first described by Torza and Mason (NON-PTL 22).

The definition of a microcapsule is comparable to an emulsion droplet. An emulsion is a heterogeneous system of two immiscible liquid phases in which one of the phases (dispersed phase) is dispersed in the other (continuous phase) as drops of microscopic or colloidal size (NON-PTLs 47 and 48). As emulsions are thermodynamically unstable, a surface-active material or emulsifier should be added to protect the formed drops from coalescence (NON-PTL 47). In the literature, both emulsions and microcapsules stabilised by electrostatic protein-polysaccharide complexes have been extensively reported. Two different experimental set ups are described (NON-PTL 20). The first methodology consists of the addition of an aqueous solution containing the protein-polysaccharide complexes followed by homogenisation. Another approach involves first the formation of a primary emulsion stabilised by a protein, followed by the addition of a polysaccharide which adsorbs onto the protein layer forming a bilayer. The complex at the emulsion droplet surface is found to enhance emulsion stability compared with emulsions of protein alone.

Despite many examples of emulsions stabilized by protein-polysaccharide mixtures are encountered in the literature, in all cases the protein acts as an emulsifier on its own. In this work we follow up the idea introduced in a

previous paper, in which we present the first example of emulsions stabilized by the complex formed between two oppositely charged strong polyelectrolytes (PSSNa and PDADMAC) (NON-PTL 49). The novelty of this system compared to the emulsions stabilized by the protein-polysaccharide complexes is that each polyelectrolyte is not surface active by its own. Here, we evaluate the ability of emulsion stabilization of a new system constituted by a strong (PDADMAC) and a weak (PAANa) polyelectrolyte. We first study the behaviour of aqueous mixtures by varying the pH and the mole fraction of one polyelectrolyte. The size and charge of the obtained complexes are examined and visual inspection of the type of associative phase separation is carefully appraised. Emulsions are then prepared from the aqueous polymer mixtures and oil and their stability and arrangements of the complexes around drops is evaluated. Finally, the equilibrium morphologies of the droplets formed with n-dodecane are predicted with the calculated spreading coefficients.

EXAMPLES

Hereinafter, the present invention will be explained in greater detail using examples and comparative examples, but the present invention is not limited to the examples.

## 2. EXPERIMENTAL

### 2.1. Materials

Poly(acrylic acid) sodium salt, PAANa, and poly(diallyldimethylammonium chloride), PDADMAC were purchased from Polymer Standard Services (PSS, Mainz) and were used as received. The chemical structures as well as some other properties are shown in Table 1. The two polyelectrolytes have been chosen to have a similar average molecular weight (Mw). For the emulsion preparation, five different oils were selected (Table 1). They include non-polar alkanes, an aromatic oil and a silicone oil. Prior to use, all the oils were passed twice through a basic alumina column (particle size: 0.06 to 0.20 mm, Merck kGaA) to remove polar impurities. Water was first passed through a reverse osmosis unit and then a Milli-Q reagent water system (Millipore). After treatment, its surface tension measured with a Kruss K11 tensiometer and Wilhelmy plate was 72.0 mN $m^{-1}$ at 25 °C. Hydrochloric acid, HCl (Fisher Chemical, 37%) and sodium hydroxide, NaOH (Fisher Scientifc, >97%) were used as received. Four liquids and water were selected to estimate the surface energy of the coacervate phase from contact angle measurements. They include glycerol (VWR Chemicals, 98%), formamide (>99%), $\alpha$ - bromonaphtalene (97%) and n-hexadecane (99%). All of them were purchased from Sigma-Aldrich unless otherwise stated.

### 2.2. Methods

#### 2.2.1. Preparation and characterization of PEC aqueous dispersions

A potentiometric titration for PAANa was performed with a calibrated pH meter (3510, Jenway) to determine the curve of the degree of ionization versus the pH. The natural pH of a 1 g $L^{-1}$ PAA solution was 9.7. The pH was raised up to pH = 12 with NaOH to titrate afterwards the full pH range with HCl 0.1 M.

Individual polyelectrolyte solutions of different concentrations (0.01, 5, 10 and 30 g $L^{-1}$) were prepared by weighing the corresponding amount of each PEL and dissolving them in Milli-Q water. PEL solutions were adjusted at the desired pH with NaOH and HCl solutions of various concentration with a pH meter. Aqueous PEC dispersions of different mole fractions of PAANa ($x_{PAANa}$) were obtained by mixing known volumes of each individual polyelectrolyte solution of a fixed concentration and pH with a magnetic stirrer (VWR VMS-C7) at room temperature. All solutions were prepared in 14 mL screw-cap glass vials. Due to the remarkable influence of the mixing procedure on the characteristics of the resulting PEC structures, we decided to work under fixed conditions in order to obtain reproducible results. The experimental set up tailed in a previous paper was followed (NON-PTL 49). The pH of the resulting PEC aqueous dispersions was checked with a pH meter. Dynamic light scattering (DLS) and the cumulant method (NON-PTL 50) was used to determine the size of the complex present in the aqueous PEC dispersions prepared at different $x_{PAANa}$ and pHs with no added electrolyte. Measurements were carried out at 25 °C using a Zetasizer Nanoseries NanoZS (ZEN3600, Malvern Instruments) and samples were placed in a plastic disposal cuvette of 1 cm path length. The instrument was equipped with a 4mW He-Ne laser beam as a light source, operating at $\lambda$ = 633 nm under a scattering angle of 173°. The results are given as the average of three measurements. The zeta potential was measured at 25 °C by a Zetasizer Nanoseries NanoZS (Malvern Instruments) equipped with a 4 mW He-Ne laser beam operating at $\lambda$ = 633 nm. Measurements were made by introducing a universal dip cell (ZEN1002, Malvern Instruments) inside a plastic disposal cuvette. The Smoluchowski approximation (NON-PTL 51) was used to convert measured electrophoretic mobilities to zeta potentials. Each value was averaged from three parallel measurements. The refractive indices of water and the coacervate phase were obtained using a refractometer (M46 313, Hilger) and a sodium lamp ($\lambda$ = 589 nm) at 25 °C and were 1.333

and 1.395, respectively. The refractive index of the coacervate phase was measured by triplicate from the gel phase obtained after mixing equimolar concentrations of each 30 g $L^{-1}$ PEL solutions at pH = 10.

To characterise the type of associative phase separation achieved at different pHs (precipitation or complex coacervation), visual inspection of the vials together with optical microscope images were carried out. Micrographs of a drop of the PEC aqueous dispersions were obtained on a glass slide (Fisher Scientific) using an Olympus BX-51 microscope fitted with a DP50 digital camera. The presence of both the coacervate phase and the precipitate was further investigated by centrifugation of 1 mL of the PEC aqueous dispersion with a minicentrifuge (Minispin plus, Eppendorf) at 10,000 rpm for different periods of time. Optical microscope images of the different phases separated were also taken.

The determination of the water content of the coacervate phase was performed following the procedure reported by NON-PTL 11. The PEC dispersion in this case was prepared from the 30 g $L^{-1}$PEL solutions at pH = 10 ($x_{PAANa}$ = 0.5). The coacervate phase was placed on a clean glass slide and the water was let to evaporate in an oven at 100 °C until constant weight. The water content was given as the average of three measurements.

2.2.2. Preparation and characterization of emulsions

The density of the oils was determined using a density meter (DMA 35N, Anton Paar) at 20 °C. The result is given as the average of two measurements. After each measurement, the instrument was rinsed with copious amounts of ethanol and water.

Emulsions between an aqueous solution containing the PECs and n-dodecane were prepared in 14 mL screw-cap glass vials. Both phases were emulsified with an Ultra-Turrax homogenizer (IKA® T25 digital) with a dispersing element of 8 mm (stator diameter). Mixing was maintained for 2 min at a constant speed of 13,000 rpm. Pictures of the obtained emulsions were taken just after preparation and over time to evaluate their stability. Micrographs of the freshly prepared emulsions were obtained on a dimple glass slide (Fisher Scientific) using and Olympus BX-51 microscope fitted with a DP50 digital camera. The mean droplet diameter of the emulsion was calculated from at least fifty individual droplets on digital micrographs with ImageJ 1.47v. The fraction of oil released 3 days after preparation was carefully separated from the top of the cream, weighted and converted to volume through the oil density (equation 26).

$$v = m/\rho \qquad \text{...equation 26}$$

where v is the volume, m is the weight, and ρ is the density.

Selected emulsions were imaged with Cryo-scanning electron microspcopy (Cryo-SEM). A small amount of the emulsion was mounted on the sample holder (diameter about 10 mm) with a spatula. The sample was plunged into liquid nitrogen, previously turned into a slush to decrease the temperature down to -210 °C. The frozen sample was placed inside the cryo-SEM preparation chamber (PP3010T, Quorum Technologies Ltd) and it was fractured with a sharp knife at -140 °C under high vacuum. Sublimation of the surface water (ice) was performed inside the Zeiss EVO 60 SEM chamber at -75 °C for 10 min to obtain a clearer picture of the emulsion droplets after the removal of some water. After that, the sample was coated with platinum to a thickness of about 2 nm in the preparation chamber. Afterwards, it was transfered again back to the SEM chamber for imaging at a voltage of 15 kV and a probe current of 30 pA.

Different sets of emulsions were systematically prepared by varying one of the following parameters each time: concentration of the starting PEL solutions, $x_{PAANa}$ and pH of the PEC solutions.

2.2.3. Estimation of the surface energy of the coacervate phase

The surface energy of the coacervate phase was estimated from contact angle measurements obtained from five probe liquids in air. The coacervate phase obtained after mixing the 30 g $L^{-1}$ individual PEL solutions at pH = 10 was spreaded carefully on a clean glass slide and the water was let to evaporate completely in the oven at 100 °C. The liquid-air contact angles were measured with a Kruss DSA Mk 10 apparatus with the static sessile drop method, by obtaining the profile of a liquid droplet on a coated glass slide. Between 4 and 10 L of liquids of different polarity (water, n-dodecane, glycerol, formamide, -bromonaphtalene and n-hexadecane) were placed on the glass slide with a needle. The Circle method was used to measure contact angles below 20° while the Young-Laplace-fit was applied for contact angles above 20°. When the contact angle between the liquid and the coacervate coated surface was too low to be measured accurately, it was taken to be < 5°. The contact angle values were given as the average of three independent measurements.

2.2.4. Determination of the spreading coefficients

The three spreading coefficients for the system water-coacervate phase-n-dodecane were calculated from the measured interfacial tensions between the different phases ($\gamma_{23}$, $\gamma_{12}$ and $\gamma_{13}$). The interfacial tension between n-dodecane and water ($\gamma_{12}$) was measured with the tensiometer (Kruss K11) and the Du Nouy ring method at 25 °C. The radius of the ring was 9.545 mm and the wire diameter 0.37 mm. The applied correction method was Harkins & Jordan (NON-PTL 52). The results of 3 separate measurements were averaged. After each measurement, the plate was rinsed with ethanol and heated to glowing in a blue Bunsen flame. The two remaining interfacial tensions ($\gamma_{23}$ and $\gamma_{13}$) were calculated from Young's equation. The contact angles were measured following the procedure defined previously.

3. RESULTS AND DISCUSSION

3.1. Characterisation of aqueous dispersions of PEC

The study of the PEC aqueous dispersions and emulsions was evaluated for the system PDADMAC-PAANa. Regarding the nature of each polyelectrolyte, PDADMAC is a strong polybase and it is fully ionised along all the pH range. On the contrary, PAA is a weak polyacid. Therefore, its degree of ionisation varies with the pH. A potentiometric titration of a 1 g $L^{-1}$ PAA solution was carried out in triplicate to determine the curve of the percentage of ionization versus the pH and consequently the pKa of the polyacid. In Figure 13, the potentiometric titration of the polyacid against HCl (0.1 M) is shown (empty circles). The black line that connects the dotted points correspond to the derivative of the pH as a function of the added volume of the titrating agent (dpH/dV). In this case, two jumps were observed during the titration, which is in agreement with the data reported in NON-PTLs 53 and 54. The one at low pH corresponds to the titration of the hydroxyl groups coming from the NaOH used to increase the pH of the solution (the natural pH of the solution at the concentration stated above was 9.7). The distance between the two maxima corresponds to the titration of the polyacid. The inset plot in Figure 13 displays the curve of the degree of ionization against the pH and it is obtained from the data of the titration curve. In order to do so, PAA is considered to be fully ionized (degree of ionization = 100%) at pH = 9.8 and fully protonated (degree of ionization = 0%) at pH = 4.25. These two values correspond to the pHs where the inflection points take place. Intermediate points at different degrees of ionization were selected and interpolated in the titration curve to build the full ionization curve. From this results, one would expect stronger electrostatic interactions with the oppositely charged polyelectrolyte (PDADMAC) at pHs higher than 9.8, when PAA is fully ionized. The pKa value was found to be 6.6, which is in agreement with the literature. The reported pKa values for PAA in absence of salt range from 5.5 to 6.79 (NON-PTLs 41 and 42). It is noteworthy the shift in the pKa of a polyacid compared to the value of the corresponding simple electrolyte. While poly(acrylic acid) has a pKa of 6.6, the pKa of a simple electrolyte such as acetic acid (which contain the same ionizable group) is 4.76. The behaviour of weak polyelectrolytes upon increasing or decreasing the charge density is not equivalent to the case of simple electrolytes. In a polyelectrolyte not all the counterions will dissociate away from the polymer chain. In fact, as the magnitude of the charge on the chain increases, it becomes progressively more difficult to remove the next ion due to the close proximity of charged groups. This is known as counterion condensation and is described by Manning's theory (NON-PTL 38). When the polyelectrolyte is strongly charged, some of the counterions remain bound to the polymer structure due to the large electrostatic potential on the chain, thereby reducing the effective charge of the polyion.

Therefore, the pH is a key parameter to consider in the study of the PEC formation of this polyelectrolyte mixture. Aqueous PEC dispersions at low concentration were first characterised in terms of size and zeta potential. The appearance of aqueous PEC dispersions prepared from 0.1 g $L^{-1}$ PEL solutions at different $x_{PAANa}$ and pHs is shown in Figure 14. x refers to the mole fraction using the values of Mw given in Table 1 (131.2 kDa PAANa and 174 kDa PDADMAC).

**Table 1.** Molecular characteristics of polyelectrolytes employed in PEC formation and chemical structure, source, purity and density (20 °C) of all the oils used.

| Polyelectrolyte | Repeat unit | Molar mass per charged unit/g $mol^{-1}$ | Mw[a]/Da | Mn[b]/Da | Mp[c]/Da | PDI[d] (Mw/Mn) |
|---|---|---|---|---|---|---|
| **PAANa** | 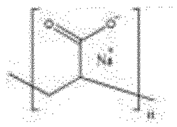 | 94.04 | 131,200 | 78,400 | 115,000 | 1.67 |
| **PDADMAC** | 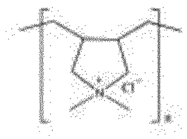 | 161.67 | 174,000 | 85,400 | - | 2.04 |

| Name | Structure | Supplier | Purity/ % | Density/g $cm^3$ |
|---|---|---|---|---|
| **n-dodecane** | 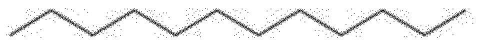 | AlfaAesar | >99 | 0.796 |
| **Squalane** | 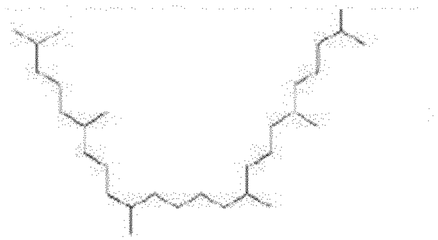 | Aldrich | ≥99 | 0.818 |
| ***Iso*propyl myristate** | 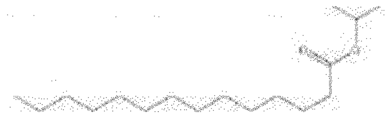 | Aldrich | >98 | 0.859 |
| **Toluene** | 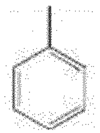 | Analar Normapur | 100 | 0.866 |

(continued)

| Name | Structure | Supplier | Purity/ % | Density/g cm$^3$ |
|---|---|---|---|---|
| **50 cS polydmiethyl siloxane (PDMS)** | 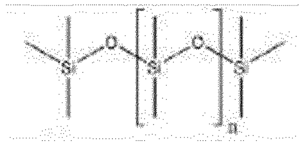 | Dow Coming | 100 | 0.964 |

[a]Mw: Weight average molecular weight, [b]Mn: Number avenge molecular weight; [c]Mp: Molar mass at the peak maximum; [d]PDL Polydispersity index

In general, all PEC solutions are transparent and colourless (Figure 14). However, some vials at specific mole fractions of PAANa are bluish or show precipitation. The size of the entities obtained after mixing each polyelectrolyte solution at different mole fractions and at specific pHs was measured with the Zetasizer. At pH = 2, despite no complexes are expected to be formed through electrostatic interactions as PAA is fully protonated, monomodal peaks at a $x_{PAANa}$ < 0.64 were achieved. The size of the objects is centred at around 100 nm except for the most turbid sample whose average diameter is about 450 nm (data not shown). At the other pH extreme (pH = 12) one would expect to obtain complexes through electrostatic interactions as both polyelectrolytes are fully charged. However, in this case the size was just measurable for the samples prepared with a $x_{PAANa}$ = 0.34 and 0.55 with sizes of 382 nm and 74 nm, respectively. At the other mole fractions, the samples were too polydisperse for cumulant analysis and in some cases the size distribution plot showed more than one peak. Therefore, from the results obtained at the extreme pHs, one can state that the interaction between the system PDADMAC-PAANa cannot be ruled by the electrostatics alone. This associative phase separation has to be also mediated through additional inter-macromolecular interactions, such as van der Waals, hydrogen bonding or hydrophobic and dipole interactions (NON-PTL 44). This agrees with a previous work from Alonso et al. (NON-PTL 55). They detected aggregates at pH = 3 were PAA is not ionized. Their occurrence was explained by the formation of H-bonds between different chains of PAA. The carboxylic acid moiety is a highly polar organic functional group due to the strongly polarized carbonyl and hydroxyl groups which can interact through hydrogen bonding with other carboxylic acids or water molecules. For the case of low pH, the authors also pointed out the possibility that PDADMAC could be forming complexes not with the whole aggregate of PAA, but with single chains or smaller aggregates of PDADMAC. Evaluating the situation at high pH, no complex was detected at pH = 13 either. By increasing the pH, the charge of PAA increases. As a result, there is an increase in the amount of water linked to the polymer through electrostatic interactions and hydrogen bonding. Conversely, the charges in PDADMAC are given by a quaternary amine surrounded by an organic environment, which makes it less favourable for structured water. At intermediate pHs these quaternary amines can destroy the water shell that surrounds the carboxylic acid groups of PAA and the electrostatic interaction takes place. However, with increasing the pH, the number of water molecules associated with the carboxylates increases and more unfavourable becomes the breaking of the water shell with the quaternary amines. As a result, no complexation is achieved. For the intermediate pHs, a measurable size was achieved at all mole fractions (Figure 15). The size was around 100 nm in all cases although some specific samples displayed a larger size. This match with the samples that visually show higher haziness.

The zeta potential of each solution at different pHs was measured with the Zetasizer (Figure 16). At pH = 2 all the dispersions displayed a positive value of zeta potential. As PAANa is fully protonated (from the titration curve), the positive charge is given by the quaternary nitrogen groups in PDADMAC. For the other pHs, the curve shows a sigmoidal shape with positive and negative values. However, the change on the sign of the zeta potential occurs at lower $x_{PAANa}$ as soon as the pH increases. At low pH values, not all the PAA groups are ionized. Therefore, a higher amount of PAA chains are required to fully neutralize the PDADMAC charges. As soon as the pH increases, the change in the sign of the zeta potential occurs at a lower $x_{PAANa}$. The mole fraction where the zeta potential is zero at different pH values is plotted in Figure 17. From a pH equal to the pKa onwards, the $x_{PAANa}$ of zero zeta potential reaches a plateau, as expected.

In terms of visual inspection at this low concentration, this system is comparable to the system between PSSNa and PDADAMAC (two strong polyelectrolytes) reported in a previous work (NON-PTL 49). However, things change upon increasing the concentration of the PEL solutions in order to prepare the PEC aqueous dispersions. This study was done at four different pH values to cover the full pH range: 2, 4, 6 and 10. Figure 18 shows the appearance of the PEC aqueous dispersions prepared at pH = 4 and 10 at different $x_{PAANa}$. At pH = 4 precipitation is observed at all mole fractions while at pH = 10 no complex precipitated out from the solution despite the fact that the PEC aqueous dispersions are whitish at low and intermediate $x_{PAANa}$. A close inspection of a drop of the whitish dispersions at pH = 10 under the optical microscope reveals the presence of the so-called coacervate droplets in contrast to the solid particles achieved at pH = 4 (Figure 18(c) and (d), respectively). They are distinguishable as the coacervates are fluid spherical entities and the particles are less spherical and solid in nature. This transition of precipitates to coacervate by increasing the pH was also reported by Jha and co-workers for the same polyelectrolyte system (NON-PTL 11). The appearance of the PEC dispersions at pHs 2 and 6 are included in Figure 19 together with optical microscope images of the resulting solutions. At pH = 2 all the dispersions were transparent apart from the one at $x_{PAANa}$ = 0.92, which was a bit bluish. At pH = 6 all the solutions were whitish and the most whitish one was obtained around charge neutralization. Spherical entities, which are related to coacervate droplets, were observed at all pHs and mole fractions.

In order to further evaluate the difference between these two types of associative phase separation (precipitation and coacervation) centrifugation of 1 mL of the PEC aqueous dispersions ($x_{PAANa}$ = 0.5) prepared at two different pHs (4 and 10) was carried out. The concentration of the PEC aqueous dispersions were 5 g $L^{-1}$ and 10 g $L^{-1}$ for pH = 4 and 10, respectively. At pH = 4, after centrifugation a white precipitate is collected on the bottom of the Eppendorf tube (Figure 20 (2)), which agrees with the solid-liquid type of phase separation. The appearance of the PEC aqueous dispersion at pH = 10 is shown before and after centrifugation (Figure 20 (1)). The solution was initially whitish and after centrifugation a phase separated system arise and fits with the liquid-liquid type of phase separation of the complex

coacervate. Three different phases were identified and designed as: supernatant (Phase A), interface (Phase B) and coacervate phase (Phase C). Each phase was observed under the microscope. The supernatant (Phase A) was mostly water depleted in coacervate droplets. Some spherical entities were still evident around the edges of the drop as soon as the water was evaporating. However, they were hardly visible at the beginning (Figure 20 (A)). The interface (Phase B) consisted of an aqueous phase rich in coacervate droplets (Figure 20, B). Pictures of the same drop placed on a glass slide were taken along the time. Coalescence of the coacervate droplets occurred, as after 17 minutes substantial grown in size was detected. Finally, the coacervate phase (Phase C) was a transparent and viscous phase concentrated in polymers (Figure 20 (C)). The water content of this phase was measured following the experimental procedure used in NON-PTL 11 for the same polyelectrolyte system. The water content averaged from three individual measurements was 63 % (± 3.4) which is in agreement with the value they reported for the same system but at pH = 7 (66 %).

The full pH range was studied at higher concentration (5 g $L^{-1}$) for a specific mole fraction ($x_{PAANa}$ = 0.5). Figure 21 shows the appearance of the vials at different pHs immediately after preparation and along the time. The same conclusions, regarding the type of associative phase separation, arise from this set of experiments. At pH = 2 the sample displays turbidity and coacervate droplets are detected through optical microscope images despite no electrostatic interaction is expected to occur. At pHs 3 and 4 both coacervate droplets and solid precipitates were formed. Co-appearance of both types of phase separation are reported in the literature for the system under study. Koetz and Kosmella found co-existence of flocculation and coacervation at specific Mw and PEL concentrations (NON-PTL 56). From pH = 5 onwards, no precipitates are observed neither visually, nor through optical microscope images. The amount of coacervate droplets seems to be higher at pHs 5 and 6 from optical microscope images and those samples display the maximum turbidity. As pointed out in the introductory section regarding the type of associative phase separation that takes place, it is generally assumed that precipitation rather than coacervation is obtained for strong interacting pairs. Therefore, one would expect precipitate formation at high pH values, which is not the case. This can be related to the enhanced ability of PAANa to form hydrogen bonds at high pH, as explained above. Due to the dipoles present in the carboxyl acid moieties, water is linked through hydrogen bonding and might partially screen the negative charge of the ionic groups. Therefore, the electrostatic interaction with PDADMAC is weakened and this leads to the formation of a complex coacervate instead of a precipitate.

Another important feature to point out in Figure 21 is the evolution of the stability along the time of the PEC aqueous dispersion at pH = 6. Despite the fact that it was the one that displayed higher turbidity, 2 months after preparation the majority of the coacervate phase was deposited on the bottom and a completely transparent solution was left. However, some coacervate droplets were still detected under the microscope in the supernatant.

3.2. Preparation and characterization of emulsions

Our interest here is whether the PEC prepared in water are surface-active enough to adsorb to an oil-water interface created on emulsifying the aqueous phase with a non-polar alkane. As identified on the previous section, the pH has a dramatic influence in the associative phase separation process that takes place upon mixing the two polyelectrolytes. Therefore, this parameter will be a key one on the study of emulsion stabilization.

Figure 22, shows the appearance of the emulsions obtained from the PEC aqueous dispersions prepared at pHs from 2 to 6 from the 5 g $L^{-1}$ PEL solutions ($x_{PAANa}$ = 0.5). At pH = 2 foam was achieved during mixing and was stable for few minutes. The surface activity was related to the presence of fully protonated poly(acrylic acid). In order to check this assumption, emulsions with the individual polyelectrolyte solutions at the same concentration and pH were prepared with n-dodecane. The emulsion with PDADMAC coalesces rapidly after preparation (data not shown) while the emulsion with PAA produces considerable amount of foam during homogenization and the cream on the top is stable for a longer period of time. The reduction of the surface tension of PAA at low pHs was reported by Ishimuro and Ueberreiter (NON-PTL 57). Further characterization of the ability of the PAA solution at pH = 2 to stabilize emulsions was carried out. PAA solutions at different concentrations (0.05 to 5 g $L^{-1}$) were prepared and emulsions were obtained following the standard procedure (Figure 23). The emulsions prepared with a PAA concentration of 0.05 and 0.10 g $L^{-1}$ completely coalesced in less than 10 minutes after preparation and no bubbles were formed during high-shear homogenization. On the other hand, emulsions prepared from the PAA solutions of a higher concentration (0.50, 0.75, 1.00 and 5.00 g $L^{-1}$) generated bubbles while mixing with the Ultra-turrax and a stable cream on the top was achieved for a longer period of time despite some coalescence of oil. Therefore, the concentration of poly(acrylic acid) required to display surface activity must be higher than 0.10 g $L^{-1}$. After two months, the oil released increased.

At pHs 3 and 4, no stable emulsions were obtained either. Presumably, the amount of particles was not high enough. Therefore, PEC aqueous dispersions were prepared from the PEL solutions of a higher concentration (10 g $L^{-1}$). However, the amount of particles did not increase substantially compared to the ones prepared from the 5 g $L^{-1}$ PEL solutions. The emulsion completely coalesced after homogenization (data not shown). At pHs 5 and 6 an unexpected situation was encountered. As seen from the appearance of the PEC solutions (Figure 21), the aqueous dispersions at pHs 5 and 6 were the most turbid ones. However, after the homogenization step, the aqueous phase was almost

transparent. In both cases, the coacervate droplets coalesced on the head of the homogenizer and therefore they were not taking part on emulsion stabilization. Hence, the coacervate system is a difficult system to deal with due to its specific nature.

For higher pHs (7 to 10) the behaviour was comparable so the study will be focused for the solution prepared at pH = 10. Two different preparations were tested: addition of oil in one step and addition of oil stepwise (Figure 22). Emulsions with an oil fraction ($\phi_o$) of 0.20 were prepared in both cases. For the emulsion prepared with the addition of oil stepwise, the oil was added in fractions of 200 L and homogenized with the Ultra-turrax each time up to a final fraction of oil of 0.20. Between each addition, the emulsion was let to stand for 3 days and the release of oil was measured by weight and expressed in volume through the density. For the addition of oil in one step, the emulsion was almost broken 1 day after preparation. The amount of coalescence was high but there was still some cream around the walls of the vial. For the emulsion prepared stepwise, the cream on the top was stable for a longer period of time for low volume fractions of oil. However, the emulsion prepared stepwise with a final content of oil of 0.20, despite that it was more stable than the one prepared with one step addition, the oil released 3 days after preparation represented the 12% of the total volume of oil. The amount of oil released was higher compared to the lower concentrations (<10%). Therefore, maybe the fraction of oil that the system can emulsify has to be lower than 0.13.

Optical microscope images were taken on a glass slide without coverslip for all the freshly prepared emulsions (Figure 24 (a)). The oil droplets seem to be coated by a coacervate phase. In some cases, concentric spheres are visible while in other cases the inner sphere shows horns. Finally, some groups of droplets seem to be surrounded by a coacervate film that groups them all together. This behaviour was already reported in the microencapsulation area in which oil droplets were surrounded by the coacervate complex prepared between a protein and a polysaccharide (NON-PTL 58). Cryo-SEM images of a selected emulsion show that both, the oil droplets and the continuous phase, are surrounded by frozen monodisperse spherical entities which are attributed to coacervate droplets of 154 nm of diameter (Figure 24 (b)). The size of the coacervate droplets has been checked through dynamic light scattering for this concentration of polyelectrolytes (5 g $L^{-1}$) and is 187 nm. This is in agreement with the sizes measured from the cryo-SEM images.

In order to evaluate an enhance on emulsion stability by increasing the concentration of the initial PEL solutions, a PEC aqueous dispersion prepared from the 30 g $L^{-1}$ individual PEL solutions at pH = 10 was prepared (Figure 25 (a)). The solution show the same turbidity compared to the one prepared from the 5 g $L^{-1}$ PEL solutions. However, a viscous coacervate phase coalesced on the bottom on the vial, around the walls and around the magnetic bar as seen from the upside-down vial in Figure 25 (a). Spherical cocacervate droplets were visible through optical microscope images (Figure 25 (c). However, upon the addition of oil and homogenization with the Ultra-turrax, the head of the homogenizer was covered by the coacervate phase and the oil on the top was mixed with a viscous phase without being emulsified. The aqueous phase after homogenization was depleted in coacervate droplets (Figure 25 (b, e)).

Therefore, again, the complex coacervation phenomena is a very sensitive system and the study of emulsion stabilization has to be optimized for each individual pair of polyelectrolytes.

As the most promising results on emulsion stabilisation were achieved from the PEC aqueous dispersions prepared from the 5 g $L^{-1}$ PEL solutions ($x_{PAANa}$ = 0.5) by stepwise addition of oil, the emulsion stability with different oils (squalane, isopropyl myristate, PDMS and toluene) was evaluated. In this case, different behaviours were obtained depending on the oil used. Isopropyl myristate and Squalane completely phase separate in less than one hour after preparation (Figure 26). PDMS also starts coalescing one day after preparation but the optical microscope images of the emulsion once creaming stopped, reveals the same kind of behaviour as n-dodecane: oil droplets surrounded by a coacervate phase (Figure 27). For toluene, the emulsion was stable for a longer time compared to the other oils and the optical microscope images reveal a different morphology of the oil droplets (Figure 28). The droplets were more deformed and stretched.

In the microencapsulation area, the complex coacervates suitable for microencapsulation have to spontaneously spread over the surface of dispersed liquid droplets or particles thereby totally engulfing and coating them. This occurs if the engulfing process reduces the interfacial free energy and this happens when a coacervate adsorbs on the surface of dispersed oil droplets/solid particles being encapsulated. Therefore, the adsorption process is a key step in the encapsulation practice. This can be evaluated with the calculation of three spreading coefficients (equations 20 to 22, Introduction) one for each phase that takes part on the process (oil phase, continuous phase and coacervate phase). Depending on the sign combinations of these three spreading coefficients, three different scenarios are encountered: complete engulfing, partial engulfing and no engulfing. Among all oils tested in this study we might have encountered examples of complete engulfing (n-dodecane and toluene) and no engulfing (isopropyl myristate and squalane). The analysis is first accomplished for n-dodecane but needs to be repeated for the rest of the oils.

In order to do so, the equations of the three spreading coefficients stated in the introduction section were resolved. For it, three interfacial tensions have to be considered: $\gamma_{23}$, $\gamma_{12}$ and $\gamma_{13}$. For our convenience and from now on, they are designed as: $\gamma_{WC}$, $\gamma_{OW}$ and $\gamma_{OC}$, respectively. The $\gamma_{OW}$ was measured with the tensiometer and the Du Nouy ring method and the average of three measurements is 53.3 mN $m^{-1}$ at 25°C, which is in agreement with the literature value (53.7 mN $m^{-1}$ at 22 °C) (NON-PTL 59). The other two interfacial tensions $\gamma_{WC}$ and $\gamma_{OW}$ can be calculated by solving

the following Young equations:

$$\gamma_{CG} = \gamma_{WC} + \gamma_{WG} \cos \theta_{WG} \qquad \text{...equation 27}$$

$$\gamma_{CG} = \gamma_{OC} + \gamma_{OG} \cos \theta_{OG} \qquad \text{...equation 28}$$

From those equations, $\gamma_{WG}$ was measured with the tensiometer and the Du Nouy ring method. The average of three measurements resulted to be 72.1 mN $m^{-1}$ at 25 °C, which is in agreement with the literature value (71.99 at 25 °C) (NON-PTL 60). The $\gamma_{OG}$ value of equals to 24.9 mN $m^{-1}$ and is taken from NON-PTL 61. $\theta_{WG}$ and $\theta_{OG}$ are measured with the DSA by placing a drop of water or n-dodecane onto a glass slide coated by the coacervate phase. The film of coacervate phase placed on the glass slide after water removal was completely transparent and homogeneous. $\theta_{WG}$ has a value of 46.95° and n-dodecane completely spreads so $\theta_{WG}$ is considered to be < 5°. $\gamma_{CG}$ is the surface free energy of the coacervate phase and can be either found on the literature for some systems or estimated by using a modified Young's equation. As the value of the coacervate phase of this specific combination of polyelectrolytes at pH =10 and $x_{PAANa}$ = 0.5 has not been reported before in the literature, it was measured. In the field of solid particles, an indirect method to estimate the surface energy of those particles has been utilised in many occasions. Here, we apply the same methodology to estimate the surface energy of the coacervate phase. This is the first time, to the best of our knowledge, that surface energy values of the coacervate phase are estimated with this approach. This method consists of splitting the surface energy of a material into a polar ($\gamma^p$) and a dispersant component ($\gamma^d$). As described earlier (NON-PTLs 62 and 63), the contact angle $\theta_{la}$ of a liquid (1) drop in air (a) on a perfectly smooth and homogeneous surface (s) using the Young equation can be written in terms of the polar and dispersion components of the surface energies of the liquid and solid as stated in equation 29. For convenience, the subscripts s and a have been changed by C and G, respectively.

$$\frac{1}{2}\gamma_{LG}(1 + \cos \theta_{LG}) = \sqrt{\gamma_{LG}^{d}\gamma_{CG}^{d}} + \sqrt{\gamma_{LG}^{p}\gamma_{CG}^{p}} \qquad \text{...equation 29}$$

where the subscripts L, G and C refers to liquid, gas and coacervate phases, respectively.

The two unknowns in this equation are $\gamma^d_{CG}$ and $\gamma^p_{CG}$ and can be determined by solving two of the above equations simultaneously with the values of two different oils. However, it is advisable to use more than two oils of different polarity. For this purpose, water, glycerol, formamide, $\alpha$-bromonaphthalene and n-hexadecane were used. Values of the terms of $\gamma^d_{LG}$ and $\gamma^p_{LG}$ for the different oils are given in Table 2 together with the liquid-air contact angles (averaged from three measurements) on a glass slide covered with the coacervate phase.

Table 2

| Liquid | Tension/mN $m^{-1}$ | | | Liquid-air contact angle on the coacervate phase/° |
|---|---|---|---|---|
| | $\gamma^d_{LG}$ | $\gamma^p_{LG}$ | $\gamma_{LG}$ | |
| Water | 21.6 | 50.5 | 72.1 | 49.6 ± 3 |
| Glycerol | 34.0 | 30.0 | 64.0 | 55.3 ± 9 |
| Formamide | 39.0 | 19.0 | 58.0 | 38.1 ± 5 |
| $\alpha$-bromonahthalene | 44.4 | 0.0 | 44.4 | 27.0 ± 3 |
| *n*-hexadecane | 27.8 | 0.0 | 27.8 | < 5 |

The least squares calculation was carried out to determine the best combination of $\gamma^d_{CG}$ and $\gamma^p_{CG}$ that fits all the data simultaneously. The 3-D surface energy diagram so obtained is shown in Figure 29. This is a plot of the goodness of fit (inverse of the sum of the squares of variances) to contact angle set against a matrix of possible values of $\gamma^d_{CG}$ and $\gamma^p_{CG}$. The values that best fit all the contact angles are read from the coordinates that define the peak in the chart. These values are $\gamma^d_{CG}$ = 42.63 mN $m^{-1}$ and $\gamma^p_{CG}$ = 4.32 mN $m^{-1}$. Therefore, $\gamma_{CG}$ = 46.95 mN $m^{-1}$. The surface energy for the hydrophilic Crown glass (NON-PTL 65), was estimated to be 76 mN $m^{-1}$. Conversely, for a very hydrophobic

surface like PTFE, the surface energy was estimated to be 18 mN $m^{-1}$ (NON-PTL 64). Therefore, from the data obtained, the complex coacervate is found to be quite hydrophobic due to its relatively high dispersion contribution.

By solving equation 27 with the given data, $\gamma_{WC}$ = -1.29, which is assumed to be zero for the calculations. This is in agreement with the literature that constantly reports ultra-low values for the interfacial tension of the coacervate phase against the continuous aqueous phase. As stated in the introduction section, the system of coacervate droplets dispersed in an aqueous environment can be considered as a w/w microemulsion. In microemulsions, one of the most important properties is the ultra-low interfacial tension, which is confirmed with this result. The mechanism that leads to this ultra-low interfacial tension has not been encountered in the literature but might be related to the high viscosity of the coacervate phase. Moreover, with this result, the assumption of $\gamma_{WC} < \gamma_{OW}$ stated for the derivation of the three combinations of spreading coefficients is also verified. By solving equation 28, $\gamma_{OC}$ is found to be 22.05 mN $m^{-1}$.

Therefore, by substituting all this data in the initial equations of the spreading coefficients, the following values are calculated for n-dodecane: $S_1$ = -75.35 mN $m^{-1}$, $S_2$ = - 31.25 mN $m^{-1}$ and $S_3$ = 31.25 mN $m^{-1}$. Therefore, these S fulfil the conditions for complete engulfing.

The calculation has to be repeated for the other oils. For toluene the same situation should be encountered while for squalane and isopropyl myristate, conditions for no engulfing should be accomplished.

## 4. CONCLUSIONS

The PEC system prepared from the mixture between PAANa and PDADMAC, a strong and a weak polyelectrolyte, is highly dependent on the pH of the media. Two types of associative phase separation (precipitation and complex coacervation) have been encountered along all the studied pH range. Despite precipitates are expected to be formed when the interactions between the PELs are strong, at high pH when both polyelectrolytes are fully ionized, complex coacervation is achieved at all mole fractions. In this case, this weak interaction is explained by the formation of hydrogen bonds between water molecules and the carboxylate groups that might weaken the electrostatic interaction with the quaternary amines in PDADMAC.

At low pHs no stable emulsions were achieved. At intermediate pHs and high PEC concentration (30 g $L^{-1}$), the coacervate complex was collected on the head of the homogeniser, not taking part in emulsion stabilization. At pH = 10 and at specific PEC concentration, stable emulsions were achieved by the addition of oil stepwise despite the fact that some oil is released along the time. The three spreading coefficients calculated for n-dodecane proved that the complex coacervate completely engulfs the oil phase. This morphology is confirmed through optical microscope images that show droplets coated, presumably, by the coacervate phase. Using cryo-SEM, we observe spherical and monodisperse coacervate droplets located at the oil-water interface of emulsion drops as well as in the water continuous phase. The coacervate system is sensitive to the oil used. Determination of the three spreading coefficients for the rest of the oils would be useful to prove the different behaviour regarding the capsule morphology.

## SECTION 3- FUTURE WORK

Calculation of the combination of the three spreading coefficients for the other oils: toluene, isopropyl myristate, PDMS and squalane to predict the morphology of the obtained capsules and check if they are in agreement with the experimental results.

Until now, emulsions were prepared immediately after PEC formation. It might be interesting to evaluate emulsion formation from the PEC aqueous dispersions after letting it to stand for 2 months when the coacervate droplets are partially sedimented on the bottom of the vial.

Study new PEC systems obtained using different combinations of strong and weak polyelectrolytes to try to elucidate a pattern of behaviour in emulsions stabilized by polyelectrolyte complexes. Evaluate the type of associative phase separation obtained through optical microscopy and characterize the obtained entities through dynamic light scattering to determine the size and the zeta potential. Emulsions with the new PEC systems will be prepared and their ability to stabilize emulsions will be assessed. The potential PEL combinations to study could be:

1. poly(ethyleneimine)(PEI) and PSSNa (weak(+) and strong(-))

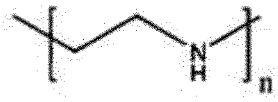

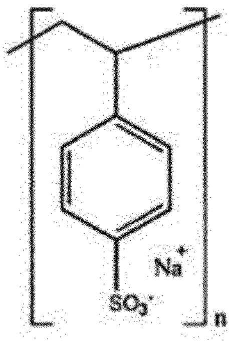

2. PDADMAC and poly(vinyl sulfate) potassium salt (PVSK) (strong(+) and weak(-))

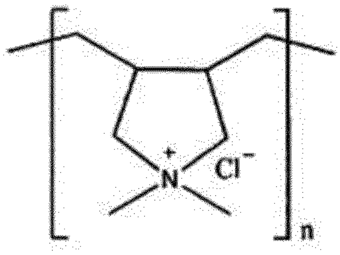

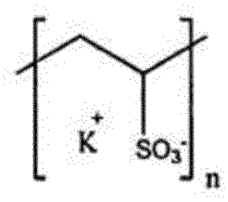

3. poly(allylamine hydrochloride) (PAH) and PAANa (weak(+) and weak(-))

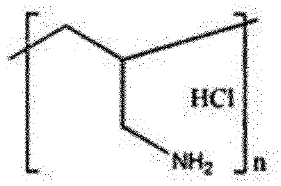

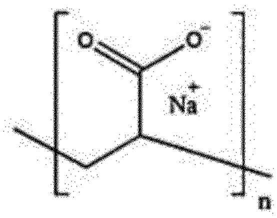

Among all the polyelectrolytes suggested, PEI exists in either a branched or linear morphological structure. Linear polyethylenimines contain all secondary amines (Figure 31), in contrast to branched PEIs which contain primary, secondary and tertiary amino groups (Figure 32). Branched PEI has a high density of amine groups. The unprotonated amines can absorb protons as the pH decreases.

Conclusions of Emulsions stabilized by polyelectrolyte complexes (PEC) in Figs. 37 to 46C

Polyelectrolyte complexes prepared with PSSNa and PDADMAC can stabilize oil-water emulsions while polyelectrolytes alone can not.

The average drop size can be tuned by changing $x_{PSSNa}$ and the initial polyelectrolyte concentration.

From cryo-SEM, particles are located at the oil-water interface of emulsion drops.

The addition of salt to the system has a remarkable influence on both the PEC aqueous dispersions and emulsions.

Matters regarding Fig. 47: Regimes in particle-stabilized emulsions depending on the [emulsifier]

Fewer [emulsifier] results in an emulsifier-poor regime, and the mean drop size is decreased with [emulsifier].

Higher [emulsifier] results in an emulsifier-rich regime, and the mean drop size is independent of [emulsifier].

Embodiments of the present disclosure separately include the following:

[Embodiment 1]

Oil-in-water emulsion, comprising a water phase as a matrix, and an oil phase dispersed therein and stabilized by a coacervate of an anionic polyelectrolyte and a cationic polyelectrolyte.

[Embodiment 2]

The oil-in-water emulsion of embodiment 1 above, wherein the anionic polyelectrolyte is a strong anionic polyelectrolyte having a pKa of 1 or less, and the cationic polyelectrolyte is a strong cationic polyelectrolyte having a pKb of 1 or less.

[Embodiment 3]

The oil-in-water emulsion of embodiment 2 above, wherein the strong anionic polyelectrolyte is one or more selected from a group consisting of poly(aryl sulfonic acid) and salts thereof, and the strong cationic polyelectrolyte is one or more selected from a group consisting of poly(quaternary ammonium salts).

[Embodiment 4]

The oil-in-water emulsion of embodiment 3 above, wherein the strong anionic polyelectrolyte is poly(styrene sulfonate) sodium salt, and the strong cationic polyelectrolyte is poly(diallyldimethyl ammonium chloride).

[Embodiment 5]

The oil-in-water emulsion of embodiment 1 above, wherein the anionic polyelectrolyte is a weak anionic polyelectrolyte having a pKa of more than 1, and the cationic polyelectrolyte is a strong cationic polyelectrolyte having a pKb of 1 or less.

[Embodiment 6]

The oil-in-water emulsion of embodiment 5 above, wherein the weak anionic polyelectrolyte is one or more selected from a group consisting of poly(carboxylic acid), poly(allyl sulfonic acid), and salts thereof; and the strong cationic polyelectrolyte is one or more selected from a group consisting of poly(quaternary ammonium salts).

[Embodiment 7]

The oil-in-water emulsion of embodiment 6 above, wherein the weak anionic polyelectrolyte is poly(acrylic acid) sodium salt, alginic acid, or poly(vinyl sulfate) potassium salt, and the strong cationic polyelectrolyte is poly(diallyldimethyl ammonium chloride).

[Embodiment 8]

The oil-in-water emulsion of embodiment 1 above, wherein the anionic polyelectrolyte is a strong anionic polyelectrolyte having a pKa of 1 or less, and the cationic polyelectrolyte is a weak cationic polyelectrolyte having a pKb of more than 1.

[Embodiment 9]

The oil-in-water emulsion of embodiment 8 above, wherein the strong anionic polyelectrolyte is one or more selected from a group consisting of poly(aryl sulfonic acid) and salts thereof; and the weak cationic polyelectrolyte is one or more selected from a group consisting of poly(primary ammonium), poly(secondary ammonium), and salts thereof.

[Embodiment 10]

The oil-in-water emulsion of embodiment 9 above, wherein the strong anionic polyelectrolyte is poly(styrene sulfonate) sodium salt, and the weak cationic polyelectrolyte is poly(allylamine hydrochloride) or poly(ethylene imine).

[Embodiment 11]

The oil-in-water emulsion of embodiment 1 above, wherein the anionic polyelectrolyte is a weak anionic polyelectrolyte having a pKa of more than 1, and the cationic polyelectrolyte is a weak cationic polyelectrolyte having a pKb of more than 1.

[Embodiment 12]

The oil-in-water emulsion of embodiment 11 above, wherein the weak anionic polyelectrolyte is one or more selected from a group consisting of poly(carboxylic acid), poly(allyl sulfonic acid), and the salts thereof; and the weak cationic polyelectrolyte is one or more selected from a group consisting of poly(primary ammonium), poly(secondary ammonium), and salts thereof.

[Embodiment 13]

The oil-in-water emulsion of embodiment 12 above, wherein the weak anionic polyelectrolyte is poly(acrylic acid) sodium salt, alginic acid, or poly(vinyl sulfate) potassium salt; and the weak cationic polyelectrolyte is poly(allylamine hydrochloride) or poly(ethylene imine).

[Embodiment 14]

The oil-in-water emulsion of any one of embodiments 1 to 13 above, which is used as a cosmetic composition.

[Embodiment 15]

A method for applying the oil-in-water emulsion of any one of embodiments 1 to 14 above to a human skin.

[Embodiment 16]

Use of the oil-in-water emulsion of any one of embodiments 1 to 13 above, as a cosmetic composition.

[Embodiment 17]

The oil-in-water emulsion embodiment 14 above, the method of embodiment 15 above, or the use of embodiment 16 above, wherein the emulsion contains one or more the powder components listed below.

[Embodiment 18]

The oil-in-water emulsion embodiment 14 above, the method of embodiment 15 above, or the use of embodiment 16 above, wherein the oil phase contains one or more the oil phase components listed below.

[Embodiment 19]

The oil-in-water emulsion embodiment 14 above, the method of embodiment 15 above, or the use of embodiment 16 above, wherein the water phase contains one or more the water phase components listed below.

Powder components

Examples of the powder component contained in the oil-in-water emulsion composition of the present disclosure include inorganic powders (such as talc, kaolin, mica, sericite, muscovite, phlogopite, synthetic mica, red mica, biotite, vermiculite, magnesium carbonate, calcium carbonate, aluminum silicate, barium silicate, calcium silicate, magnesium silicate, strontium silicate, tungsten acid metal salt, magnesium, silica, zeolite, barium sulfate, calcinated calcium sulfate (calcined gypsum), calcium phosphate, fluorine apatite, hydroxyapatite, and ceramic powder), metal soap (such as zinc myristate, calcium palmitate, aluminium stearate), and boron nitride; organic powders (such as titanium dioxide and zinc oxide); inorganic red series pigments (such as iron titanate); inorganic purple series pigments (such as mangoviolet and cobaltviolet); inorganic green series pigments (such as chromium oxide, chromium hydroxide, and cobalt titanate); inorganic blue series pigments (such as ultramarine and Prussian blue); pearl pigments (such as titanium oxide-coated mica, titanium oxide-coated bismuth oxychloride, titanium oxide-coated talc, colored titanium oxide-coated mica, bismuth

oxychloride, and fish scale foil); metal powder pigments (such as aluminum powder and copper powder); organic pigments such as zirconium, barium, or aluminum lake (e.g., organic pigments such as Red No.201, Red No.202, Red No.204, Red No.205, Red No.220, Red No.226, Red No.228, Red No.405, Orange No.203, Orange No.204, Yellow No.205, Yellow No.401 and Blue No.404, Red No.3, Red No. 104, Red No. 106, Red No.227, Red No.230, Red No.401, Red No.505, Orange No.205, Yellow No.4, Yellow No.5, Yellow No. 202, Yellow No.203, Green No.3, and Blue No.1); and natural colorants (such as chlorophyll and β-carotene). One of them can be used alone, or two or more of them can be used in combination. Also, in the present disclosure, the powder component may be a composite powder which is prepared by coating powder with metal oxide or a modified powder which is prepared by the surface treatment of the powder with compounds.

In the present disclosure, it is preferable to use silica, titanium dioxide, zinc oxide, or a composite powder thereof. In particular, silica-coated zinc oxide and silica-coated titanium oxide are preferable from the standpoint of affinity of skin, usability, UV-shielding effect, and emulsion stability.

The particle size of the powder is not limited in particular. However, the particle size is preferably 1 to 200 nm from the standpoint of handling easiness and emulsion stability when the powder is blended into cosmetics.

The blending quantity of the powder component in the oil-in-water emulsion of the present disclosure is preferably 1 to 20 mass % with respect to the total amount of the oil-in-water emulsion composition, and especially preferably 1 to 10 mass %. If the blending quantity is less than 1 mass %, the emulsification may not proceed satisfactorily. If the blending quantity exceeds 20 mass %, the sticky feeling tends to increase.

Oil phase components

Examples of the oil phase components contained in the oil-in-water emulsion composition of the present disclosure are listed in the following.

Examples of liquid oil include avocado oil, camellia oil, turtle oil, macadamia nut oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, paulownia oil, Japanese tung oil, jojoba oil, germ oil, and triglycerin.

Examples of solid oil include cacao butter, coconut oil, horse fat, hardened coconut oil, palm oil, beef tallow, mutton tallow, hardened beef tallow, palm kernel oil, pork tallow, beef bone tallow, Japan wax kernel oil, hardened oil, heatsfoot oil, Japan wax, and hardened castor oil.

Examples of wax include beeswax, candelilla wax, cotton wax, carnauba wax, bayberry wax, Chinese wax, spermaceti wax, montan wax, rice bran wax, lanolin, kapok wax, lanolin acetate, liquid lanolin, sugar cane wax, isopropyl lanolate, hexyl laurate, reduced lanolin, jojoba wax, hard lanolin, shellac wax, POE lanoline alcohol ether, POE lanoline alcohol acetate, POE cholesterol ether, lanolin fatty acid polyethylene glycol, and POE hydrogenated lanolin alcohol ether.

Examples of hydrocarbon oil include liquid paraffin, ozokerite, squalane, pristane, paraffin, ceresin, squalene, petrolatum, and microcrystalline wax.

Examples of higher fatty acid include lauric acid, myristic acid, palmitic acid, stearic acid, behenic acid, oleic acid, undecylenic acid, tolic acid, isostearic acid, linoleic acid, linolenic acid, eicosapentaenoic acid (EPA), and docosahexaenoic acid (DHA).

Examples of higher alcohol include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearyl glycerin ether (batylalcohol), 2-decyltetradecynol, lanolin alcohol, cholesterol, phytosterol, hexyldodecanol, isostearyl alcohol, and octyldodecanol).

Examples of synthetic ester oil include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentylglycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri(2-ethylhexanoate), glyceryl trioctanoate, glyceryl triisopalmitate, trimethylolpropane triisostearate, cetyl 2-ethylhexanoate, 2-ethylhexyl palmitate, glyceryl trimyristate, glyceride tri-2-heptylundecanoate, castor oil fatty acid methyl ester, oleyl oleate, acetoglyceride, 2-heptylundecyl palmitate, diisobutyl adipate, 2-octyldodecyl ester N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of silicone oil include linear polysiloxanes (such as dimethylpolysiloxane, methylphenylpolysiloxane, and diphenylpolysiloxane); cyclic polysiloxanes (such as octamethylcyclotetrasiloxane, decamethylcyclopentasiloxane, and dodecamethylcyclohexasiloxane); silicon resin forming three-dimensional network structure; silicone rubber; various kinds of modified polysiloxane (such as amino modified polysiloxane, polyether-modified polysiloxane, alkyl-modified

polysiloxane, and fluorine-modified polysiloxane); and acrylic silicones.

Water phase components

In the present disclosure, water phase include, besides water, lower alcohol, polyhydric alcohol, and so on.
Examples of lower alcohol include ethanol, propanol, isopropanol, isobutyl alcohol, and t-butyl alcohol.
Examples of polyhydric alcohol include dihydric alcohols (such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (such as glycerin and trimethylolpropane); tetrahydric alcohols (such as pentaerythritol, for example, 1,2,6-hexanetriol); pentahydric alcohols (such as xylitol); hexahydric alcohols (such as sorbitol and mannitol); polyhydric alcohol polymers (such as diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkyl ethers (such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether); dihydric alcohol alkyl ethers (such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerol monoalkyl ethers (such as chimyl alcohol, selachyl alcohol, and batyl alcohol); sugar alcohols (such as sorbitol, maltitol, maltotriose, mannitol, sucrose, erythritol, glucose, fructose, starch-degraded sugars, maltose, xylitose, and reduction alcohols of starch-degraded sugars); glysolid; tetrahydrofurfuryl alcohol; POE-tetrahydrofurfuryl alcohol; POP-butyl ether; POP/POE-butyl ether; tripolyoxypropylene glycerin ether; POP-glycerol ether; POP-glycerin ether phosphate; POP/POE-pentaerythritol ether; and polyglycerol.

INDUSTRIAL APPLICABILITY

The cosmetic composition of the present invention is preferably used as a cosmetic.

## Claims

1. A cosmetic composition, comprising a water phase as a matrix, and an oil phase dispersed therein and stabilized by a coacervate droplet of an anionic polyelectrolyte and a cationic polyelectrolyte, the coacervate droplet being one which can form a metastable water-in-water type emulsion.

2. The cosmetic composition of claim 1 above, wherein each polyelectrolyte is not surface active by its own.

3. The cosmetic composition of claim 1 or 2 above, wherein the anionic polyelectrolyte is a strong anionic polyelectrolyte having a pKa of 1 or less, and the cationic polyelectrolyte is a strong cationic polyelectrolyte having a pKb of 1 or less.

4. The cosmetic composition of claim 3 above, wherein the strong anionic polyelectrolyte is one or more selected from a group consisting of poly(aryl sulfonic acid) and salts thereof, and the strong cationic polyelectrolyte is one or more selected from a group consisting of poly(quaternary ammonium salts).

5. The cosmetic composition of claim 4 above, wherein the strong anionic polyelectrolyte is poly(styrene sulfonate) sodium salt, and the strong cationic polyelectrolyte is poly(diallyldimethyl ammonium chloride).

6. The cosmetic composition of claim 1 or 2 above, wherein the anionic polyelectrolyte is a weak anionic polyelectrolyte having a pKa of more than 1, and the cationic polyelectrolyte is a strong cationic polyelectrolyte having a pKb of 1 or less.

7. The cosmetic composition of claim 6 above, wherein the weak anionic polyelectrolyte is one or more selected from a group consisting of poly(carboxylic acid), poly(allyl sulfonic acid), and salts thereof; and the strong cationic polyelectrolyte is one or more selected from a group consisting of poly(quaternary ammonium salts).

8. The cosmetic composition of claim 7 above, wherein the weak anionic polyelectrolyte is poly(acrylic acid) sodium salt, alginic acid, or poly(vinyl sulfate) potassium salt, and the strong cationic polyelectrolyte is poly(diallyldimethyl ammonium chloride).

9. The cosmetic composition of claim 1 or 2 above, wherein the anionic polyelectrolyte is a strong anionic polyelectrolyte having a pKa of 1 or less, and the cationic polyelectrolyte is a weak cationic polyelectrolyte having a pKb of more than 1.

10. The cosmetic composition of claim 9 above, wherein the strong anionic polyelectrolyte is one or more selected from a group consisting of poly(aryl sulfonic acid) and salts thereof; and the weak cationic polyelectrolyte is one or more selected from a group consisting of poly(primary ammonium), poly(secondary ammonium), and salts thereof.

11. The cosmetic composition of claim 10 above, wherein the strong anionic polyelectrolyte is poly(styrene sulfonate) sodium salt, and the weak cationic polyelectrolyte is poly(allylamine hydrochloride) or poly(ethylene imine).

12. The cosmetic composition of claim 1 or 2 above, wherein the anionic polyelectrolyte is a weak anionic polyelectrolyte having a pKa of more than 1, and the cationic polyelectrolyte is a weak cationic polyelectrolyte having a pKb of more than 1.

13. The cosmetic composition of claim 12 above, wherein the weak anionic polyelectrolyte is at one or more selected from a group consisting of poly(carboxylic acid), poly(allyl sulfonic acid), and the salts thereof; and the weak cationic polyelectrolyte is at one or more selected from a group consisting of poly(primary ammonium), poly(secondary ammonium), and salts thereof.

14. The cosmetic composition of claim 13 above, wherein the weak anionic polyelectrolyte is poly(acrylic acid) sodium salt, alginic acid, or poly(vinyl sulfate) potassium salt; and the weak cationic polyelectrolyte is poly(allylamine hydrochloride) or poly(ethylene imine).

15. A method for applying the cosmetic composition of any one of claims 1 to 14 above to a human skin.

FIG. 1

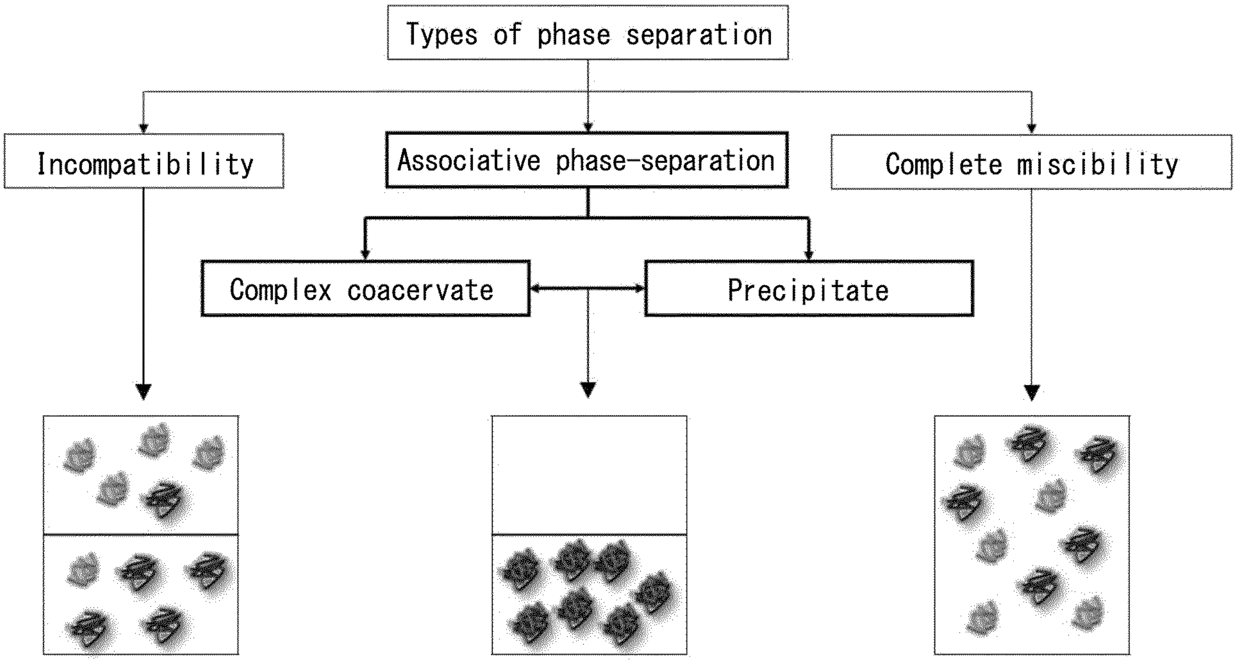
Types of phase separation
Incompatibility
Associative phase-separation
Complete miscibility
Complex coacervate
Precipitate

FIG. 2

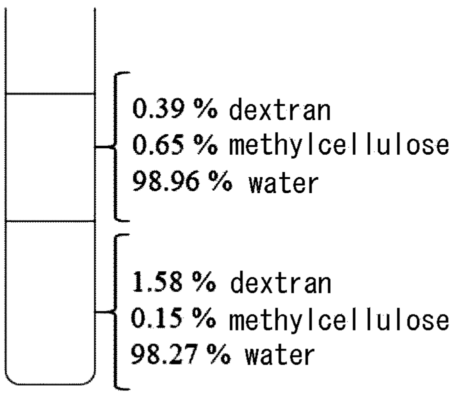
0.39 % dextran
0.65 % methylcellulose
98.96 % water
1.58 % dextran
0.15 % methylcellulose
98.27 % water

FIG. 3

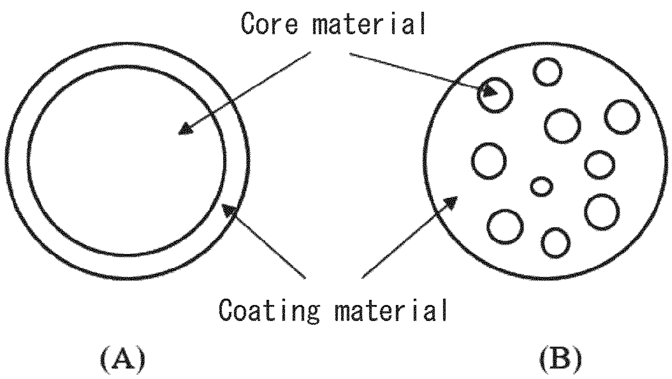


FIG. 4

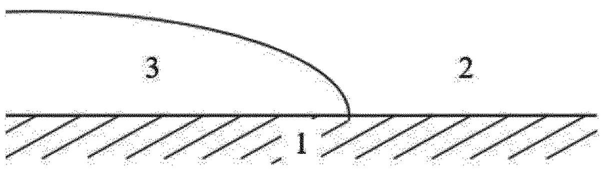


FIG. 5

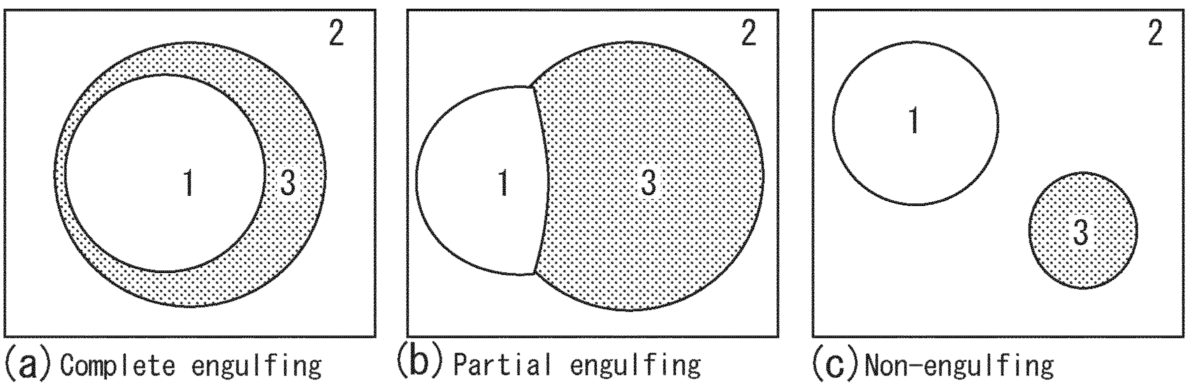


(a) Complete engulfing $S_1<0, S_2<0, S_3>0$

(b) Partial engulfing $S_1<0, S_2<0, S_3<0$

(c) Non-engulfing $S_1<0, S_2>0, S_3<0$

FIG. 6

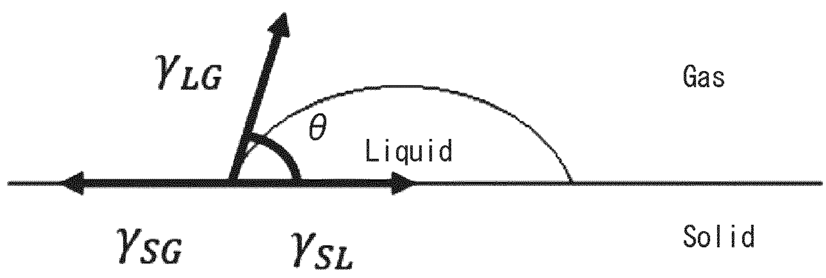
$\gamma_{LG}$
Gas
θ
Liquid
$\gamma_{SG}$
$\gamma_{SL}$
Solid

FIG. 7

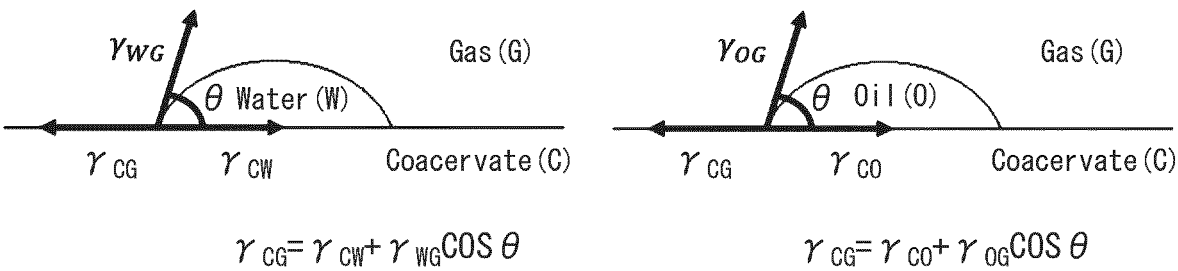
$\gamma_{WG}$
Gas (G)
θ Water (W)
$\gamma_{CG}$
$\gamma_{CW}$
Coacervate (C)
$\gamma_{CG} = \gamma_{CW} + \gamma_{WG} \cos\theta$
$\gamma_{OG}$
Gas (G)
θ Oil (O)
$\gamma_{CG}$
$\gamma_{CO}$
Coacervate (C)
$\gamma_{CG} = \gamma_{CO} + \gamma_{OG} \cos\theta$

FIG. 8

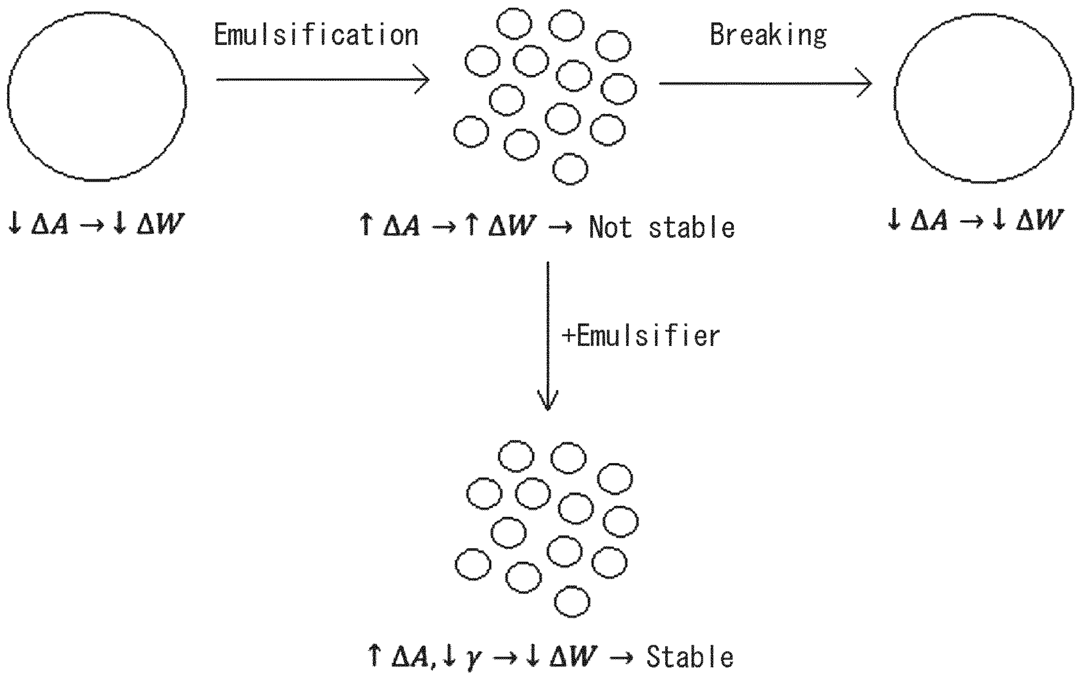
Emulsification
Breaking
↓ ΔA → ↓ ΔW
↑ ΔA → ↑ ΔW → Not stable
↓ ΔA → ↓ ΔW
+Emulsifier
↑ ΔA, ↓ γ → ↓ ΔW → Stable

FIG. 9

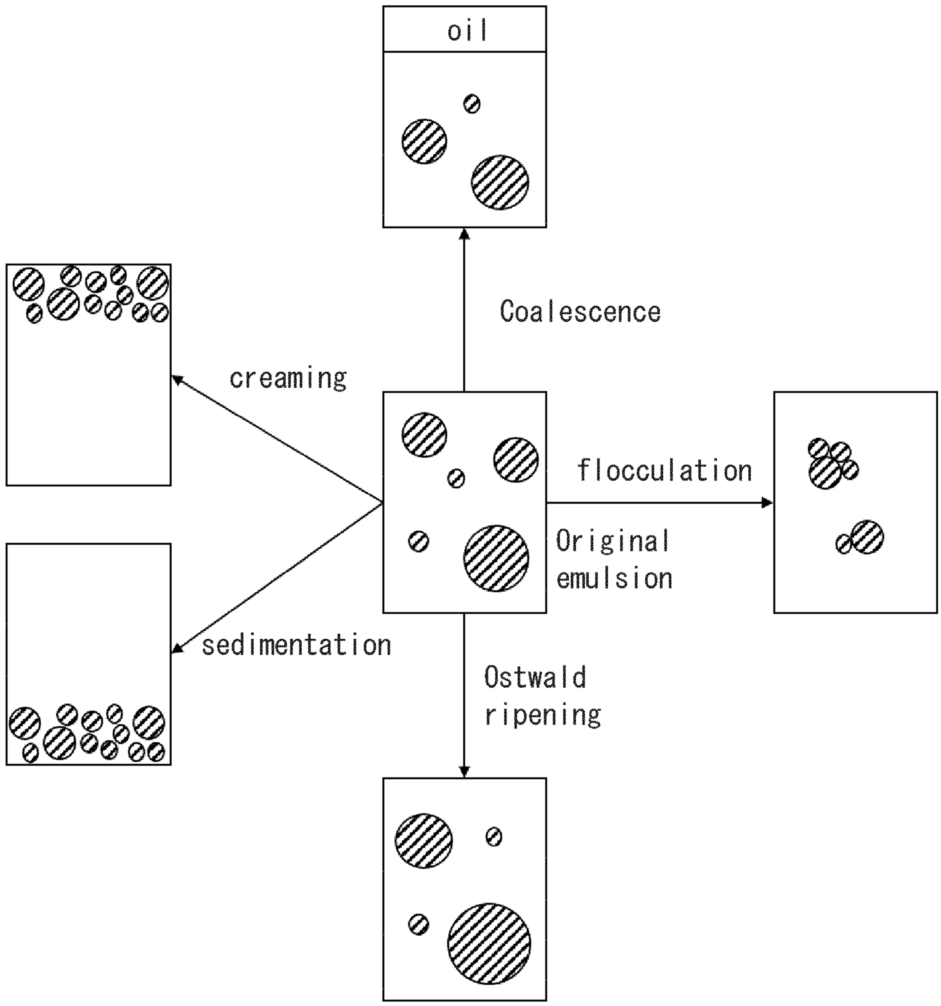
oil
Coalescence
creaming
flocculation
Original
emulsion
sedimentation
Ostwald
ripening

FIG. 10

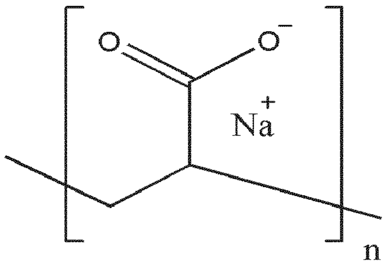

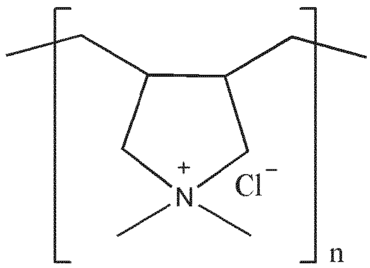

FIG. 11

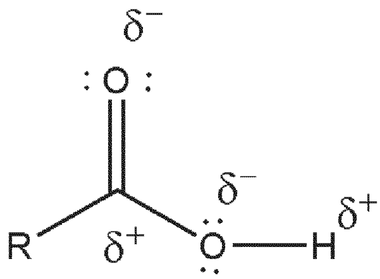

FIG. 12

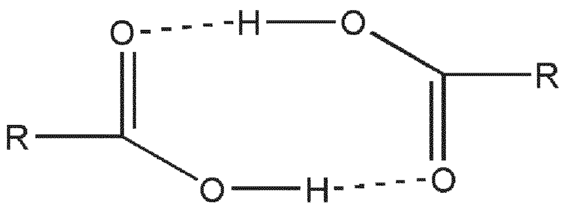

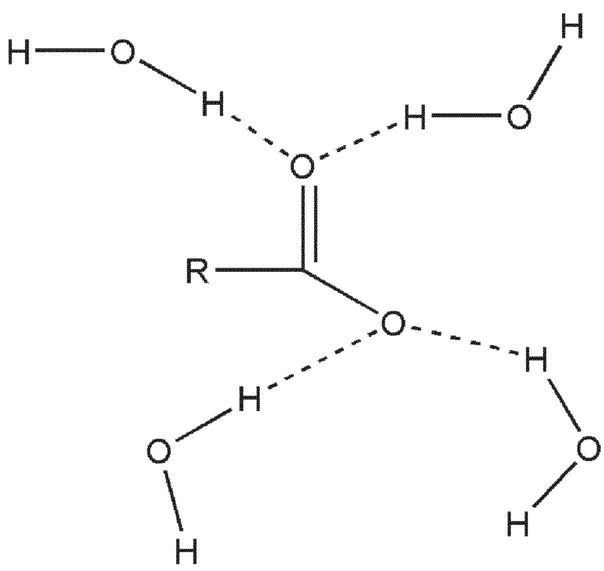

FIG. 13

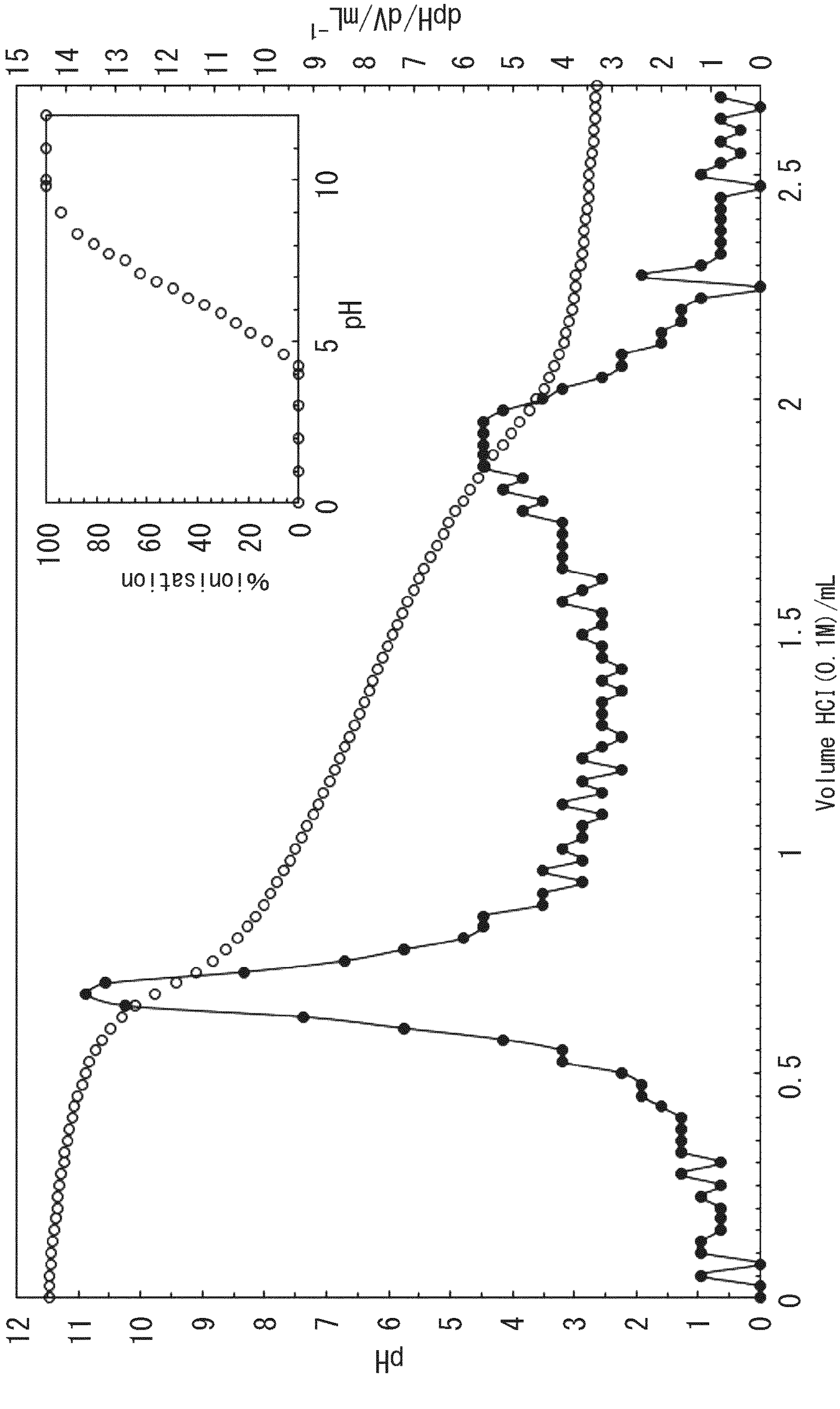

FIG. 14

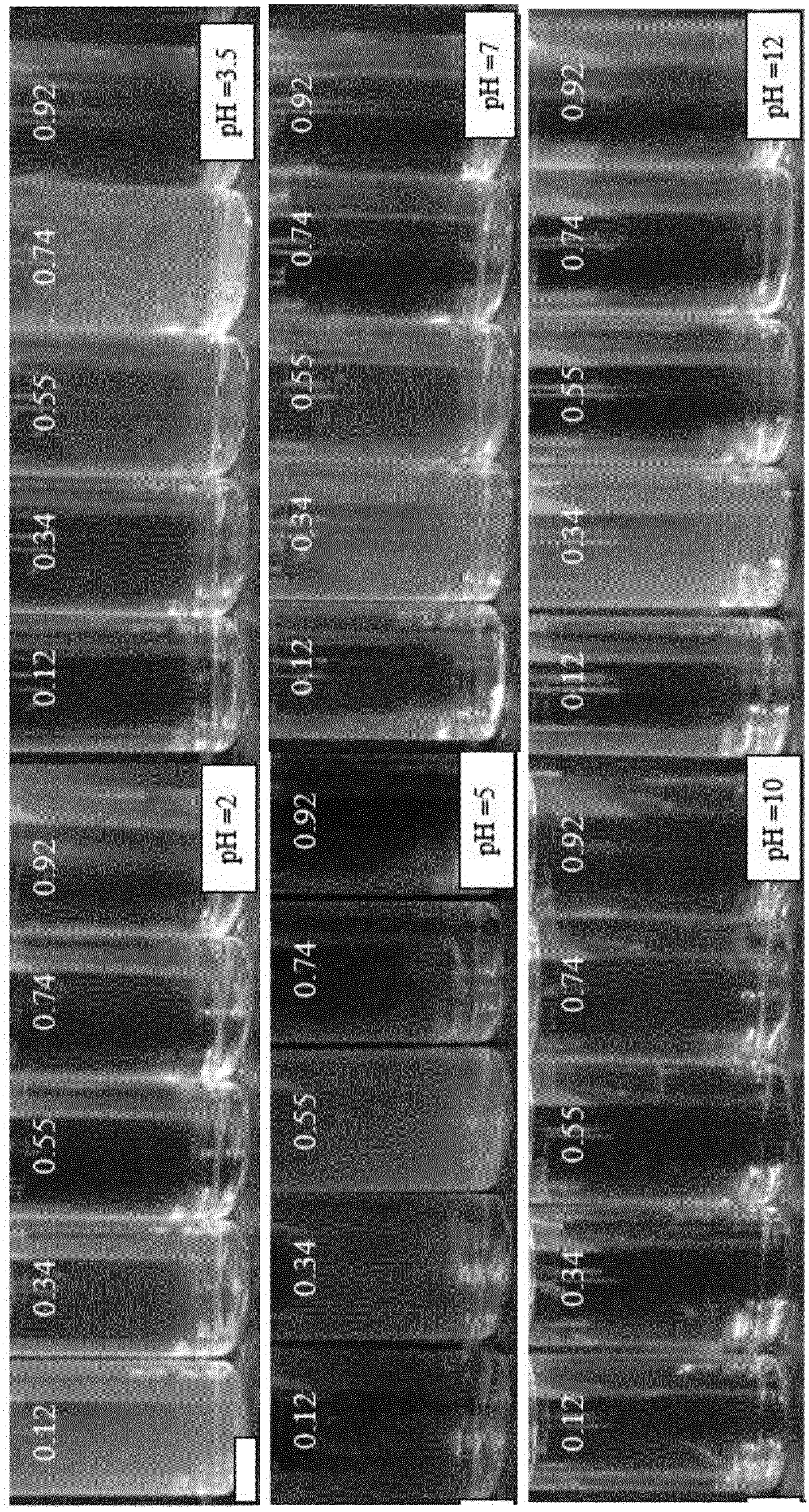
pH =2
0.12
0.34
0.55
0.74
0.92
pH =3.5
0.12
0.34
0.55
0.74
0.92
pH =5
0.12
0.34
0.55
0.74
0.92
pH =7
0.12
0.34
0.55
0.74
0.92
pH =10
0.12
0.34
0.55
0.74
0.92
pH =12
0.12
0.34
0.55
0.74
0.92

FIG. 15

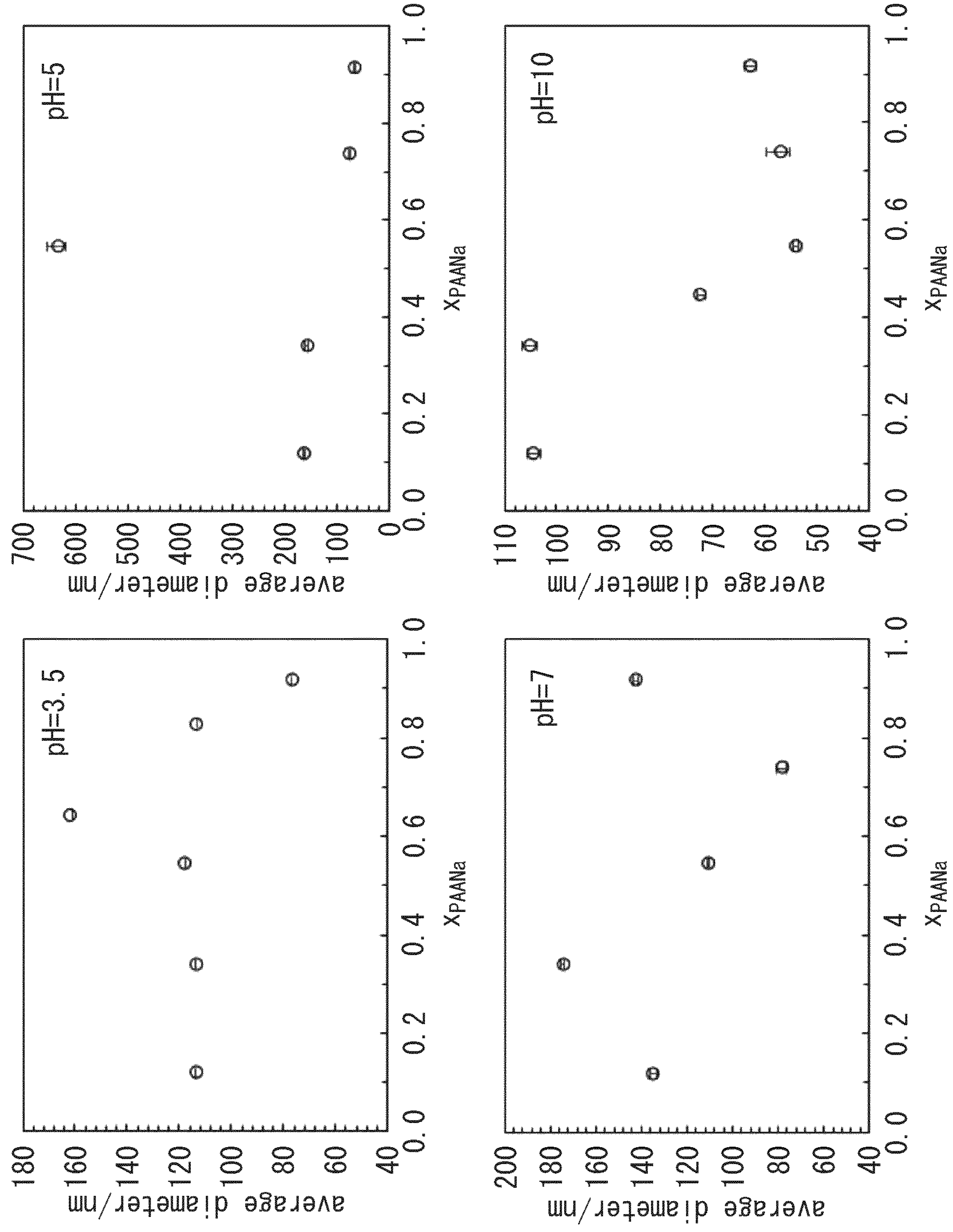

FIG. 16

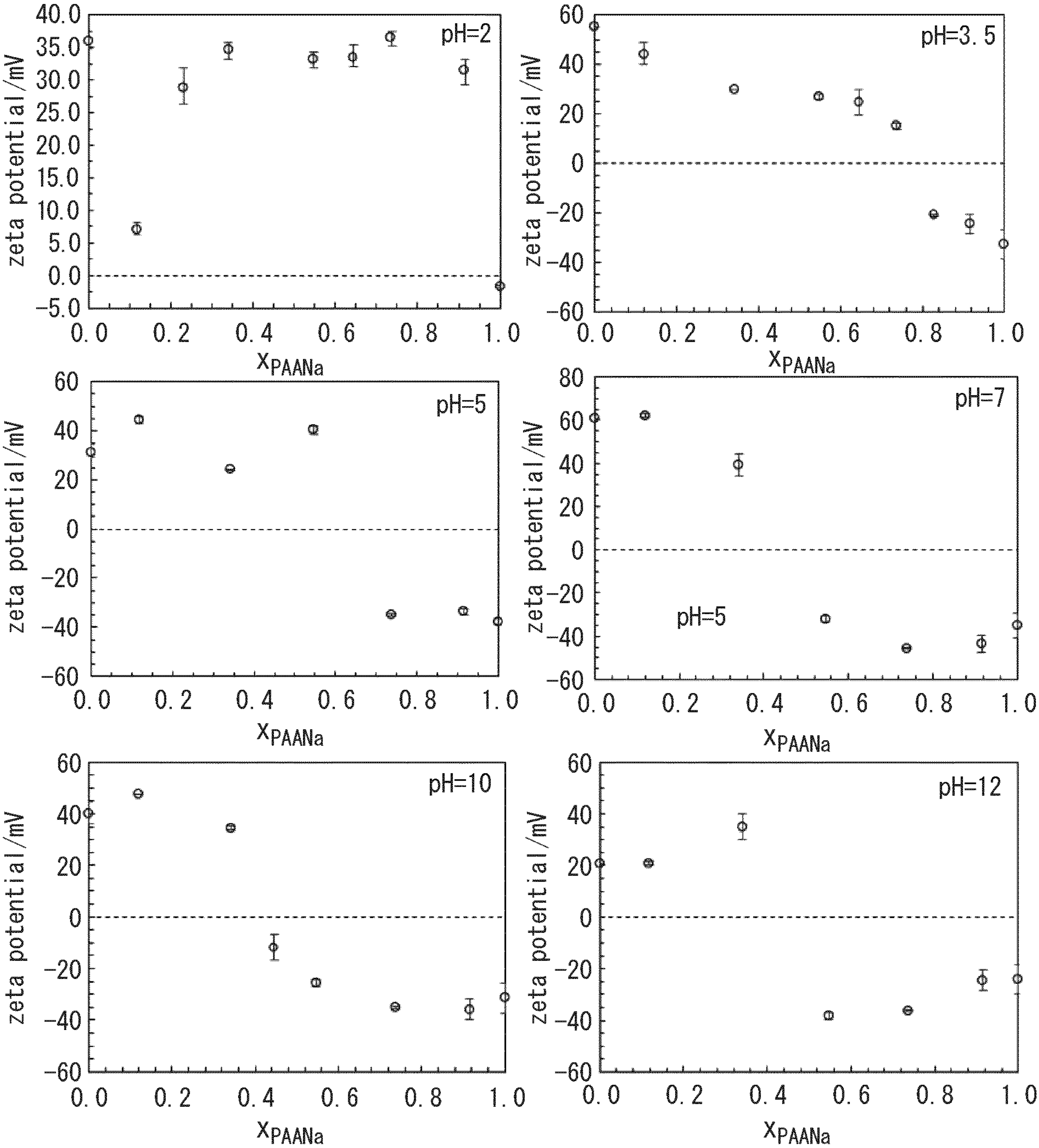

FIG. 17

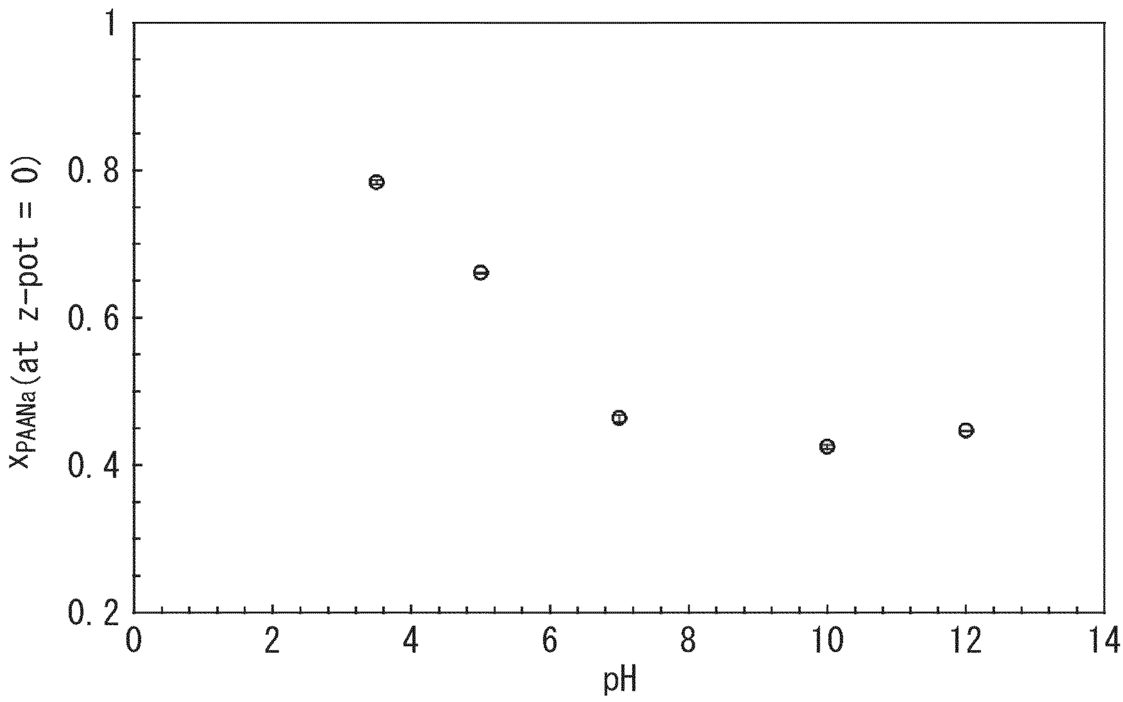

FIG. 18

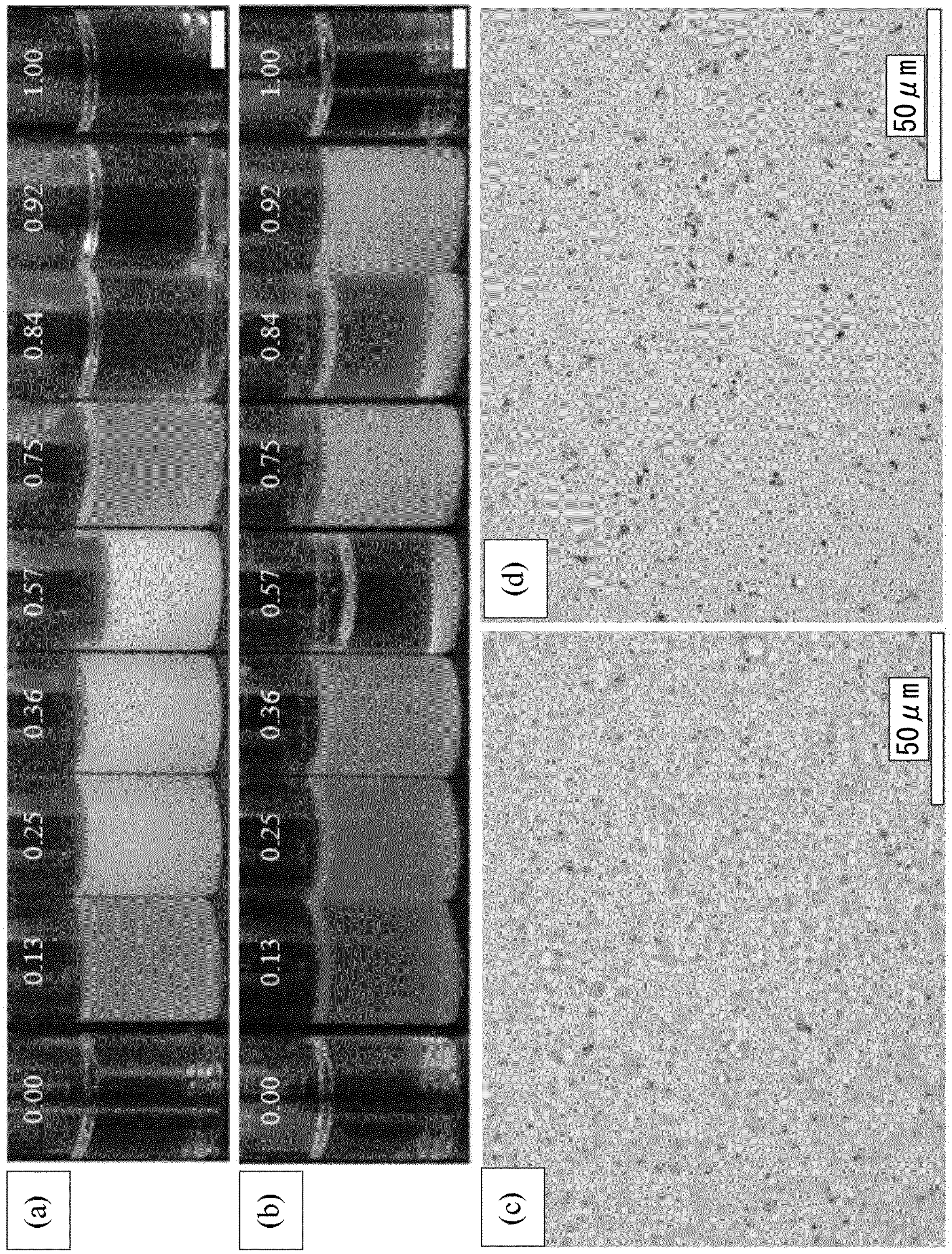
(a)
0.00
0.13
0.25
0.36
0.57
0.75
0.84
0.92
1.00
(b)
0.00
0.13
0.25
0.36
0.57
0.75
0.84
0.92
1.00
(c)
50 μm
(d)
50 μm

FIG. 19

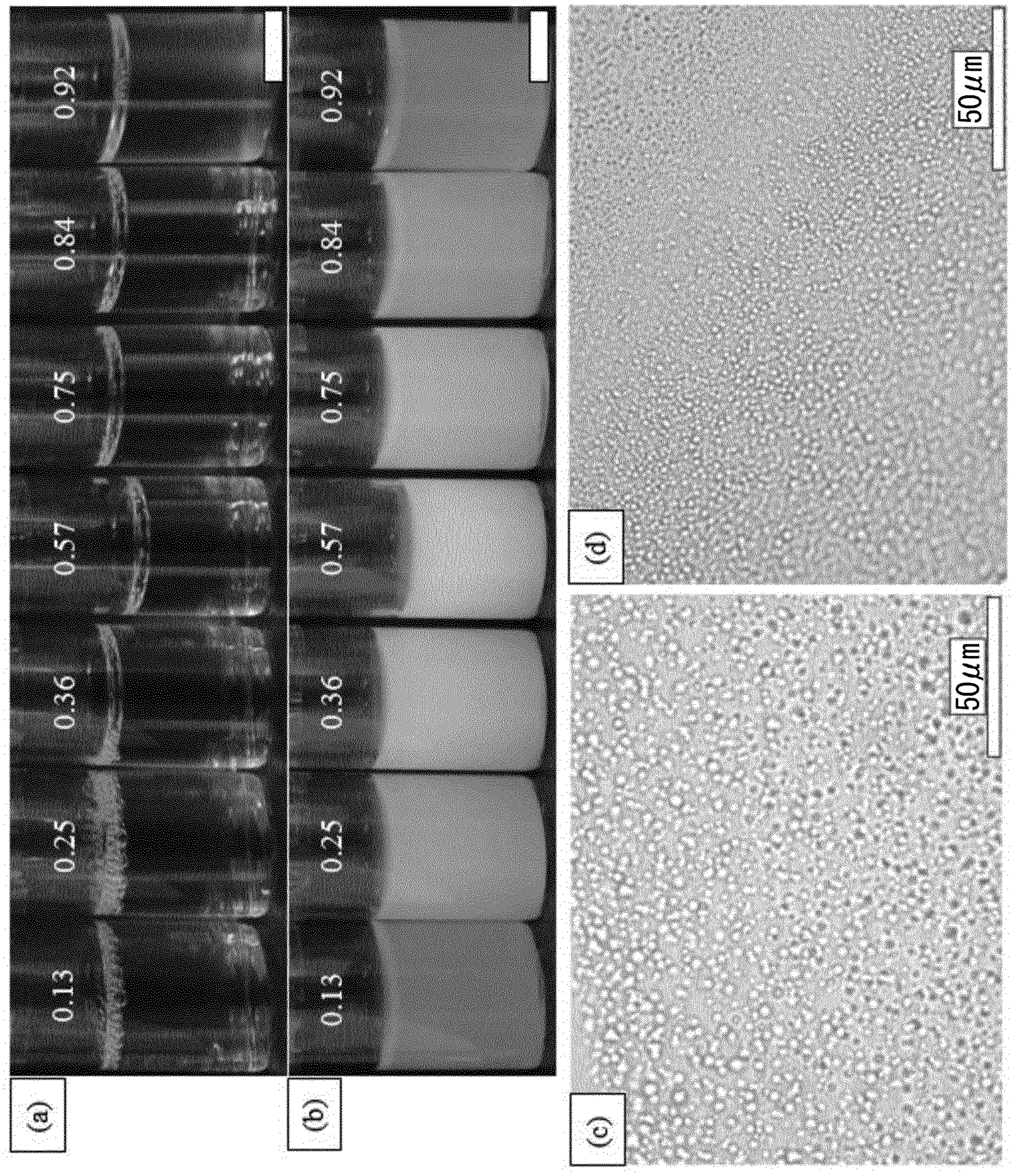
(a)
0.13
0.25
0.36
0.57
0.75
0.84
0.92
(b)
0.13
0.25
0.36
0.57
0.75
0.84
0.92
(c)
50μm
(d)
50μm

FIG. 20

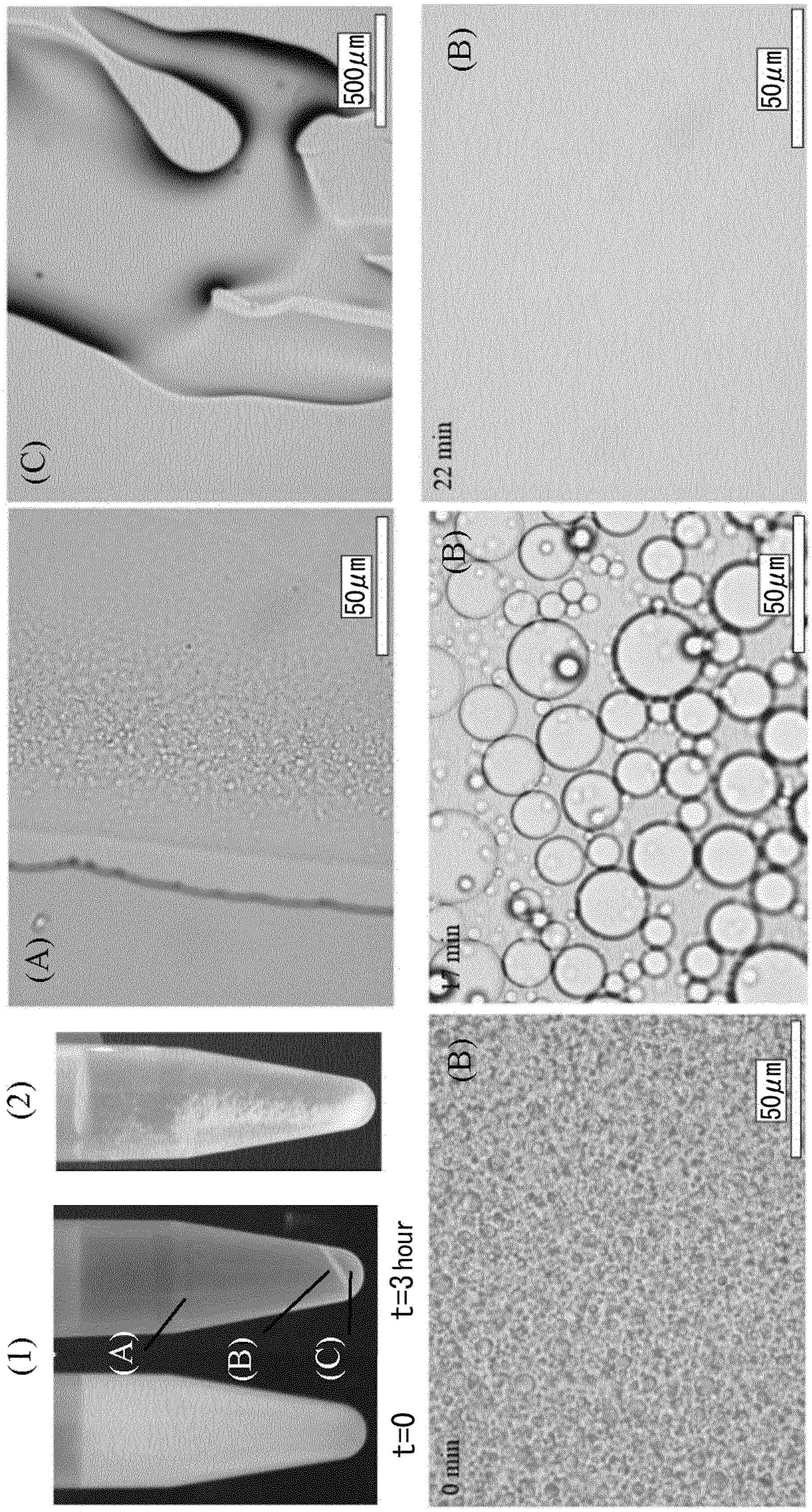

FIG. 21

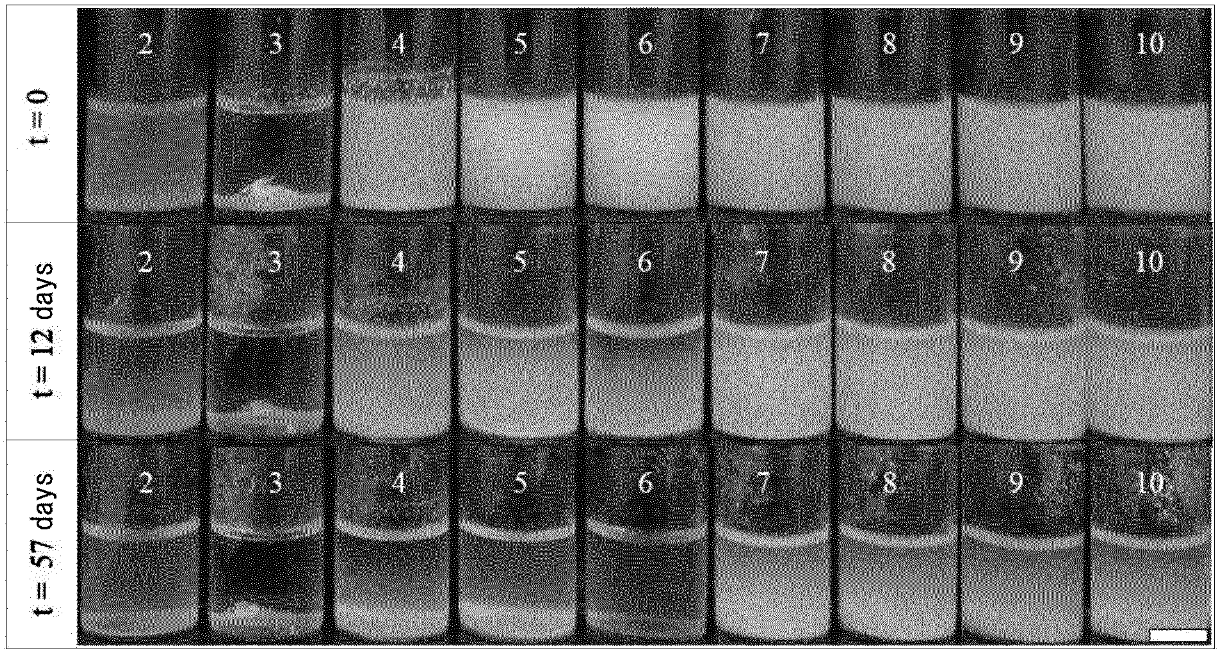

FIG. 22

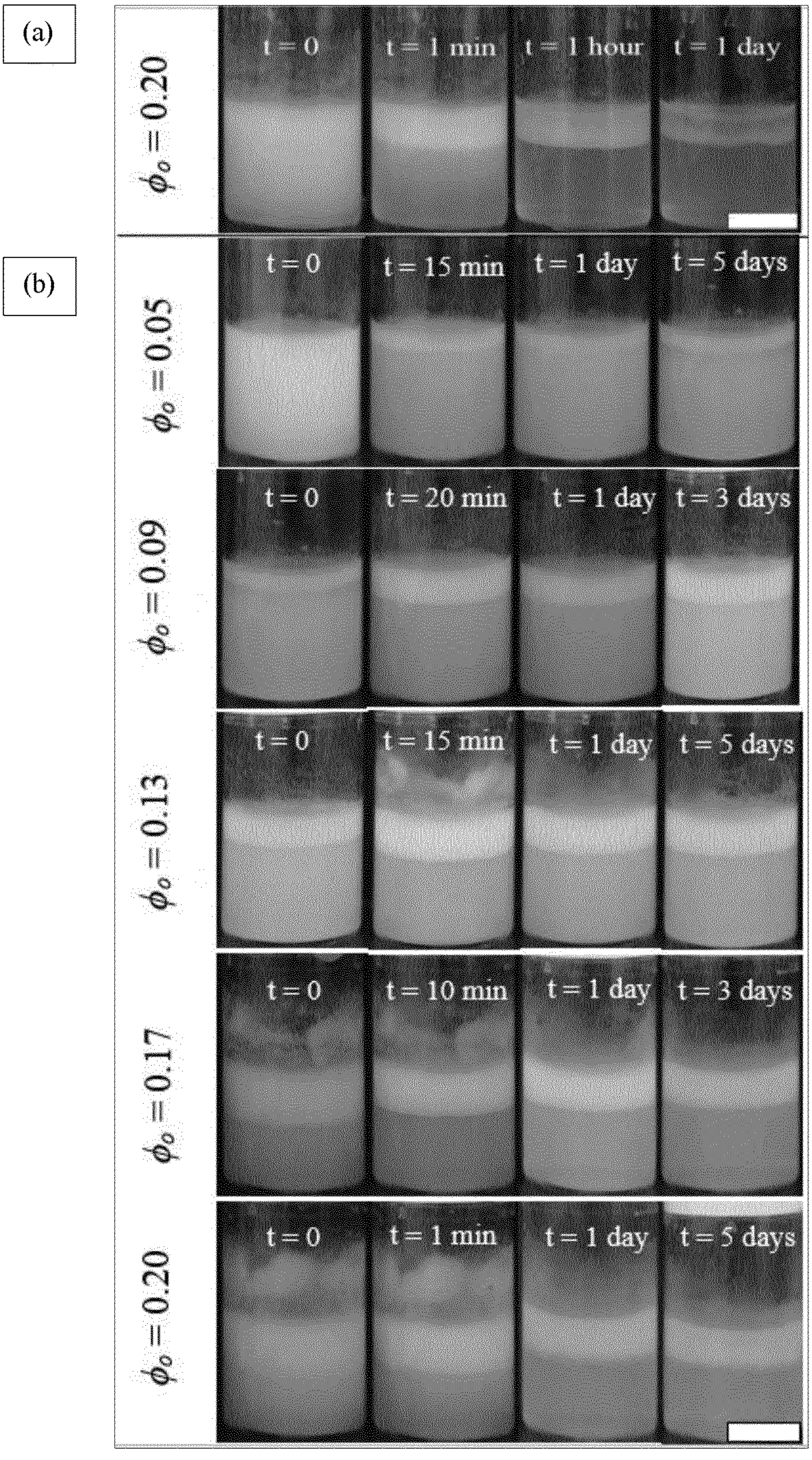
(a)
$\phi_o = 0.20$
t = 0
t = 1 min
t = 1 hour
t = 1 day
(b)
$\phi_o = 0.05$
t = 0
t = 15 min
t = 1 day
t = 5 days
$\phi_o = 0.09$
t = 0
t = 20 min
t = 1 day
t = 3 days
$\phi_o = 0.13$
t = 0
t = 15 min
t = 1 day
t = 5 days
$\phi_o = 0.17$
t = 0
t = 10 min
t = 1 day
t = 3 days
$\phi_o = 0.20$
t = 0
t = 1 min
t = 1 day
t = 5 days

FIG. 23

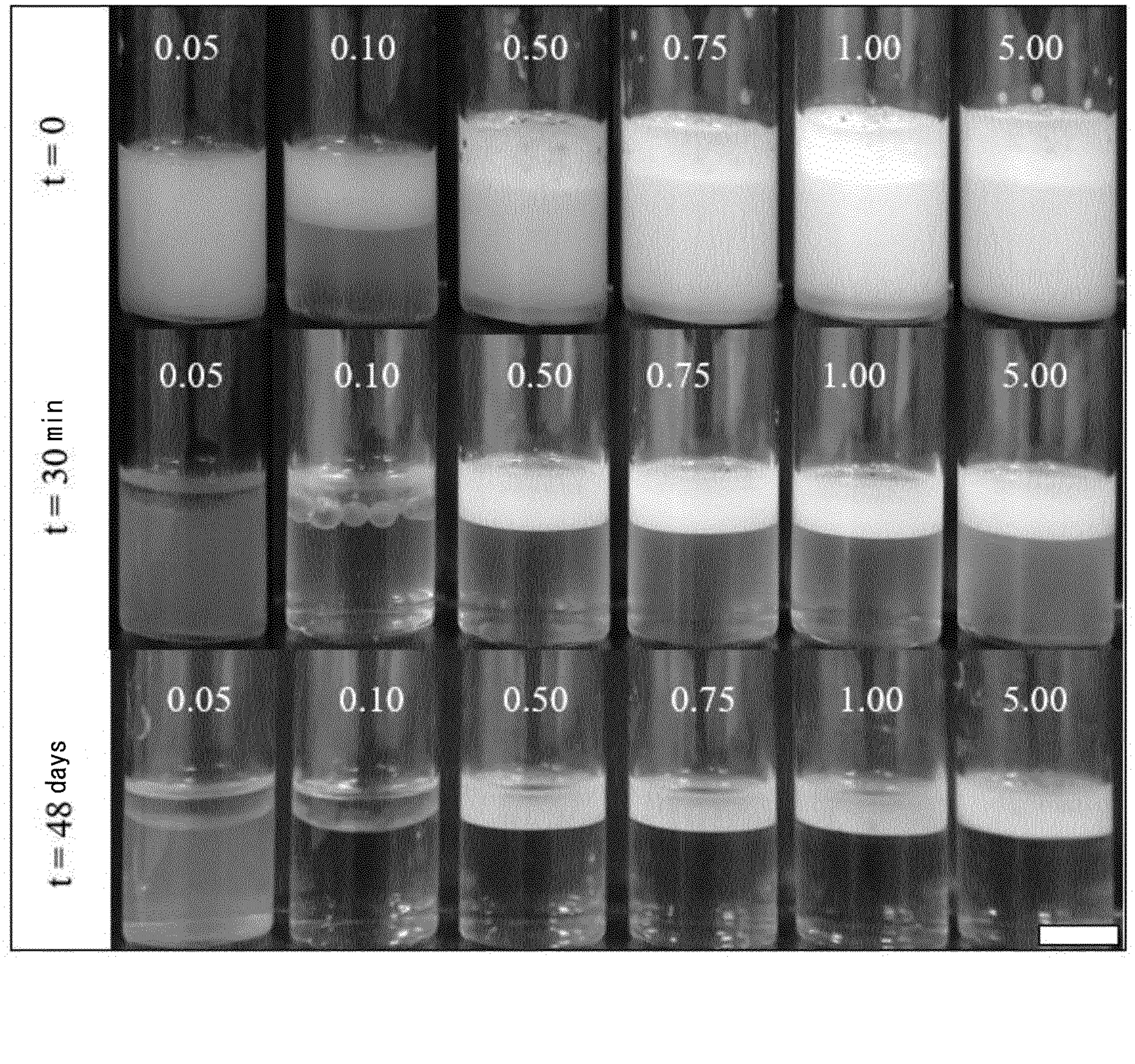

FIG. 24

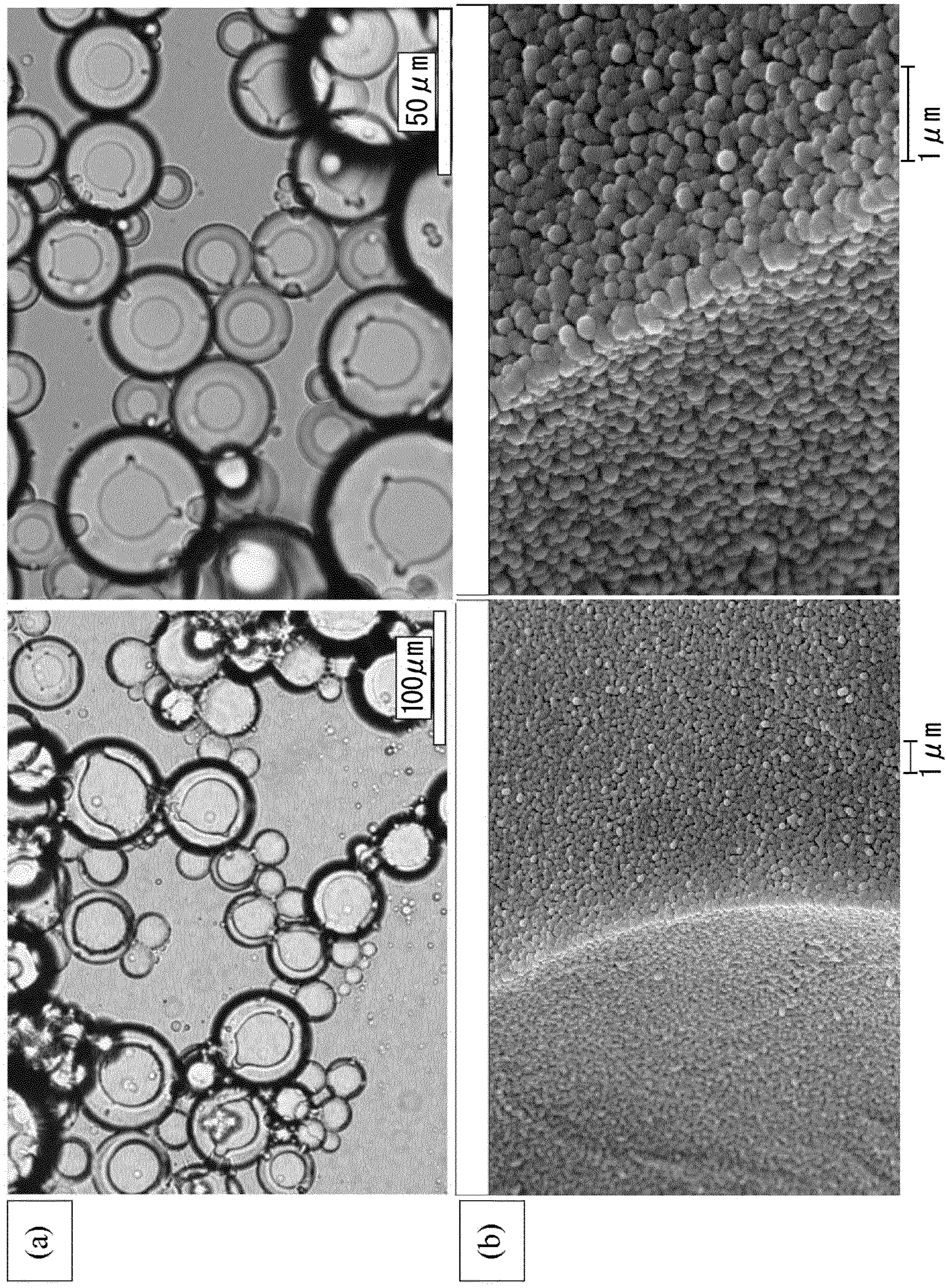

FIG. 25

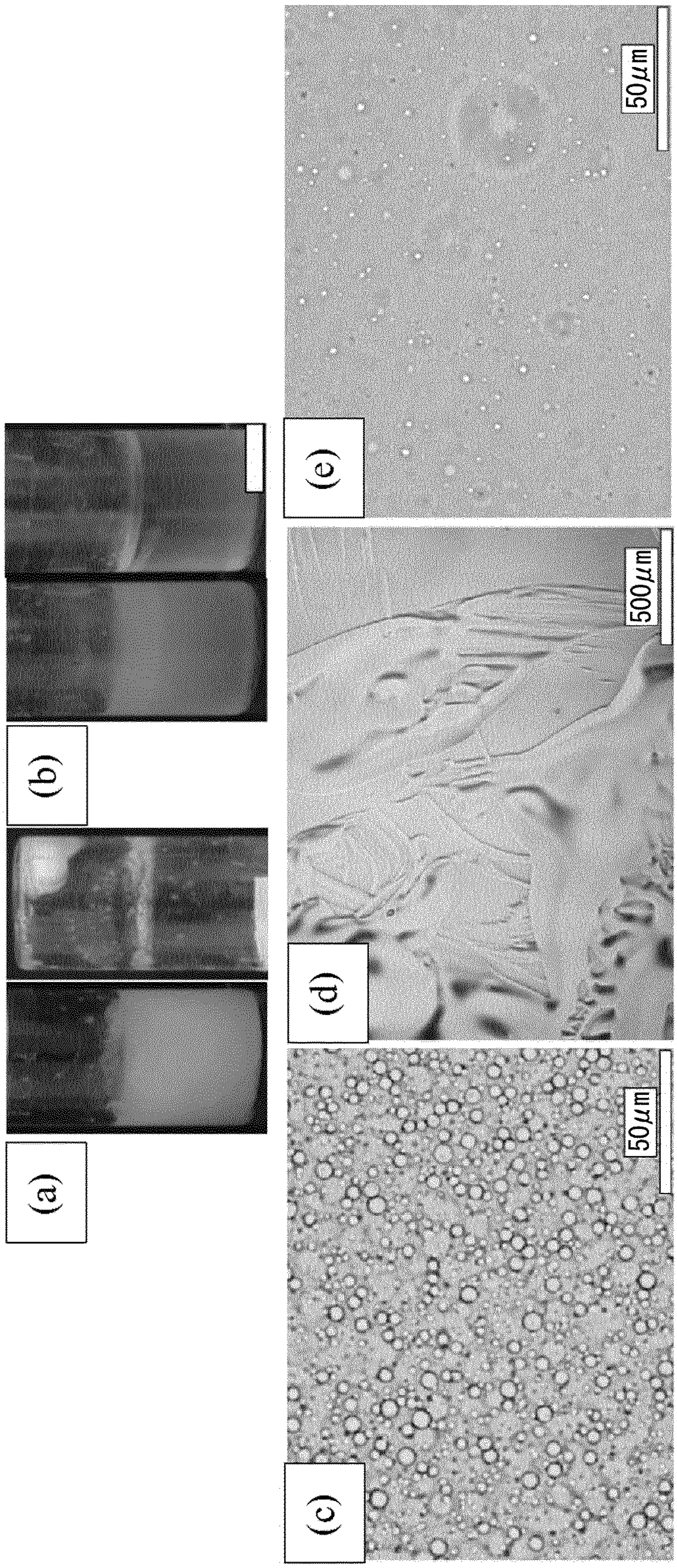

FIG. 26

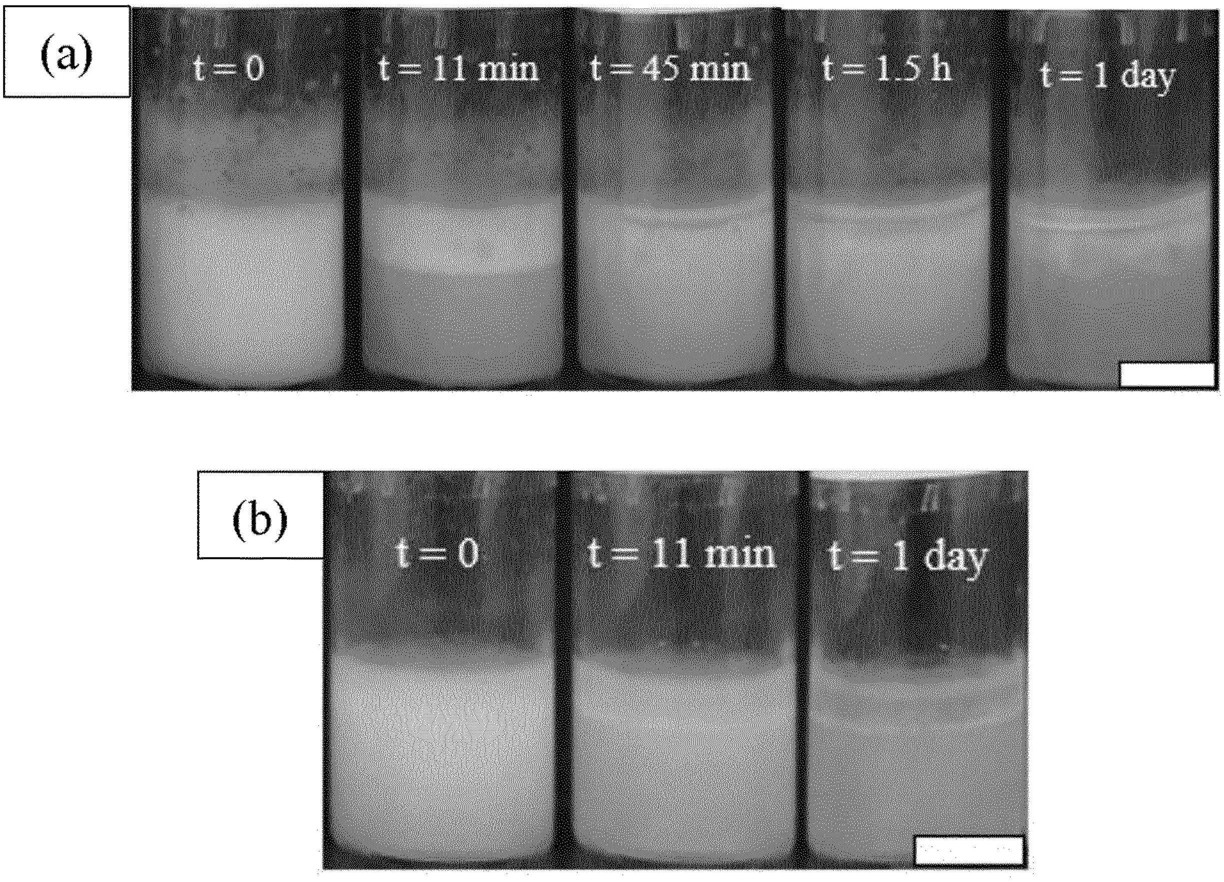

FIG. 27

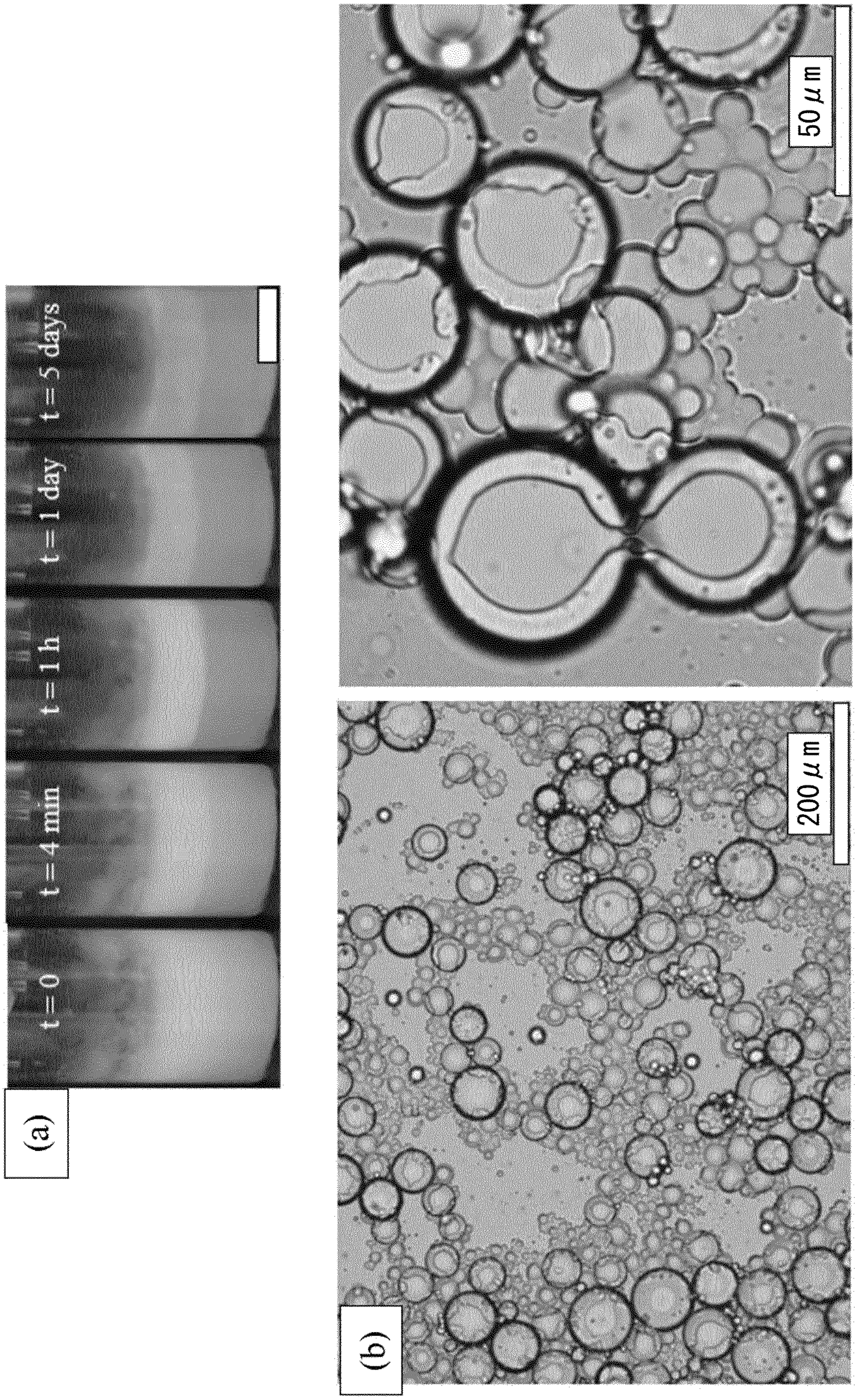

FIG. 28

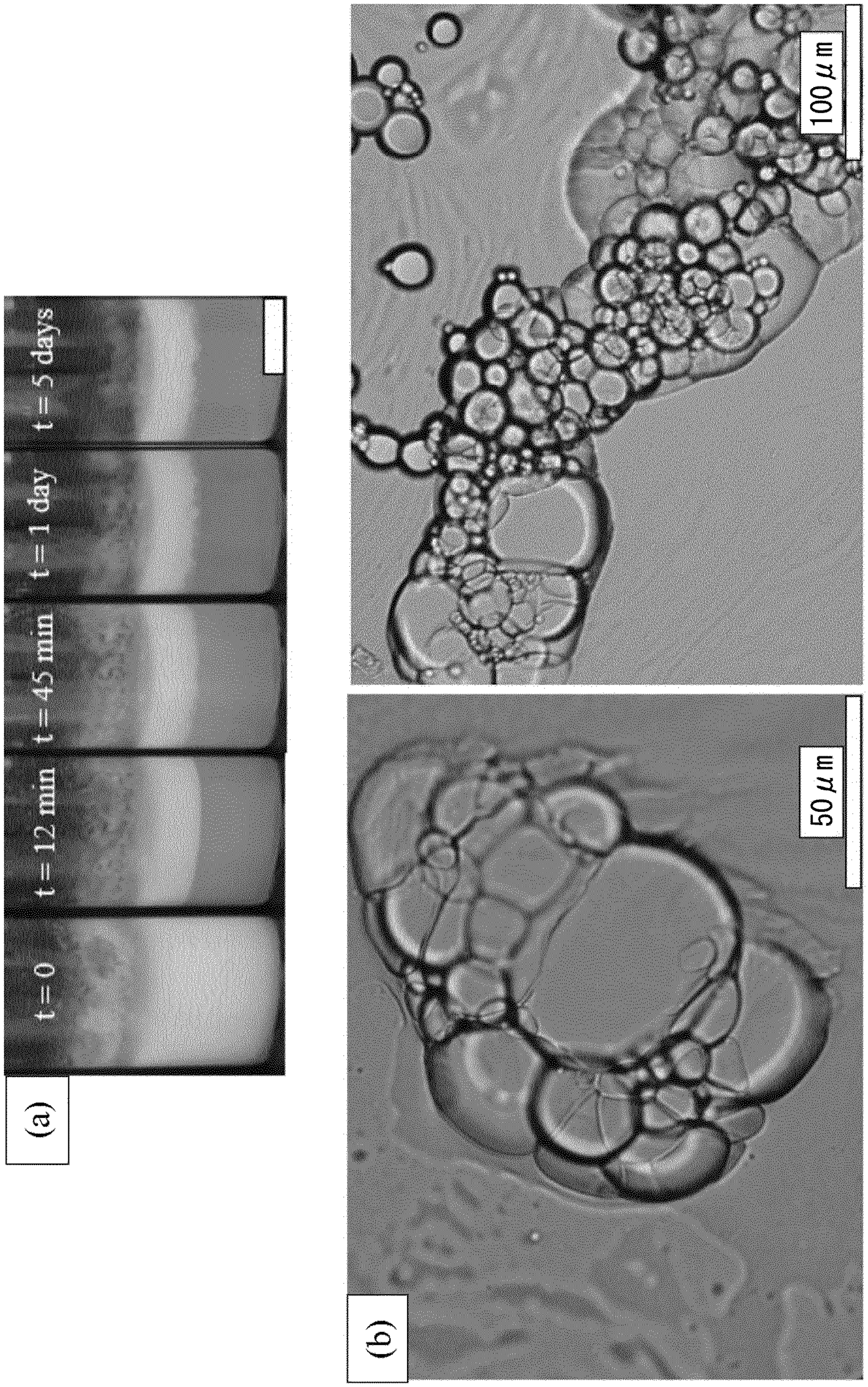

FIG. 29

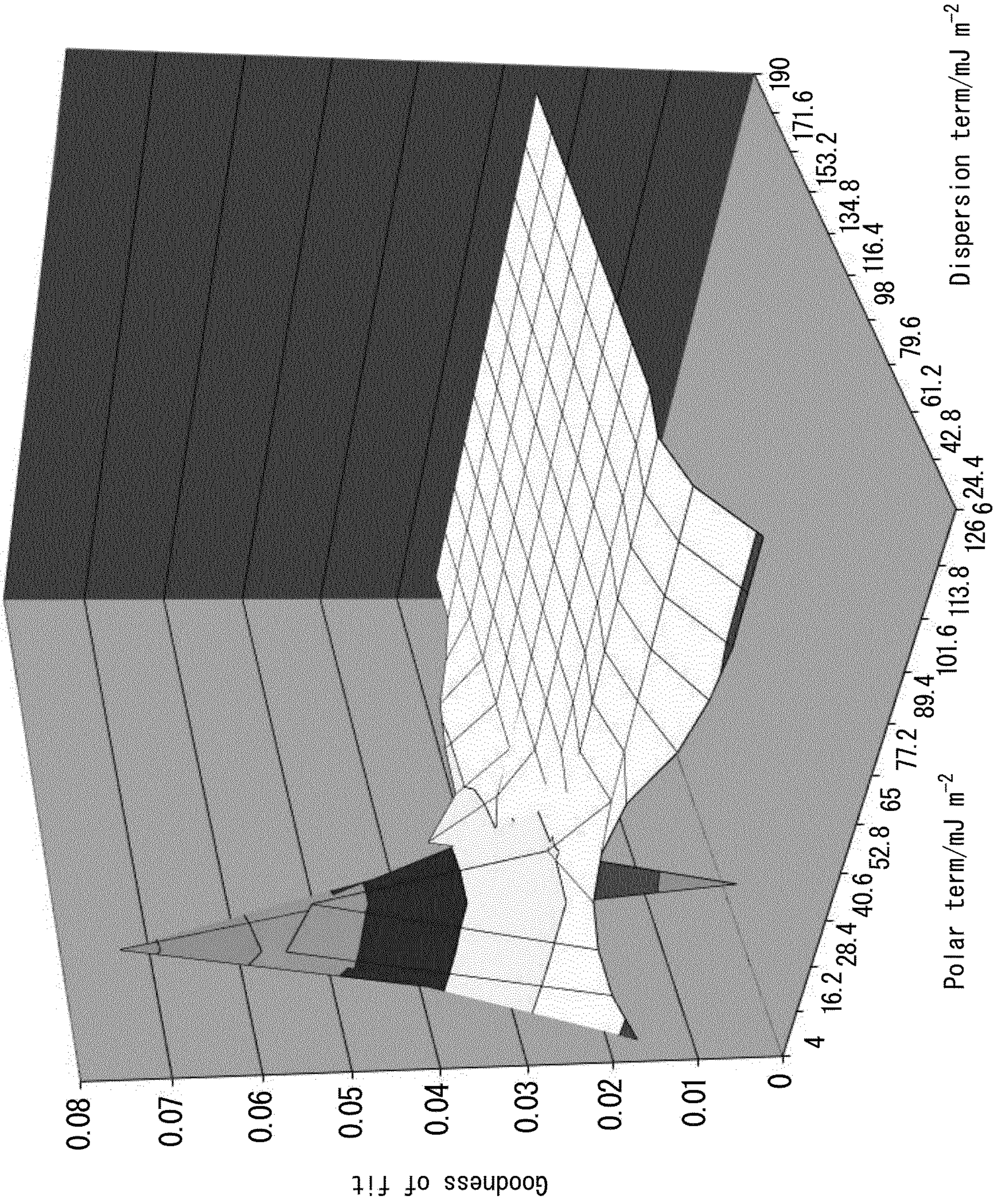
Goodness of fit
0
0.01
0.02
0.03
0.04
0.05
0.06
0.07
0.08
Polar term/mJ m$^{-2}$
4
16.2
28.4
40.6
52.8
65
77.2
89.4
101.6
113.8
126
Dispersion term/mJ m$^{-2}$
6
24.4
42.8
61.2
79.6
98
116.4
134.8
153.2
171.6
190

FIG. 30

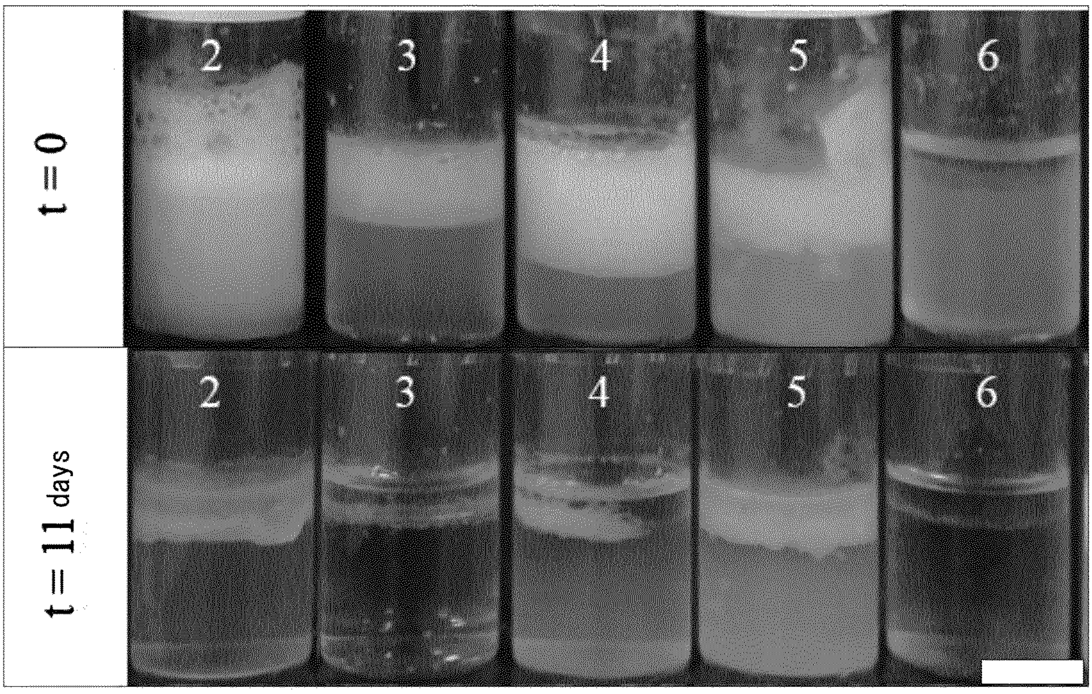


FIG. 31

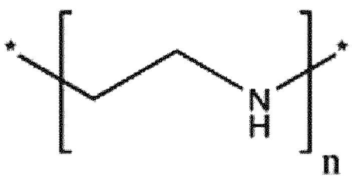

FIG. 32

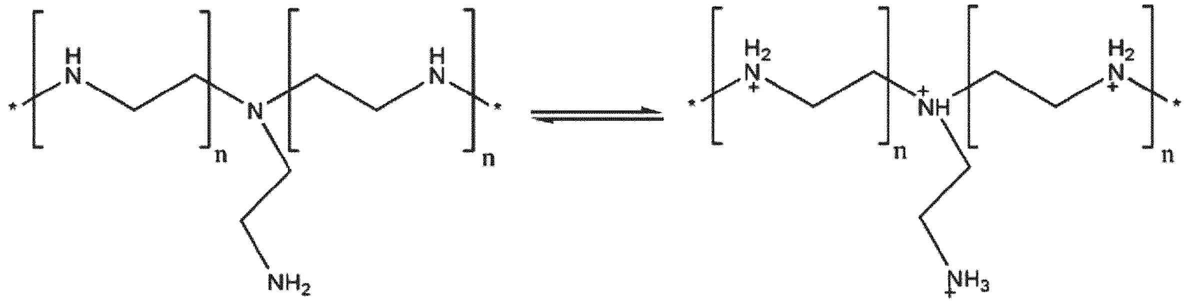

FIG. 33

STRONG

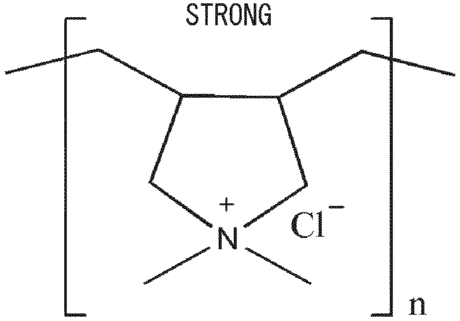

PDADMAC

STRONG

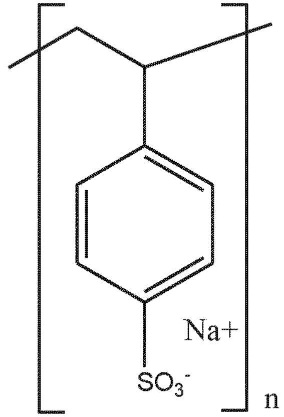

PSSNa

Interaction studied
at natural pH,
precipitate

emulsion with PEC precipitate,
results first paper

pH<5 precipitate
pH>5 cloudy
dispersions
coacervation

emulsion with PEC coacervate
under specific experimental
conditions, no emulsion PEC
precipitate due to big aggregates,
results draft second paper

Currently
under study,
see summary
slides 2 and 3

pH=2 precipitate/flocks
pH=natural precipitate/flocks
pH=10 cloudy
dispersions coacervation

WEAK

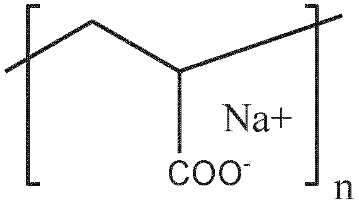

PAANa

Currently under study,
see summary slide 4

pH=4 precipitate/flocks
pH=7 cloudy dispersions coacervae
pH=10 cloudy dispersions coacervate

WEAK

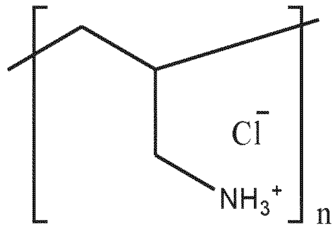

PAH

FIG. 34A

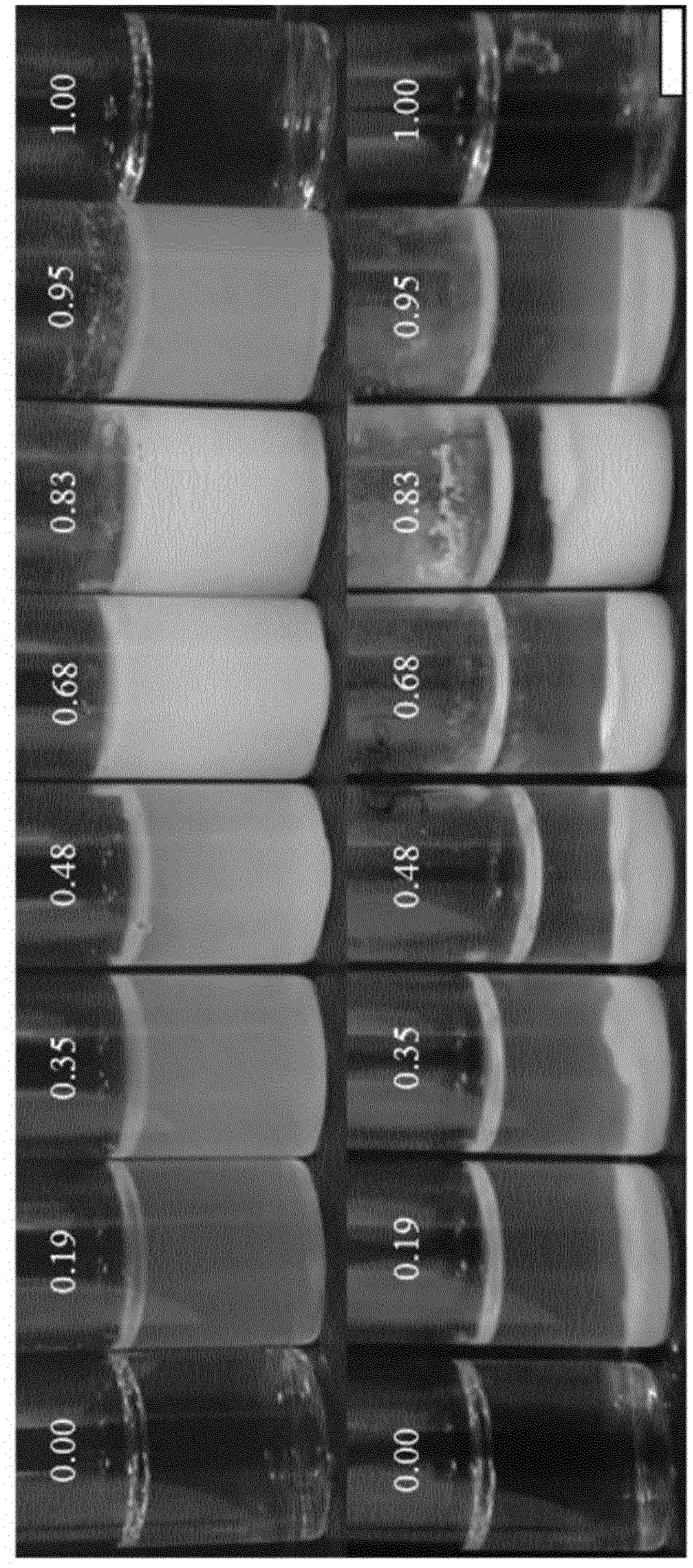


t = 0

t = 3.7 months

FIG. 34B

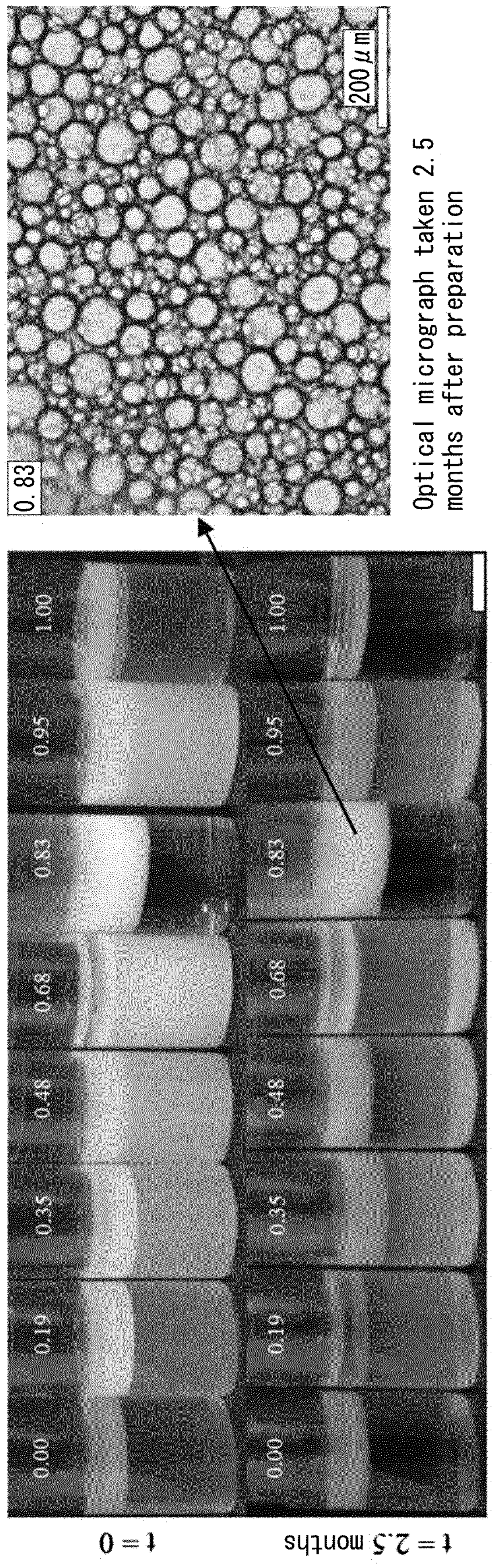

t = 0
t = 2.5 months
0.00
0.19
0.35
0.48
0.68
0.83
0.95
1.00
0.83
200 μm
Optical micrograph taken 2.5 months after preparation

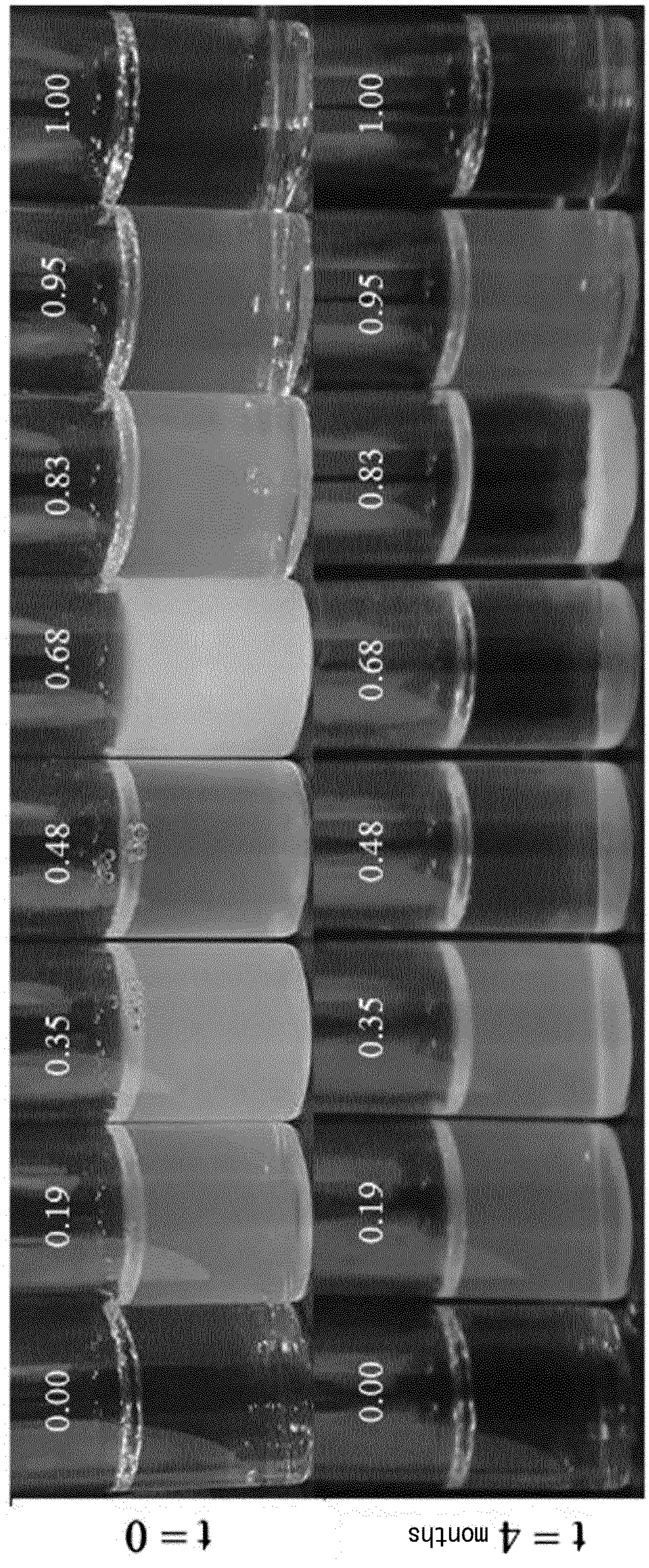


FIG. 34C

FIG. 34D

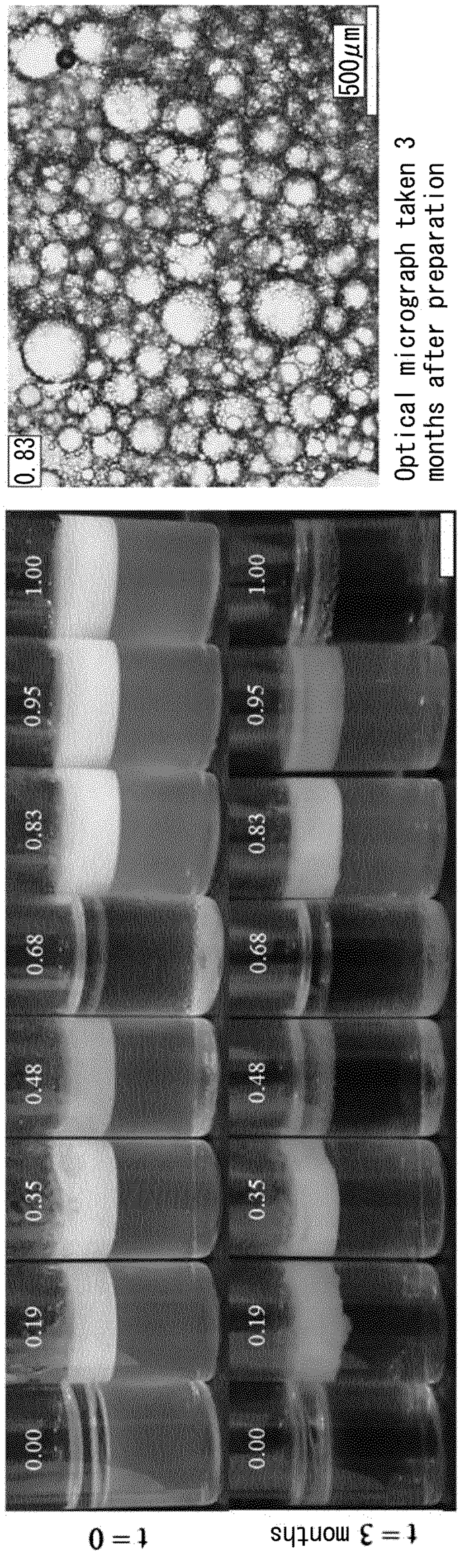


Optical micrograph taken 3 months after preparation

FIG. 35A

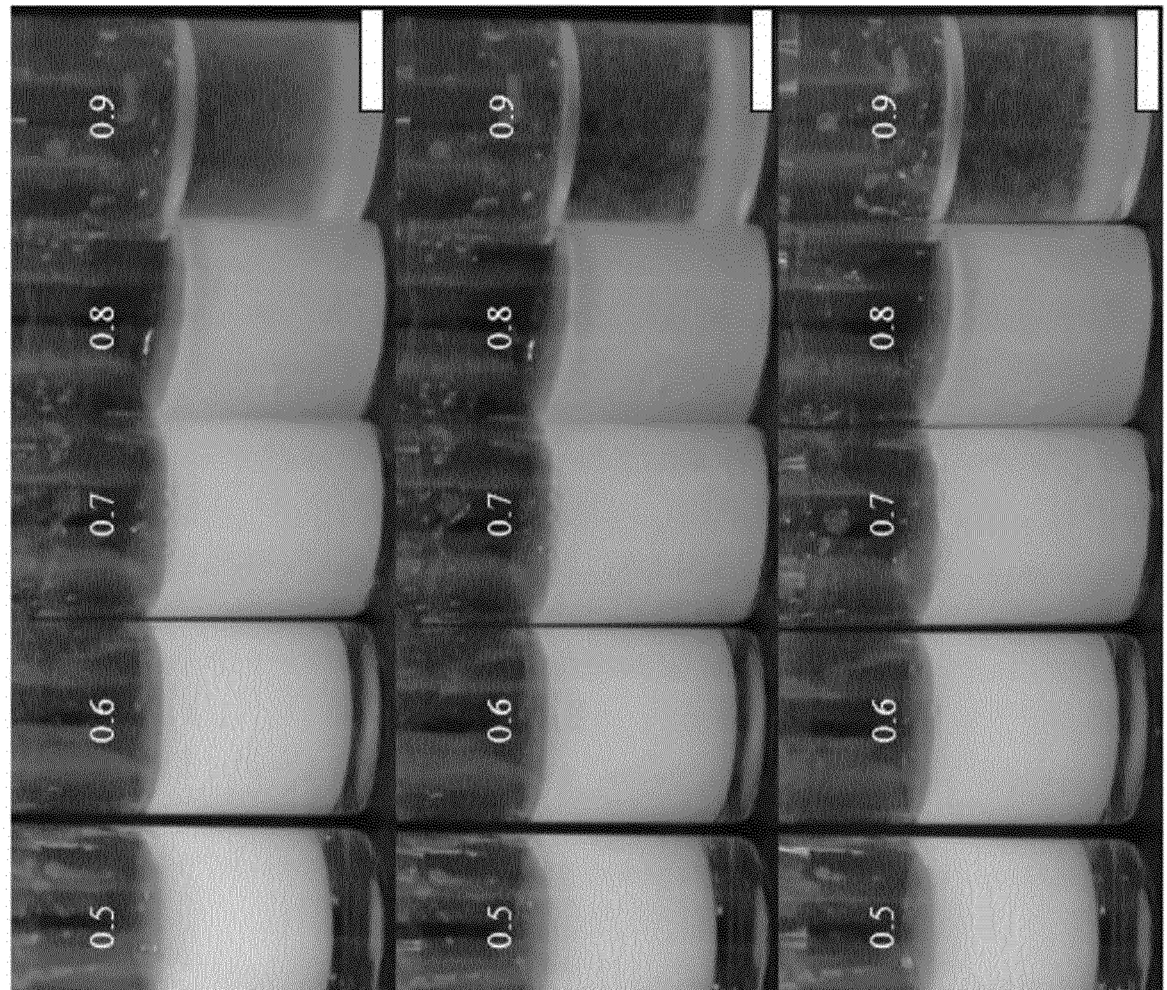

FIG. 35B

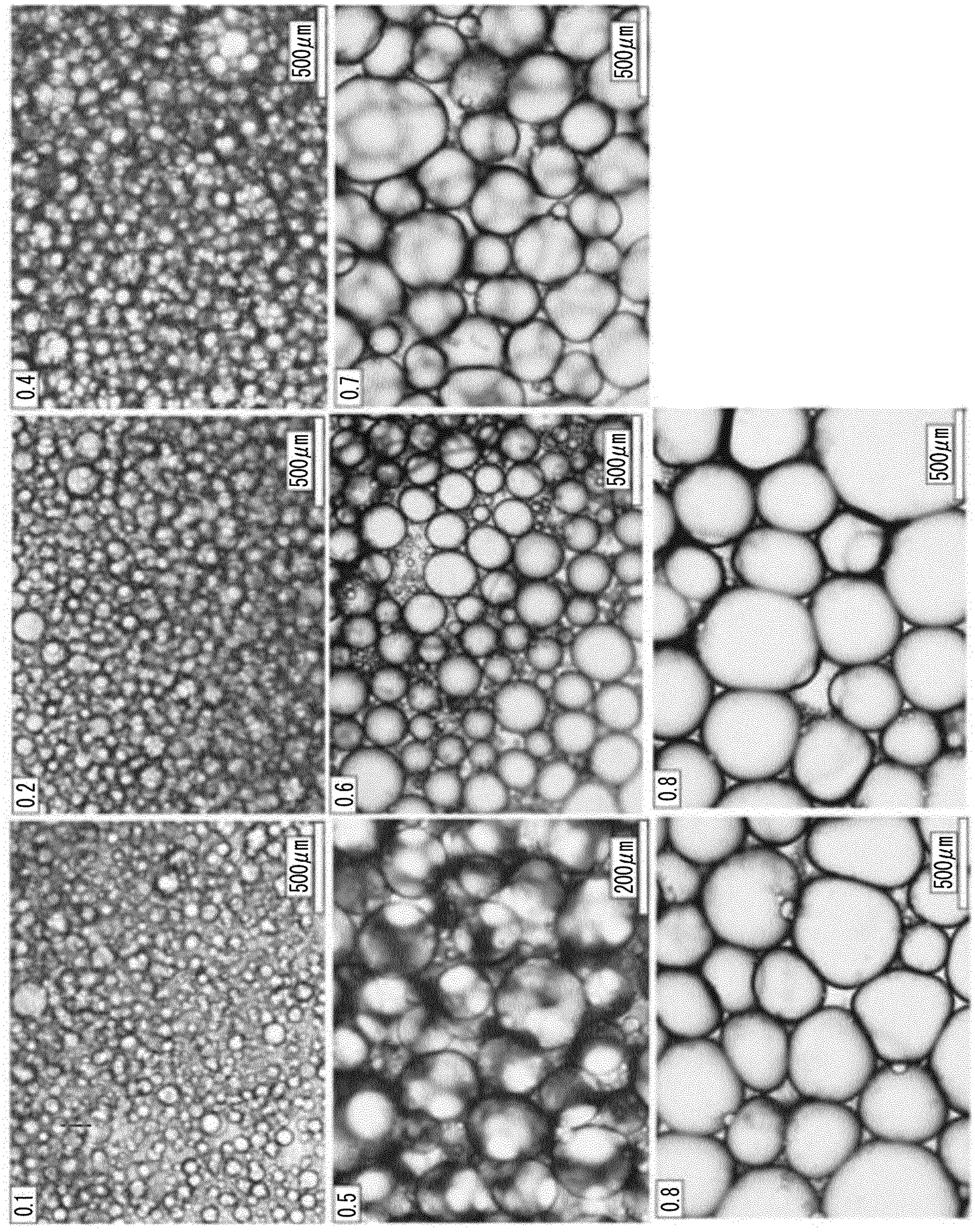
0.1
500μm
0.2
500μm
0.4
500μm
0.5
200μm
0.6
500μm
0.7
500μm
0.8
500μm
0.8
500μm

FIG. 36A

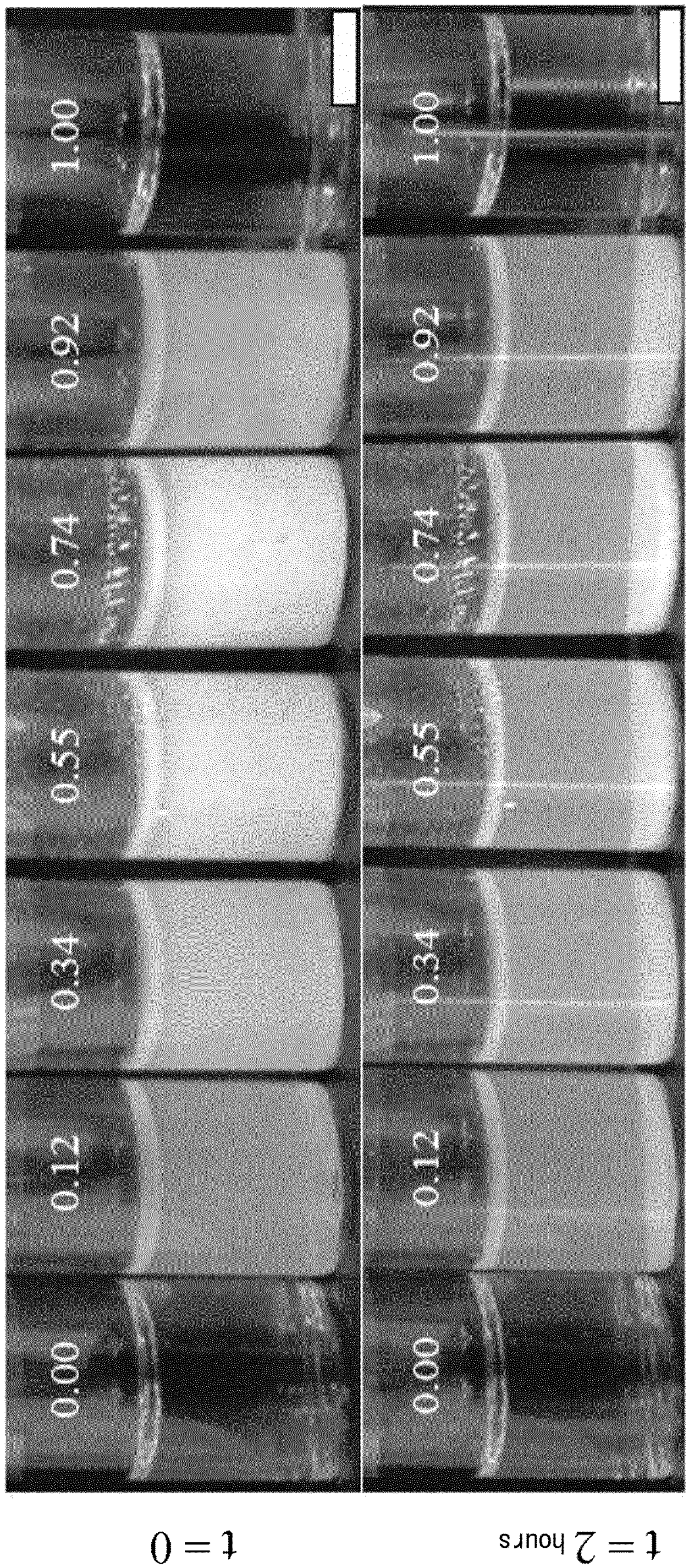

FIG. 36B

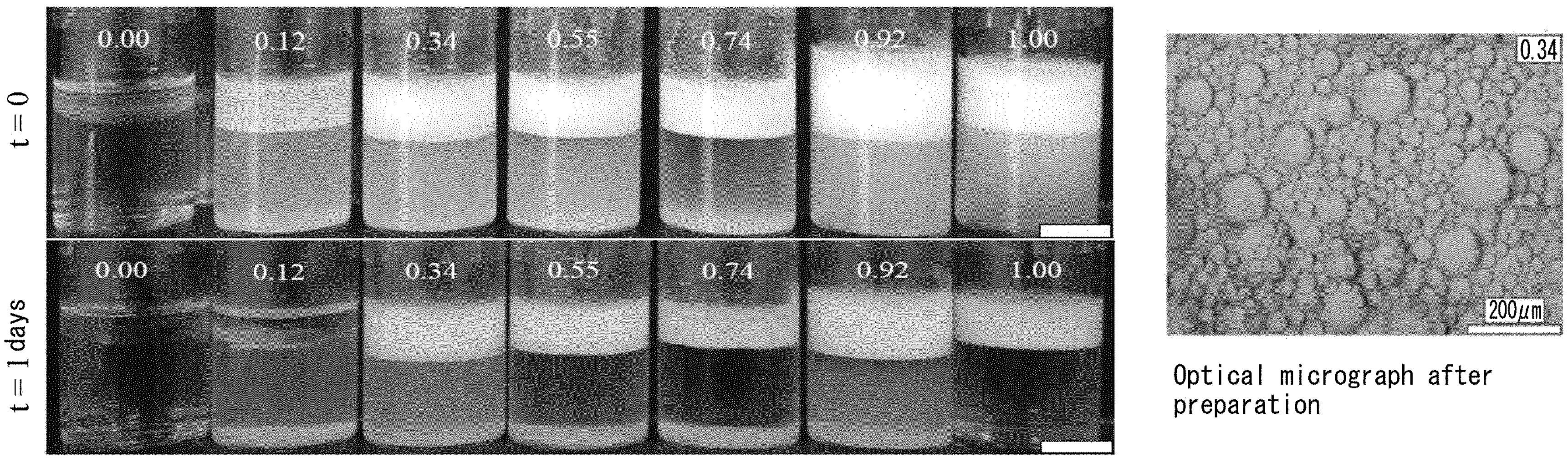


Optical micrograph after preparation

FIG. 36C

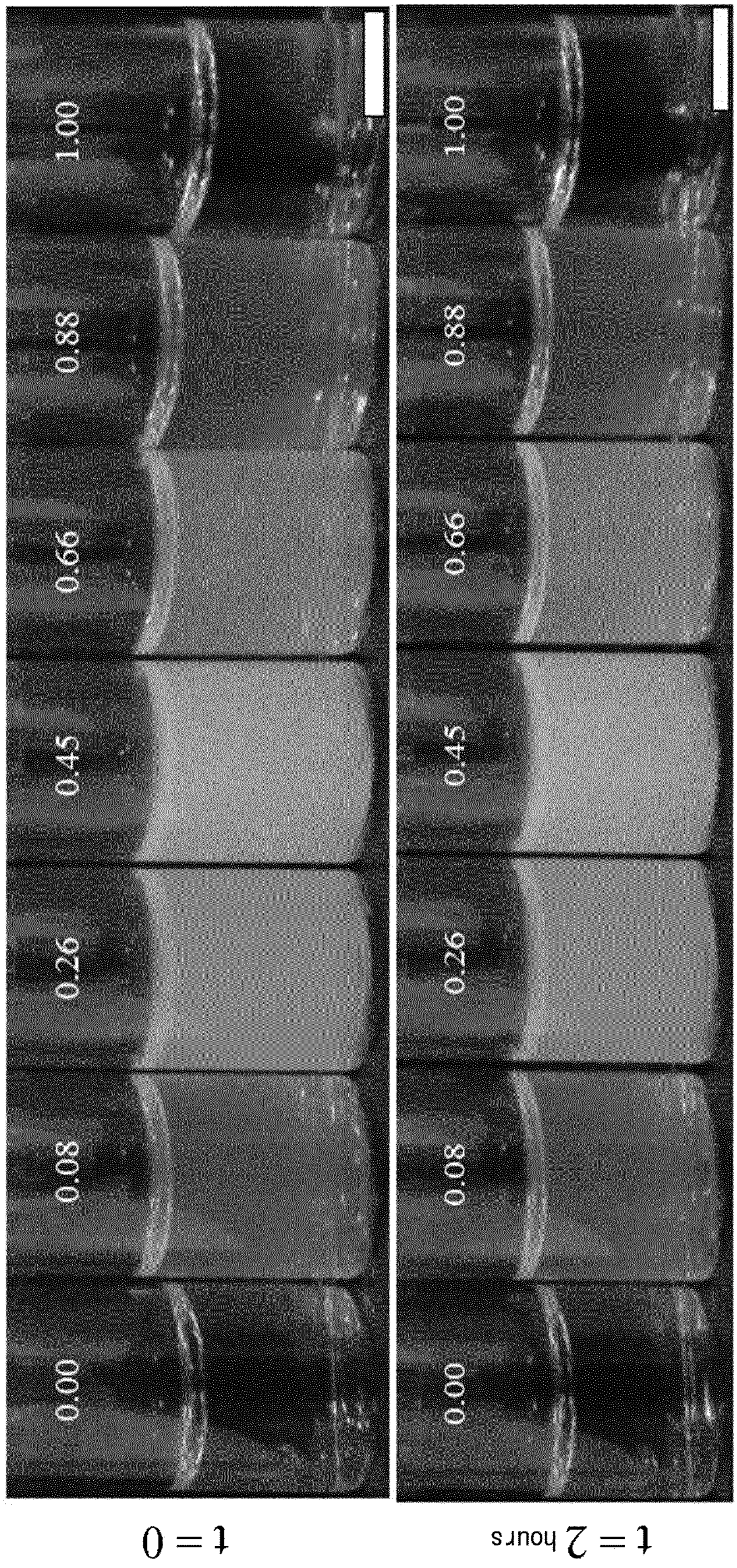

FIG. 36D

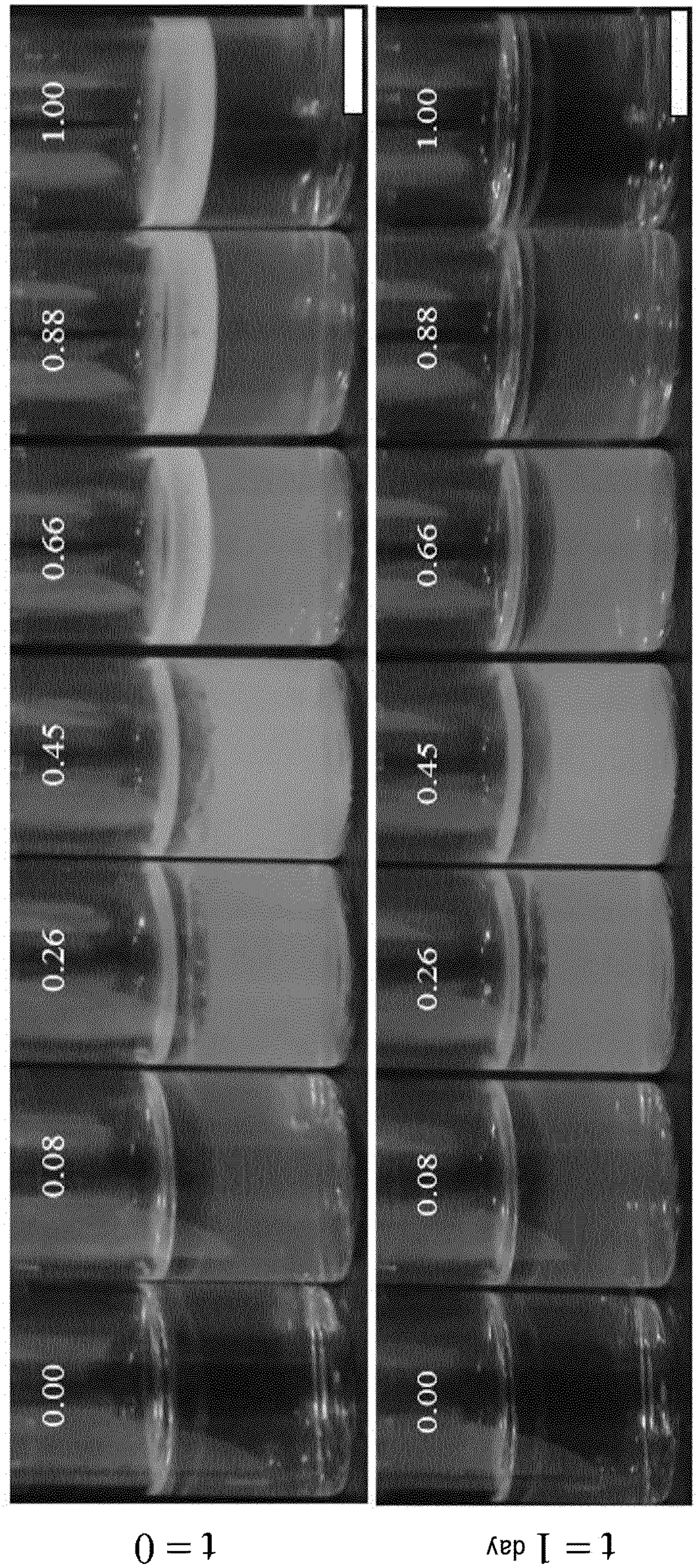

FIG. 37

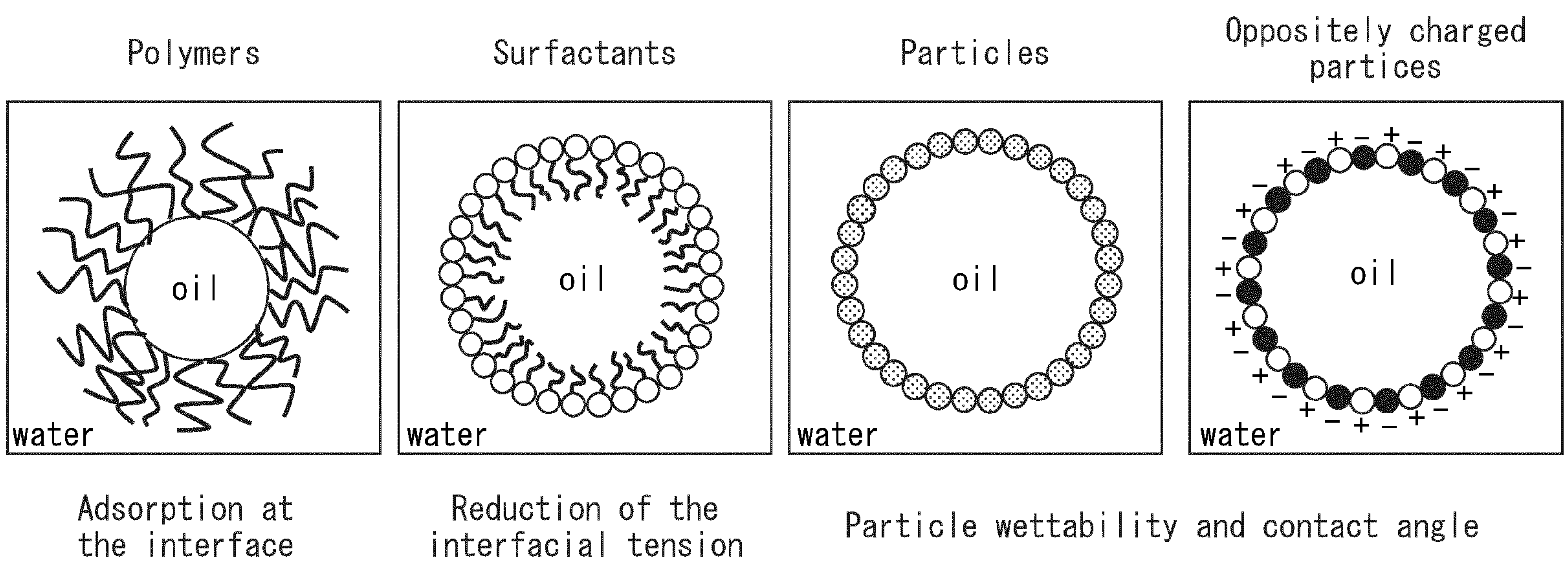
Polymers
Surfactants
Particles
Oppositely charged partices
oil
oil
oil
oil
water
water
water
water
Adsorption at the interface
Reduction of the interfacial tension
Particle wettability and contact angle

FIG. 38

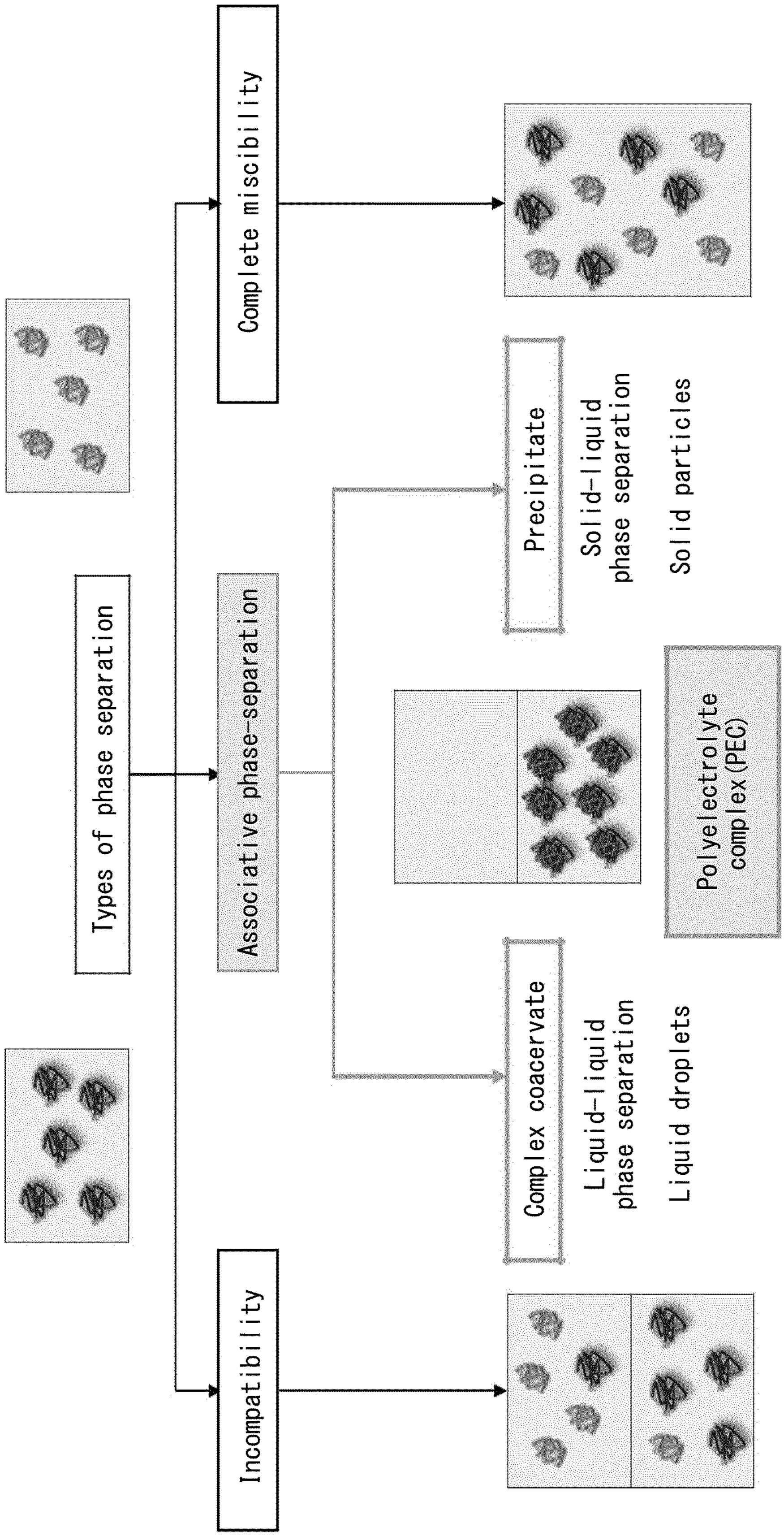
Types of phase separation
Incompatibility
Associative phase-separation
Complete miscibility
Complex coacervate
Liquid-liquid phase separation
Liquid droplets
Polyelectrolyte complex (PEC)
Precipitate
Solid-liquid phase separation
Solid particles

FIG. 39

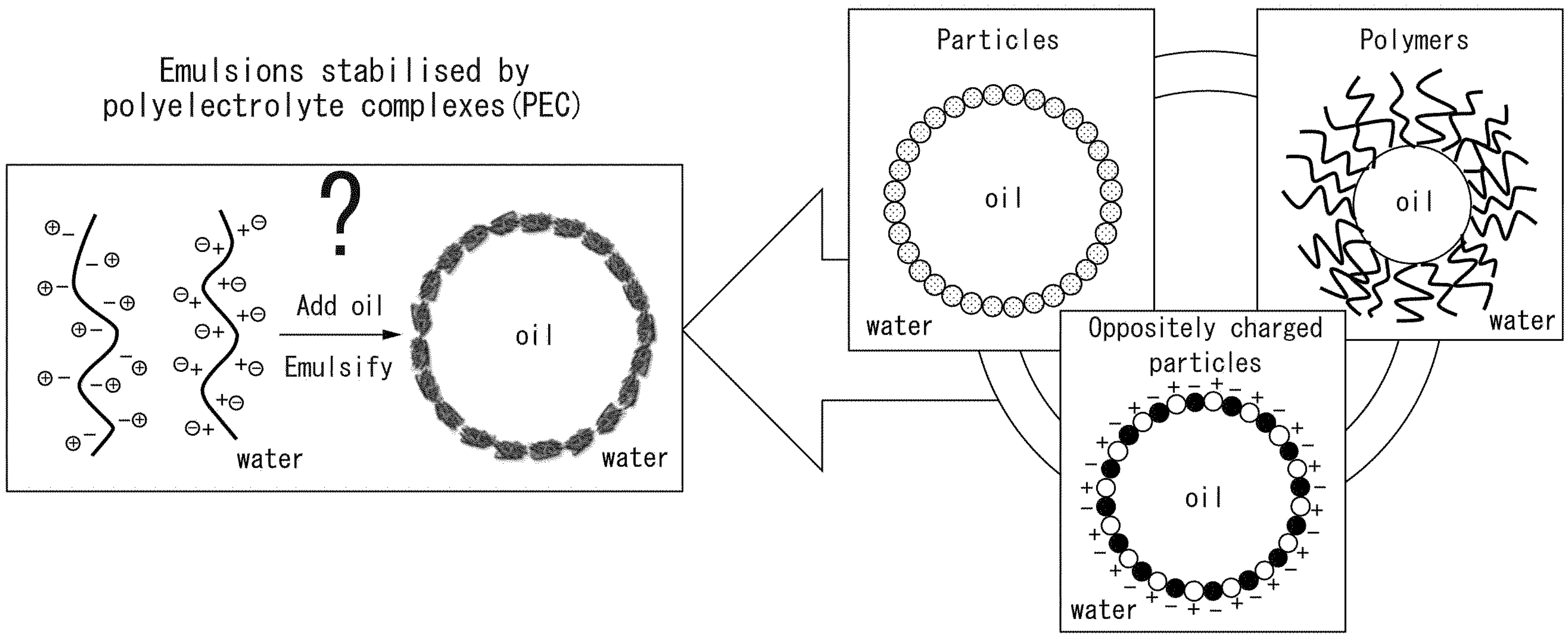
Emulsions stabilised by
polyelectrolyte complexes (PEC)
Add oil
Emulsify
?
oil
water
water
Particles
oil
water
Polymers
oil
water
Oppositely charged
particles
oil
water

FIG. 40

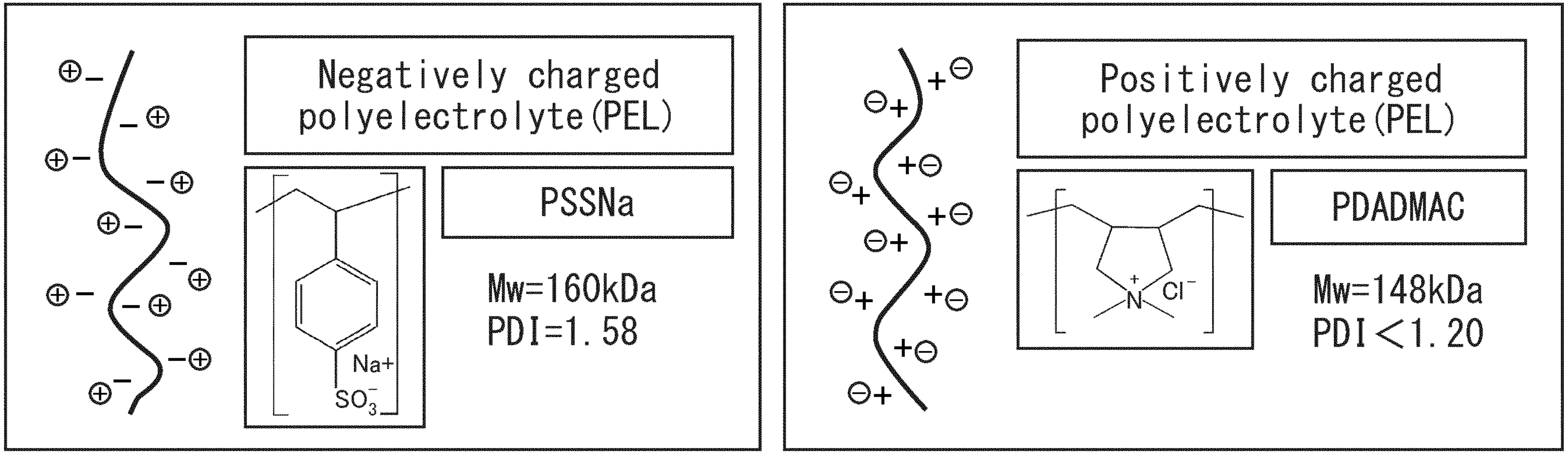


| PEC aqueous dispersions | |
|---|---|
| parameters | $x_{PSSNa} = \frac{n_{PSSNa}}{n_{PSSNa} + n_{PDADMAC}}$<br>[PEL]<br>Salt content |
| techniques | Dynamic light scattering<br>SEM |

| Emulsions with n-dodecane | |
|---|---|
| parameters | $x_{PSSNa}$<br>[PEL]<br>Salt content<br>Fraction of oil ($\phi_0$)<br>Oil type |
| techniques | Optical microscopy<br>Cryo-SEM<br>Tensiometry |

FIG. 41A

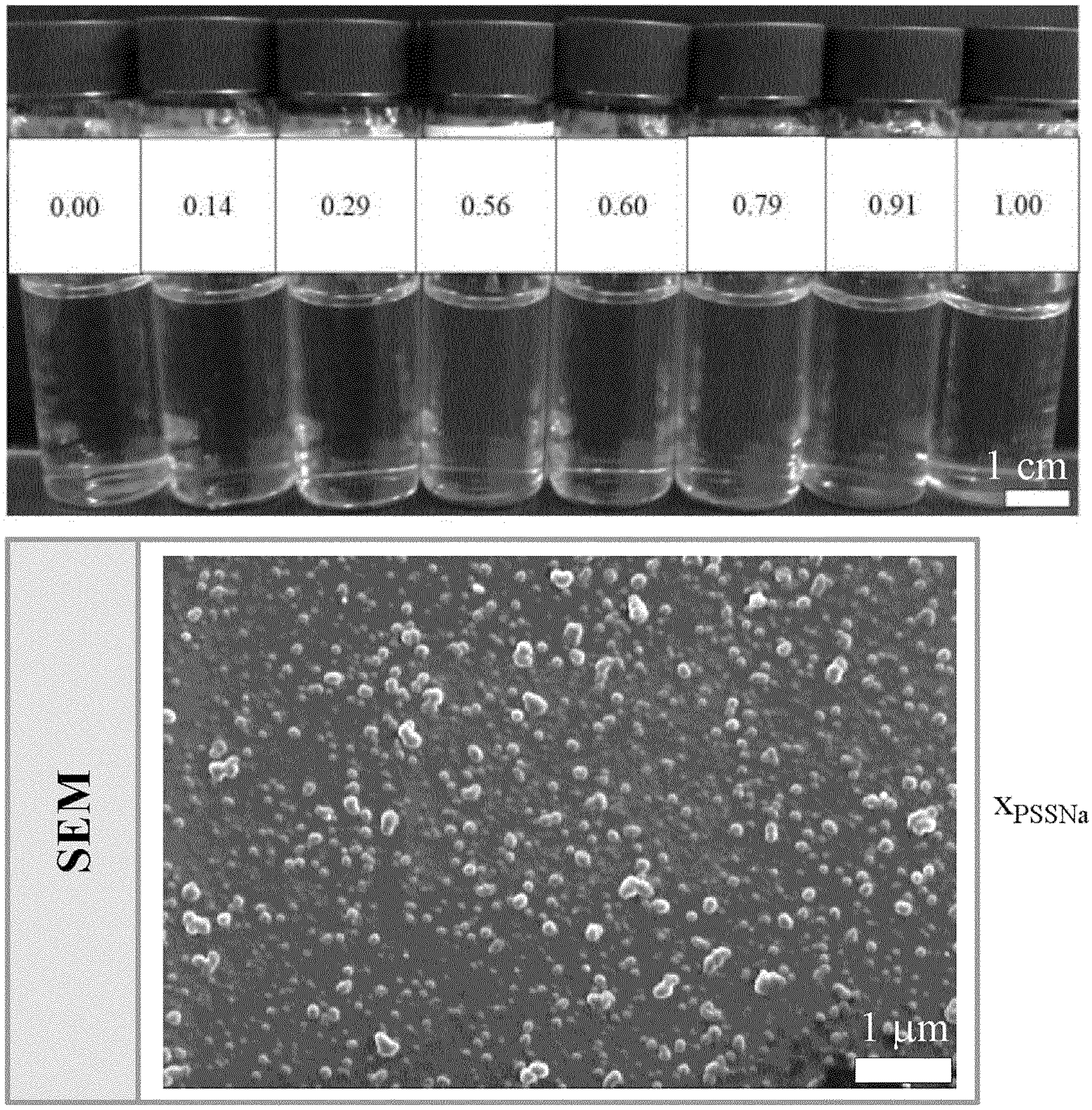


$x_{PSSNa} = 0.56$

FIG. 41B

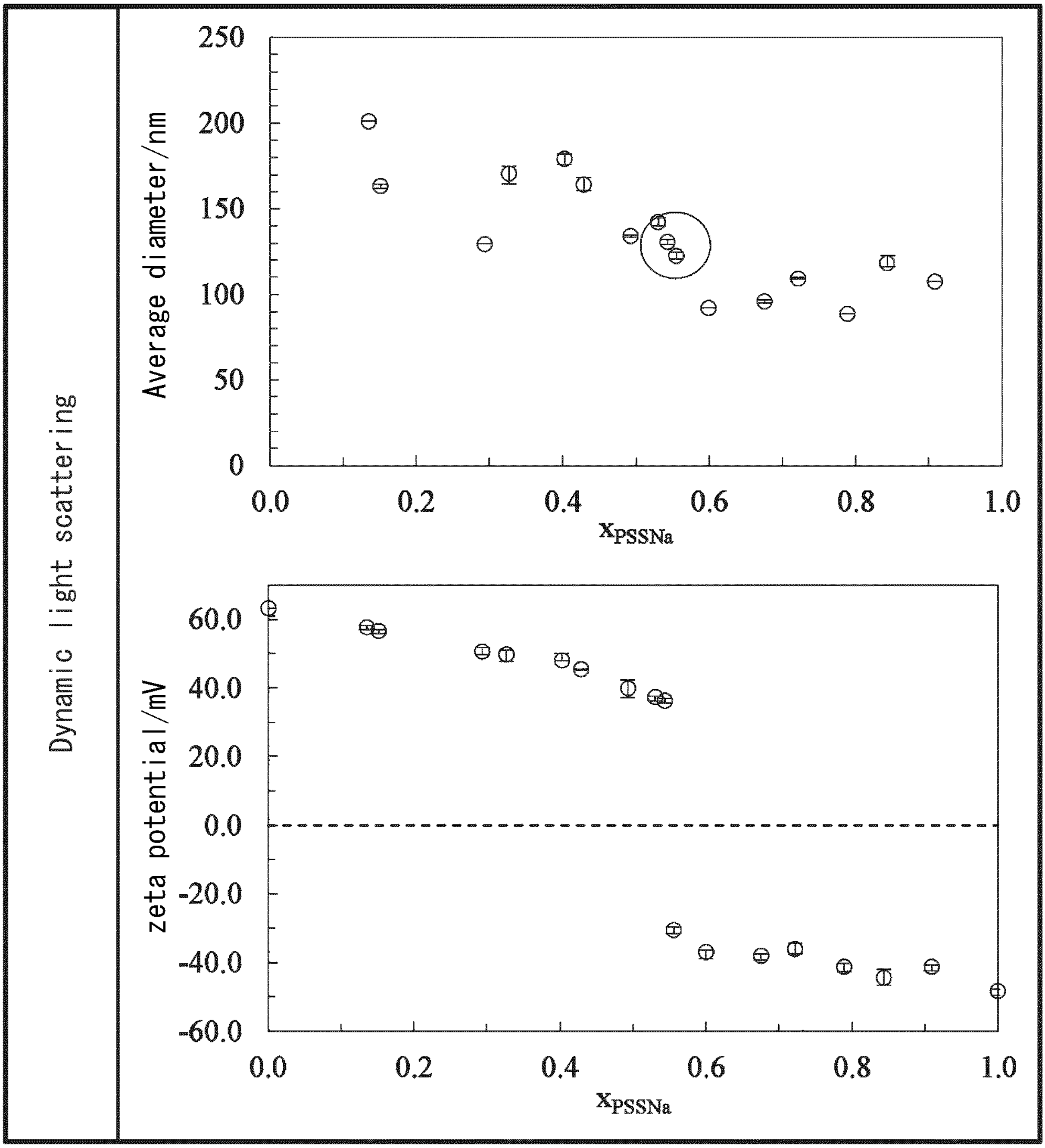
Dynamic light scattering
Average diameter/nm
250
200
150
100
50
0
0.0
0.2
0.4
0.6
0.8
1.0
$x_{PSSNa}$
zeta potential/mV
60.0
40.0
20.0
0.0
-20.0
-40.0
-60.0
0.0
0.2
0.4
0.6
0.8
1.0
$x_{PSSNa}$

FIG. 42A

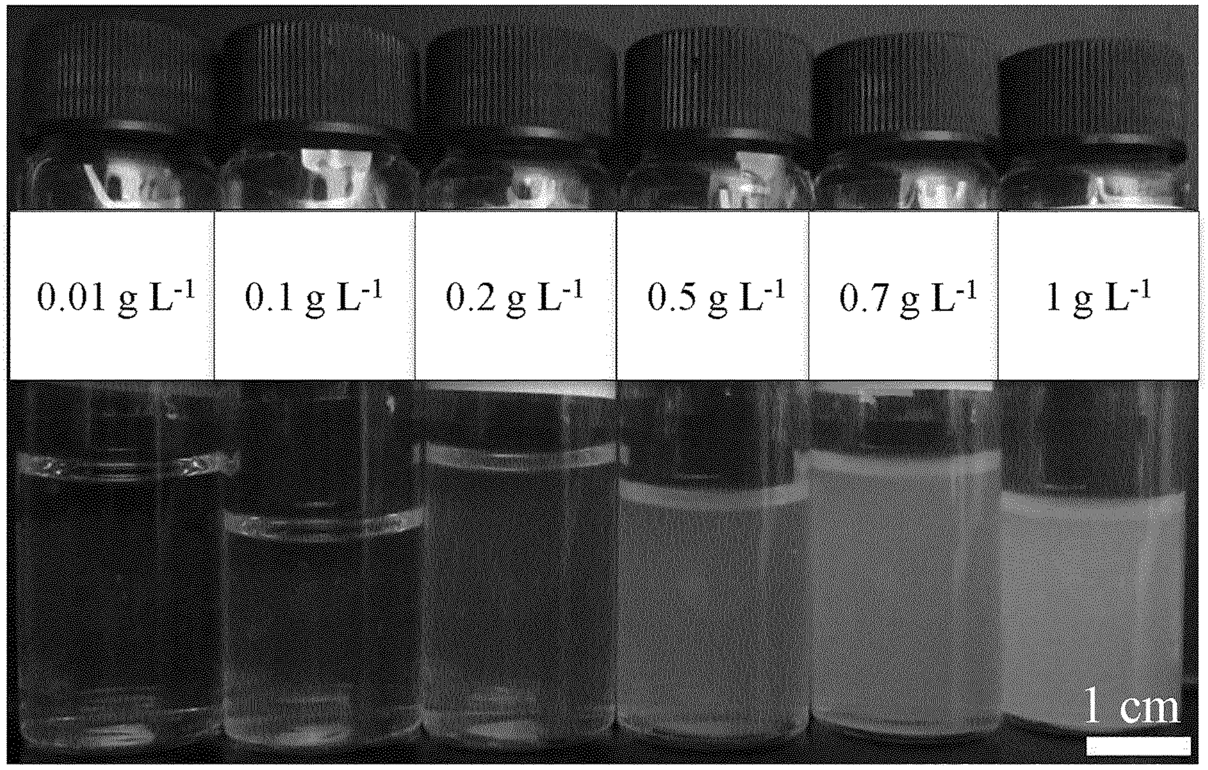

FIG. 42B

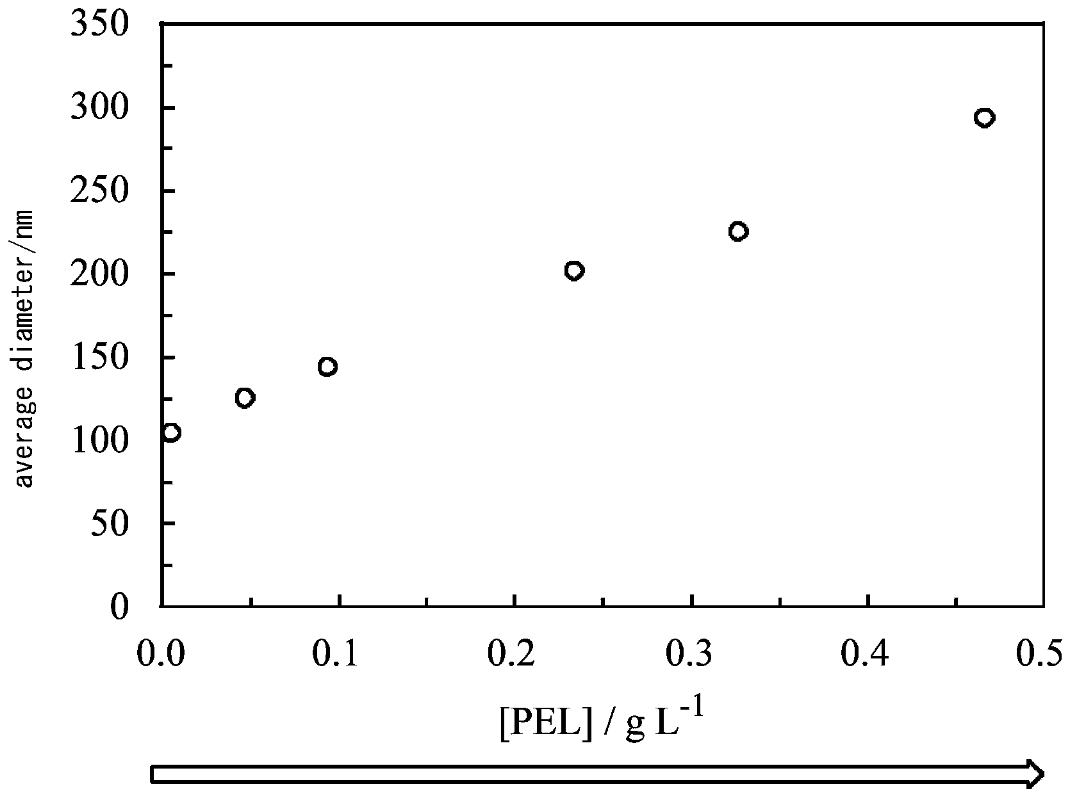


↑average diameter with the [PEL]

FIG. 43A

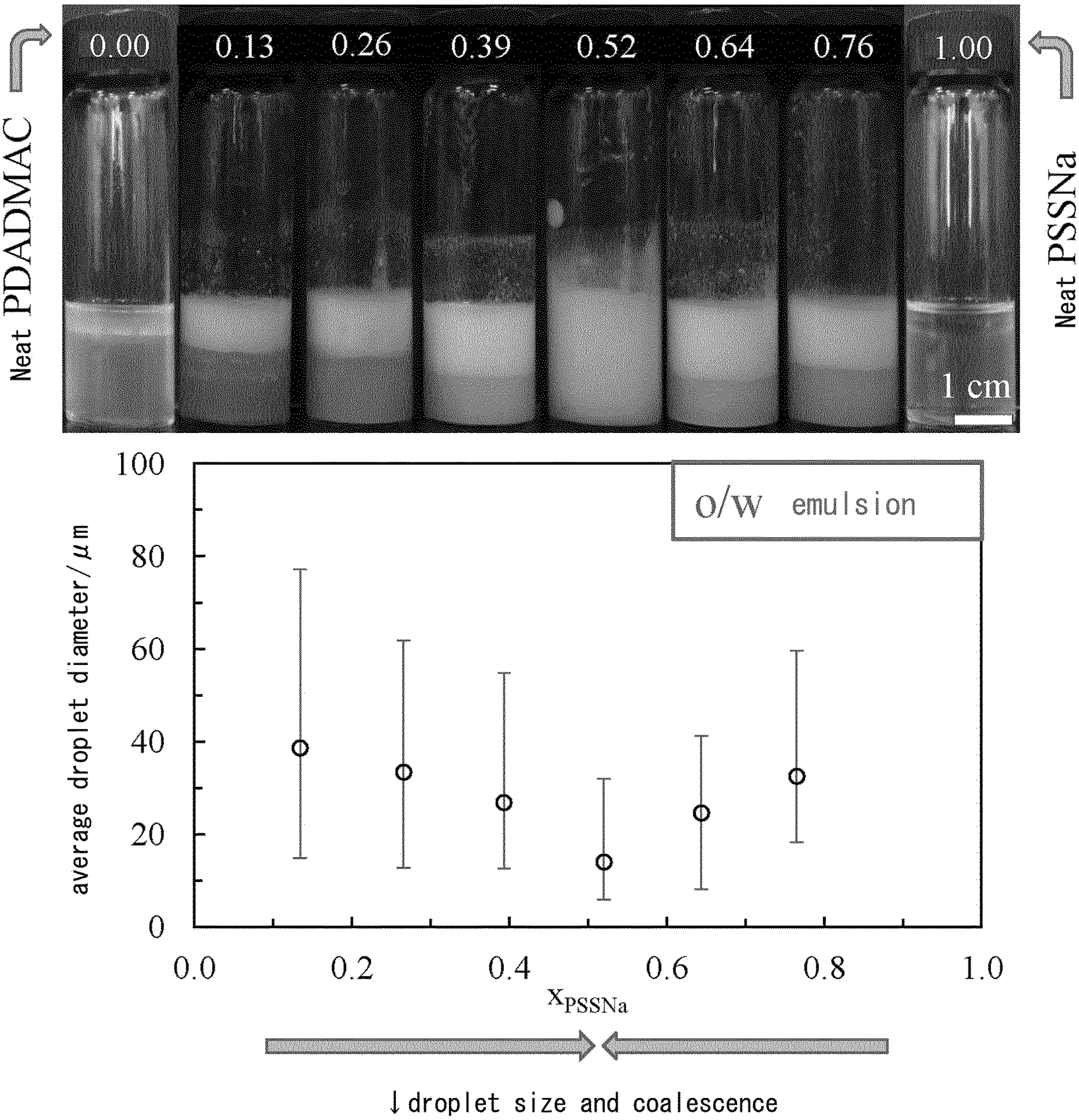
0.00
0.13
0.26
0.39
0.52
0.64
0.76
1.00
Neat PDADMAC
Neat PSSNa
1 cm
o/w emulsion
average droplet diameter/μm
100
80
60
40
20
0
0.0
0.2
0.4
0.6
0.8
1.0
$x_{PSSNa}$
↓droplet size and coalescence

FIG. 43B

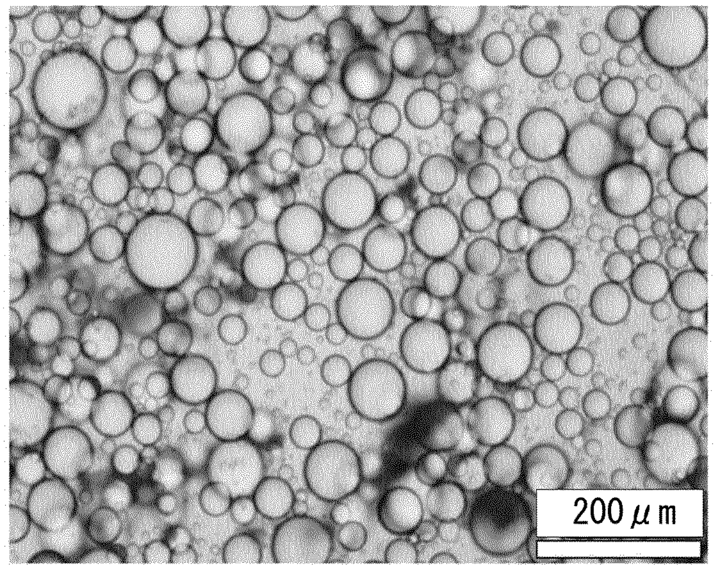


$x_{PSSNa}$ = 0.13

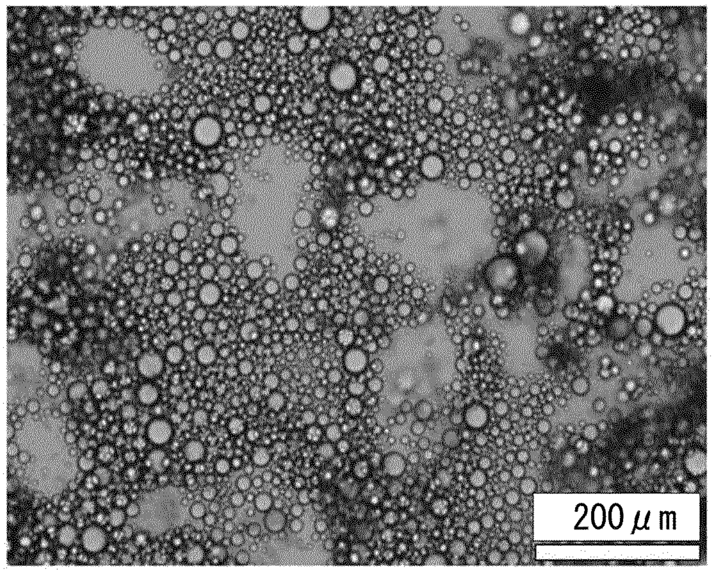


$x_{PSSNa}$ = 0.52

FIG. 44A

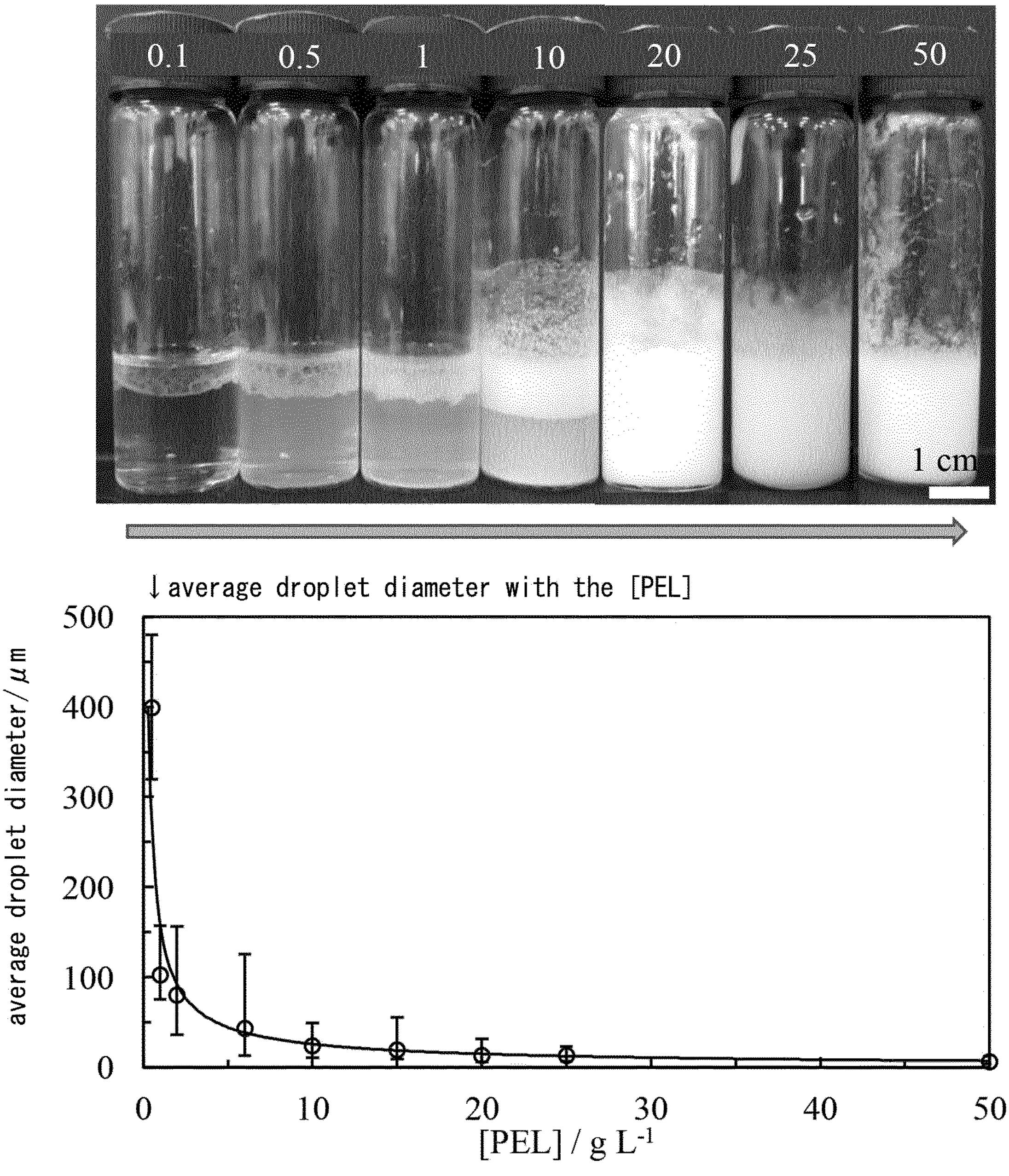
0.1
0.5
1
10
20
25
50
1 cm
↓average droplet diameter with the [PEL]
average droplet diameter/μm
500
400
300
200
100
0
0
10
20
30
40
50
[PEL] / g L-1

FIG. 44B

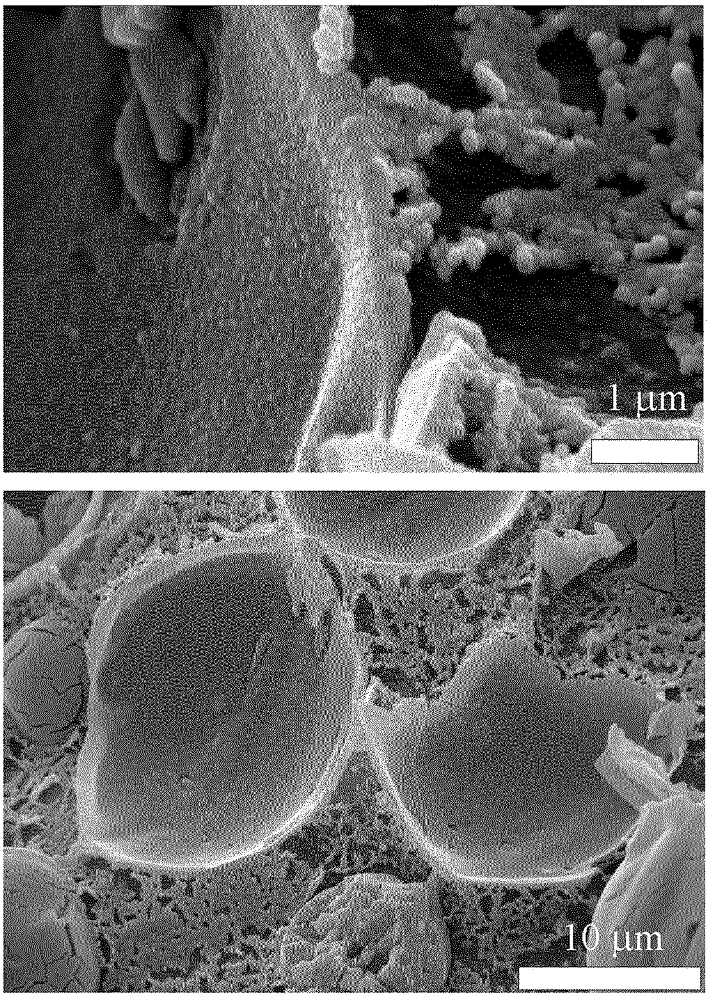


FIG. 45A

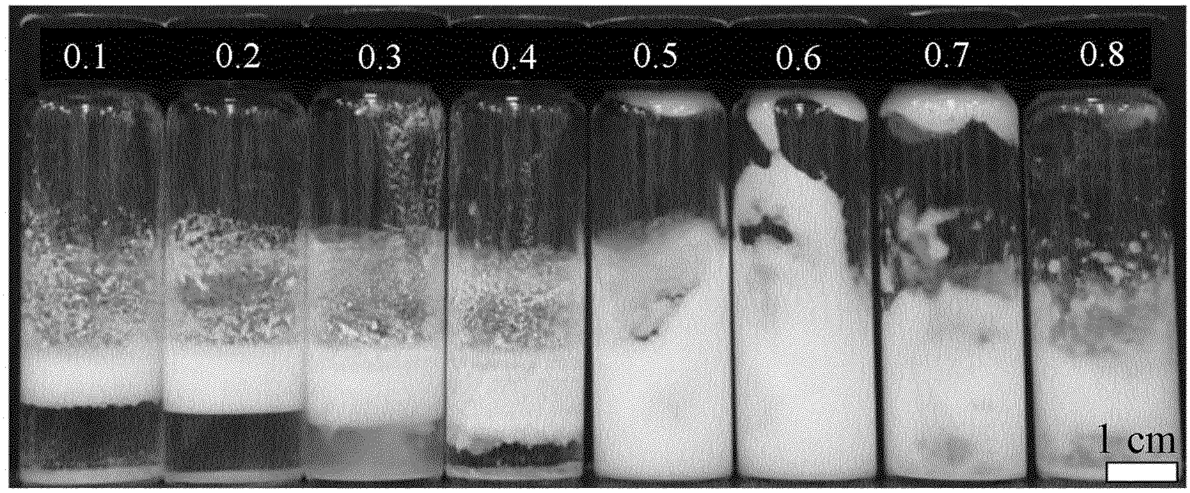

FIG. 45B

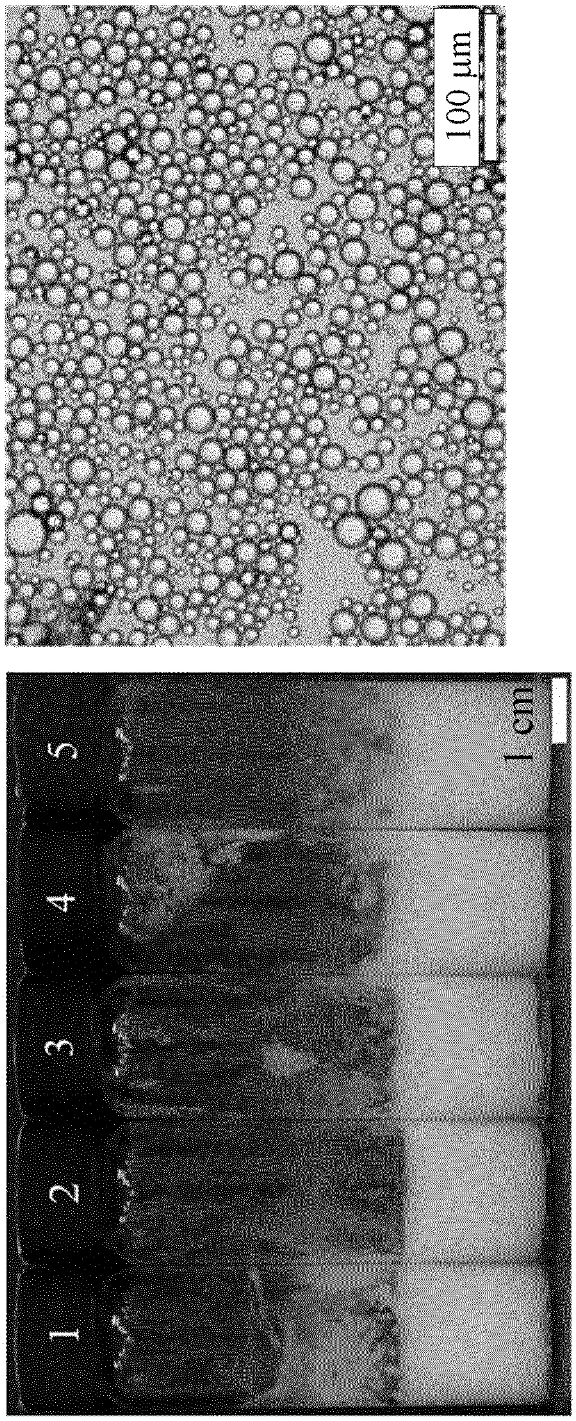

FIG. 46A

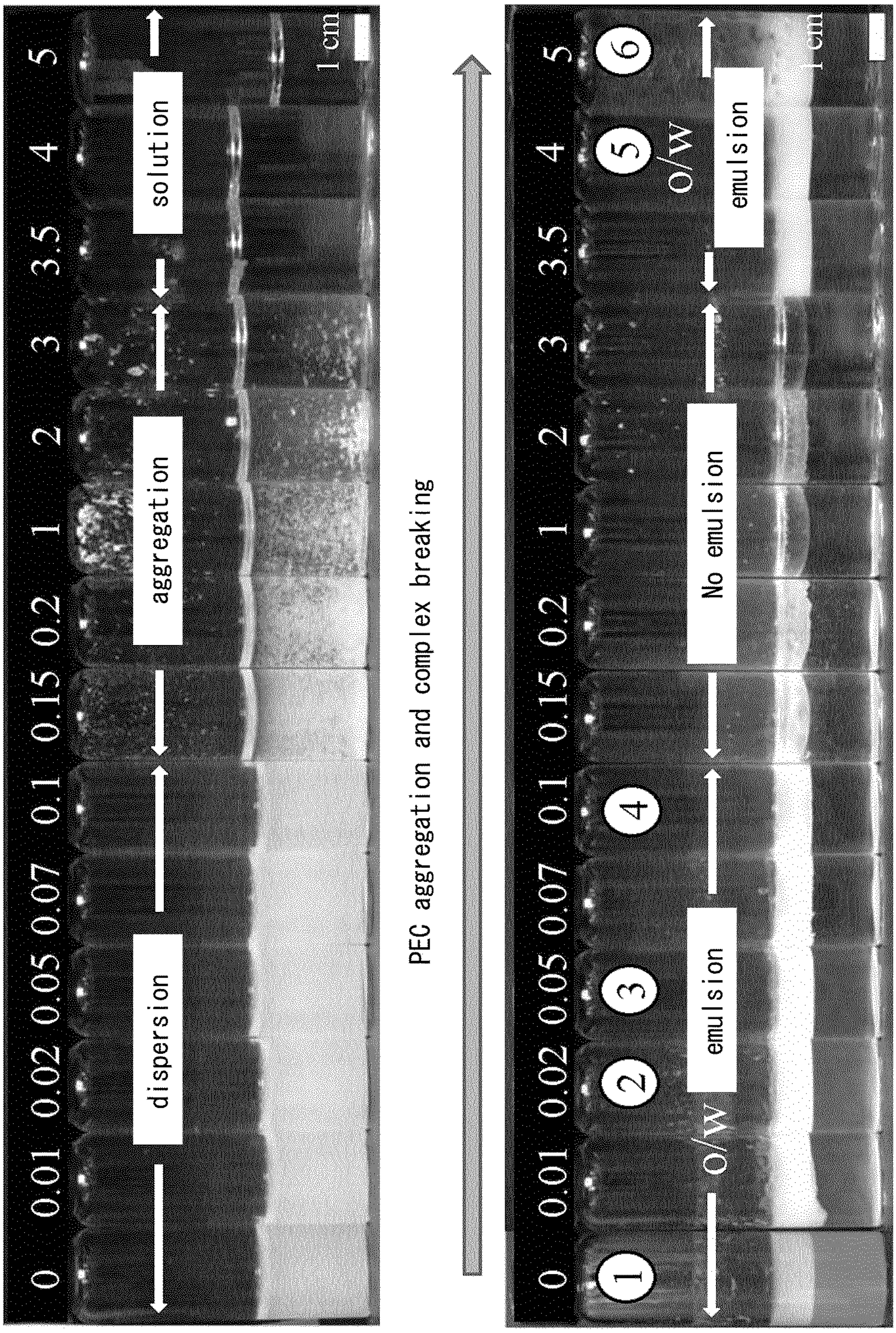
0 0.01 0.02 0.05 0.07 0.1 0.15 0.2 1 2 3 3.5 4 5
dispersion
aggregation
solution
1 cm
PEC aggregation and complex breaking
0 0.01 0.02 0.05 0.07 0.1 0.15 0.2 1 2 3 3.5 4 5
1 2 3 4 5 6
O/W
emulsion
No emulsion
O/W
emulsion
1 cm

FIG. 46B

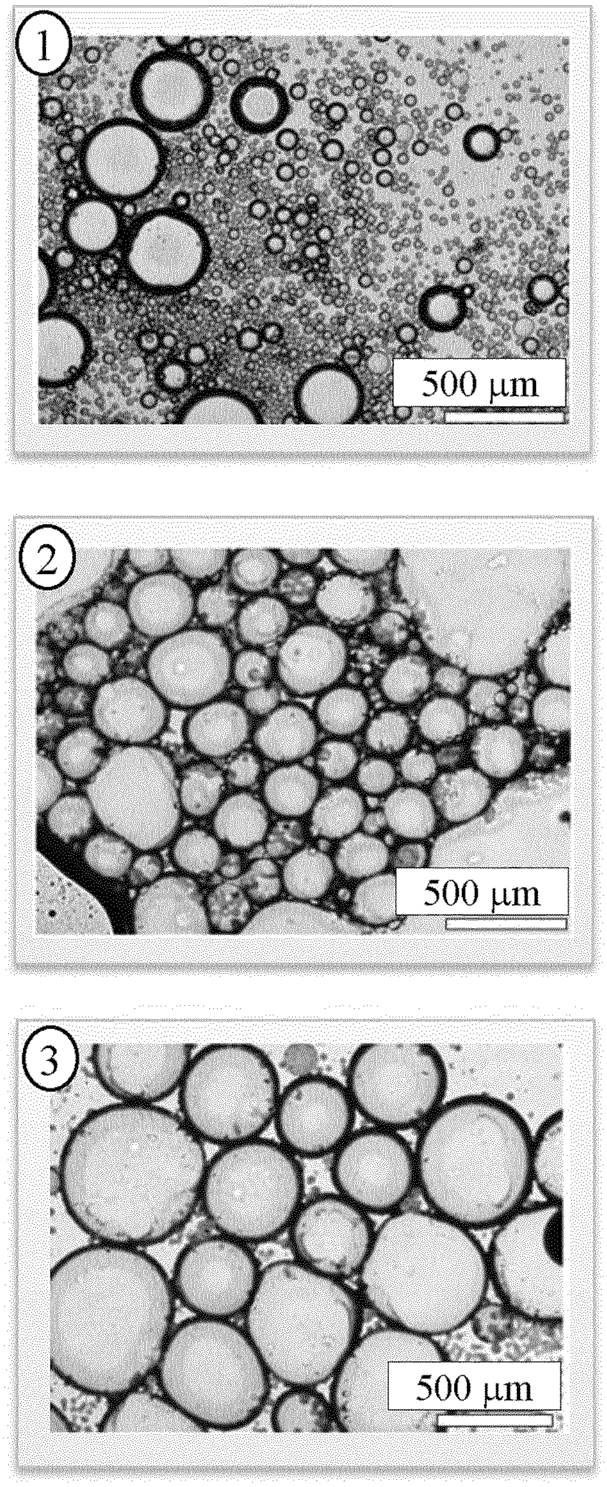

FIG. 46C

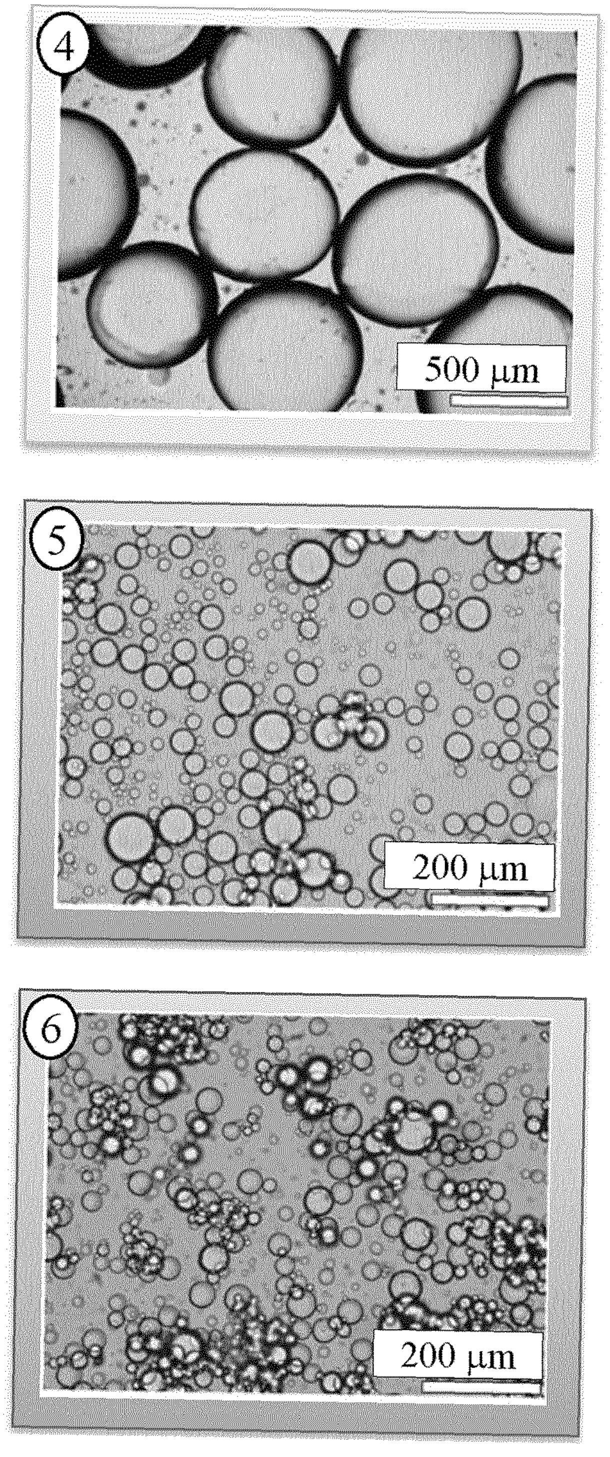

FIG. 47

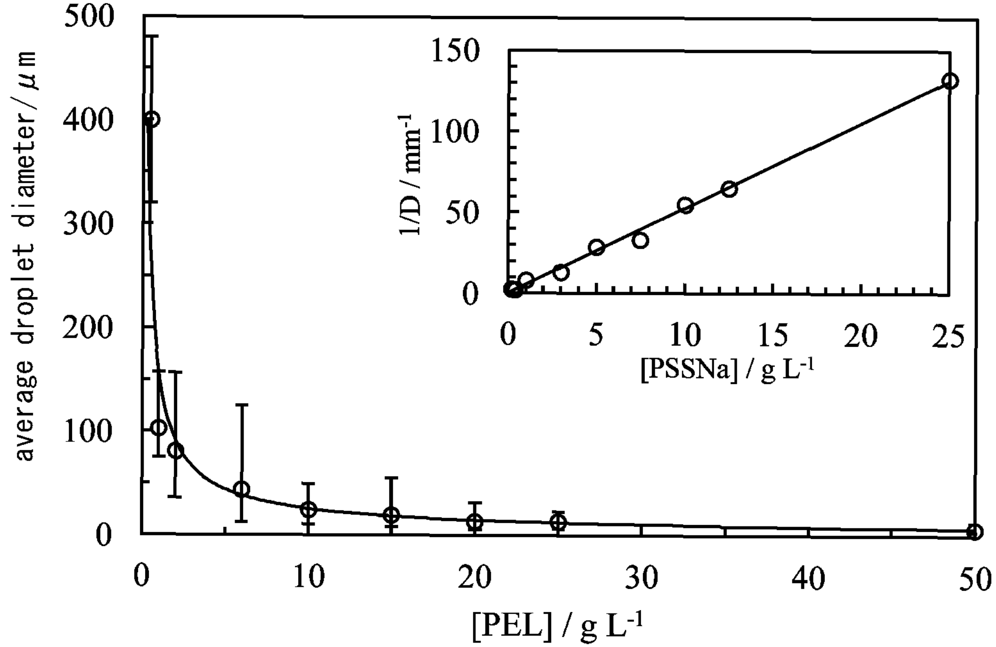


$$\frac{1}{D_{[3,2]}} = \frac{1-\phi_o}{6\phi_o\tau}c$$

$D_{[3,2]}$: Sauter mean diameter

$\phi_o$: volueme fraction of the dispersed phase

$c$: initial emulsifier concentration

$\tau$: Stabilizer adsorption density

FIG. 48

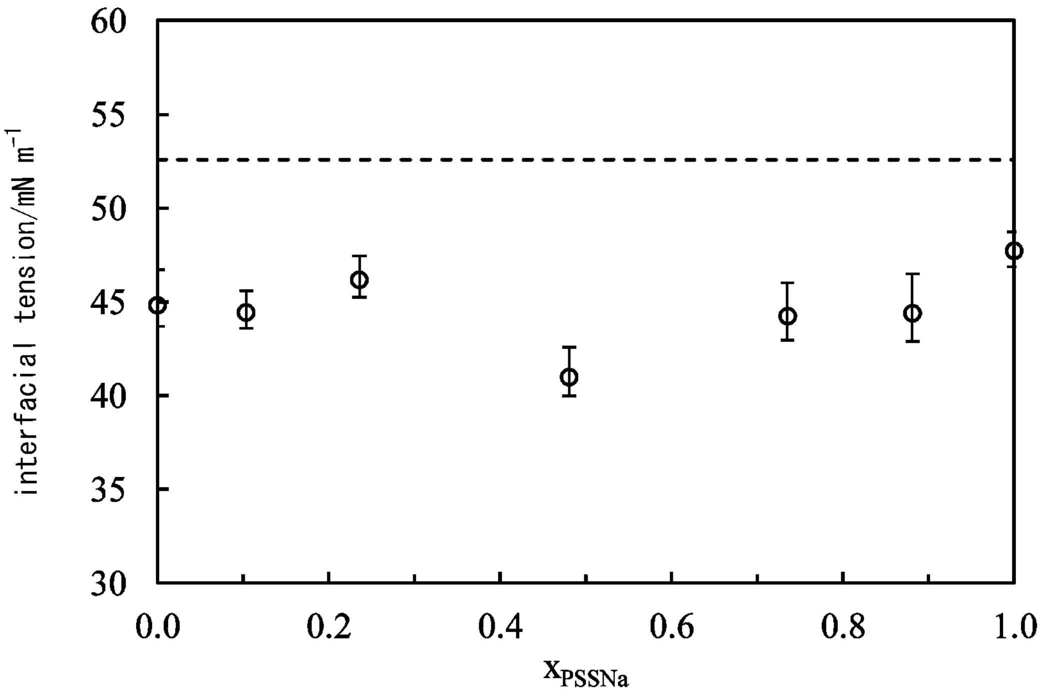
60
55
50
45
40
35
30
interfacial tension/mN m-1
0.0
0.2
0.4
0.6
0.8
1.0
xPSSNa

## INTERNATIONAL SEARCH REPORT

International application No.
PCT/JP2018/025576

A. CLASSIFICATION OF SUBJECT MATTER
Int.Cl. A61K8/06(2006.01)i, A61K8/81(2006.01)i, A61Q1/00(2006.01)i, A61Q19/00(2006.01)i

According to International Patent Classification (IPC) or to both national classification and IPC

B. FIELDS SEARCHED

Minimum documentation searched (classification system followed by classification symbols)
Int.Cl. A61K8/06, A61K8/81, A61Q1/00, A61Q19/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2016/097024 A1 (GIVAUDAN SA) 23 June 2016, claims, examples, tables 1-4, page 14, line 30 to page 17, line 7, page 25, lines 7-8 & JP 2018-500425 A & US 2017/0333302 A1 & EP 3034064 A1 & CN 106999374 A & KR 10-2017-0095222 A | 1-2, 12-15<br>3-11 |
| Y | JP 2006-505716 A (GE BAYER SILICONES GMBH & CO. KG) 16 February 2006, claims, paragraphs [0282]-[0298] & US 2006/0163524 A1 : claims, paragraphs [0256]-[0272] & WO 2004/046452 A2 & EP 1560973 A2 & CN 1735729 A | 3-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 September 2018 (27.09.2018) | 09 October 2018 (09.10.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/025576

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-529581 A (ASHLAND LICENSING AND INTELLECTUAL PROPERTY LLC) 25 October 2007 & US 2007/0203290 A1 & WO 2005/092954 A1 & EP 1727853 A1 & CN 1954021 A | 1-15 |
| A | JP 2008-543952 A (THE PROCTER & GAMBLE COMPANY) 04 December 2008 & US 2007/0010408 A1 & WO 2007/008475 A1 & EP 1901814 A1 & CN 101217998 A | 1-15 |
| A | JP 2010-522229 A (THE PROCTER & GAMBLE COMPANY) 01 July 2010 & US 2008/0317698 A1 & WO 2008/129493 A2 & EP 2134312 A2 & CN 101686902 A | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **P.A. ALBERTSSON.** Partition of cell particles and macromolecules: distribution and fractionation of cells, mitochondria, chlorophasts, viruses, proteins, nucleic acids, and antigen-antibody complexes in aqueous polymer two-phase systems. Wiley-Interscience, 1971, 18-21
- **K.R. SHARMA.** Polymer thermodynamics: Blends, copolymers and reversible polymerisation. CRC Press, 2012, 7
- **O. OLABIS ; L.M. ROBESON ; M.T. SHAW.** Polymer-polymer miscibility. Academic Press INC, 1979, 4
- **F. COMERT ; A.J. MALANOWSKI ; F. AZARIKIA ; P.L. DUBIN.** Coacervation and precipitation in polysaccharide-protein systems. *Soft Matter,* 2016, vol. 12, 4154-4161
- **X. LIU ; M. HADDOU ; I. GRILLO ; Z. MANA ; J. CHAPEL ; C. SCHATZ.** Early stage kinetics of polyelectrolyte complex coacervation monitored through stopped-flow light scattering. *Soft Matter,* 2016, vol. 12, 9030-9038
- **D. PRIFTIS ; M. TIRRELL.** Phase behaviour and complex coacervation of aqueous polypeptide solutions. *Soft Matter,* 2012, vol. 8, 9396-9405
- **F.W. TIEBACKX.** Gleichzeitige Ausflockung zweier Kolloide. *Z. Chem. Ind. Kolloide,* 1911, vol. 8, 198
- **H.G. BUNGENBERG DE JONG ; H.R. KRUYT.** Coacervation (Partial miscibility in colloid systems). *Proc. koninkl. Med. Akad. Wetershap.,* 1929, vol. 32, 849-856
- **A.I. OPARIN ; K.L. GLADILIN ; D.B. KIRPOTIN ; G.V. CHERTIBRIM ; A.F. ORLOVSKY.** *Dokl. Acad. Nauk. SSSR,* 1977, vol. 232, 485
- **E. KIZILAY ; A.B. KAYITMAZER ; P.L. DUBIN.** Complexation and coacervation of polyelectrolytes with oppositely charged colloids. *Adv. Colloid Interface Sci.,* 2011, vol. 167, 24-37
- **P.K. JHA ; P.S. DESAI ; J. LI ; R.G. LARSON.** pH and salt effects on the associative phase separation of oppositely charged polyelectrolytes. *Polymers,* 2014, vol. 6, 1414-1436
- **E. SPRUIJT ; J. SPRAKEL ; M.A. COHEN STUART ; J. VAN DER GUCHT.** Interfacial tension between a complex coacervate phase and its coexisting aqueous phase. *Soft Matter,* 2010, vol. 6, 172-178
- **D. PRIFTIS ; R. FARINA ; M. TIRRELL.** Interfacial energy of polypeptide complex coacervates measured via capillary adhesion. *Langmuir,* 2012, vol. 28, 8721-8729
- **J. QUIN ; D. PRIFTIS ; R. FARINA ; S.L. PERRY ; L. LEON ; J. WHITMER ; K. HOFFMANN ; M. TIRRELL ; J.J DE PABLO.** Interfacial tension of polyelectrolyte complex coacervate phases. *ACS Macro Lett.,* 2014, vol. 3, 565-568
- **L. DE RUITER ; H. G. BUNGENBERG DE JONG.** *Proc. KNAW,* 1947, vol. 50, 836-848
- **N. DEVI ; M. SARMAH ; B. KHATUN ; T.K. MAJI.** Encapsulation of active ingredients in polysaccharide-protein complex coacervates. *Adv. Colloid Interface Sci.*
- **A. MADENE ; M. JACQUOT ; J. SCHER ; S. DESOBRY.** Flavour encapsulation and controlled release - a review. *Int. J. Food Sci. Technol.,* 2006, vol. 41, 1-21
- **S. LIU.** MSc Dissertation, Encapsulation of flax oil by complex coacervation. University of Saskatchewan, 2009
- **S. KEIPERT ; P. MELEGARI.** Preparation and characterization of oil containing microparticles. *Drug Dev. Ind. Pharm.,* 1993, vol. 19, 603-621
- **M. EVANS ; I. RATCLIFFE ; P.A. WILLIAMS.** Emulsion stabilisation using polysaccharide-protein complexes. *Curr. Opin. Colloid Interface Sci.,* 2013, vol. 18, 272-282
- Encapsulation and controlled release technologies in food systems. Wiley Blackwell, 2007
- **S. TORZA ; S.G. MASON.** Three-phase interactions in shear and electrical fields. *J. Colloid Interface Sci.,* 1970, vol. 33 (1), 67-83
- **W.D. HARKINS.** *Z Phys. Chem.,* 1928, vol. 139, 647
- **A. LOXLEY ; B. VINCENT.** Preparation of poly(methylmethacrylate) microcapsules with liquid cores. *J. Colloid Interface Sci.,* 1998, vol. 208, 49-62
- **A.L. TASKER ; J.P. HITCHCOCK ; L. HE ; E.A. BAXTER ; S. BIGGS ; O.J. CAYRE.** The effect of surfactant chain length on the morphology of poly(methylmethacrylate) microcapsules for fragrance oil encapsulation. *J. Colloid Interface Sci.,* 2016, vol. 484, 10-16
- **G.T. BARNES ; I.R. GENTLE.** Interfacial Science: An introduction. Oxford University Press Inc, 2005, 8-15

- **C. J. VAN OSS.** Interfacial Forces in Aqueous Media. Marcel Dekker, 1994
- **C.J. VAN OSS ; R.F. GIESE.** The hydrophilicity and hydrophobicity of clay minerals. *Clays Clay Miner.,* 1995, vol. 43, 474-477
- **B.P. BINKS ; A.T. TYOWUA.** Influence of the degree of fluorination on the behaviour of silica particles at air-oil surfaces. *Soft Matter,* 2013, vol. 9, 834-845
- **B. YIN ; W. DENG ; K. XU ; L. HUANG ; P. YAO.** Stable nano-sized emulsions produced from soy protein and soy polysaccharide complexes. *J. Colloid Interface Sci.,* 2012, vol. 380, 51-59
- **A.R. PATEL ; E. DROST ; J. SEIJEN TEN HOORN ; K.P. VELIKOV.** Fabrication and characterisation of emulsions with pH responsible switchable behaviour. *Soft Matter,* 2013, vol. 9, 6747-6751
- **B.P. BINKS.** Modern Aspects of Emulsion Science. Royal Society of Chemistry, 1998, 1-48
- **I.D. MORRISON ; S. ROSS.** Colloidal Dispersions. Suspensions, Emulsions, and Foams. Wiley, 2002
- **P. BECHER.** Encyclopedia of Emulsion Technology. Marcel Dekker, 1996, vol. 4, 1-37
- **T.F. TADROS.** Emulsion Formation, Stability, and Rheology. Wiley-VCH, 2013, 1-47
- **H. DAUTZENBERG ; B. PHILIPP.** Polyelectrolytes: Formation, Application and Characterisation. Hanser Publishers, 1994, 1-45
- **P.M. VISAKH ; O. BAYRAKTAR ; G. PICO.** Polyelectrolytes. Thermodynamics and Rheology. Springer International Publishing, 2014, 1-2
- **G.S. MANNING.** Limiting laws and counterion condensation in polyelectrolyte solutions I. Colligative properties. *J. Chem. Phys.,* 1969, vol. 51, 924-933
- Polymeric Systems. John Wiley & Sons, INC, 1997, 12-14
- **R.N. GOLDBERG ; N. KISHORE ; R.M. LENNEN.** Thermodynamic quantities for the ionization reactions of buffers. *J. Phys. Chem. Ref. Data,* 2002, vol. 31, 231-370
- **J. CHOI ; M.F. RUBNER.** Influence of the degree of ionisation on weak polyelectrolyte multilayer assembly. *Macromolecules,* 2005, vol. 38, 116-124
- **S.E. BURKE ; C.J. BARRETT.** Acid-Base equlilibria of weak polyelectrolytes in multilayer thin films. *Langmuir,* 2003, vol. 19, 3297-3303
- **B.N. DICKHAUS ; R. PRIEFER.** Determination of polyelectrolyte pKa values using surface-to-air tension measurements. *Colloids Surf., A,* 2016, vol. 488, 15-19
- **J. KOETZ ; S. KOSMELLA.** Polyelectrolytes and Nanoparticles. Springer-Verlag Berlin Heidelberg, 2007, 1-4, 36-45
- **A.V. IL'INA ; V.P. VARLAMOV.** *Appl. Biochem. Micro.,* 2005, vol. 41, 5-11
- **T. ALONSO ; J. IRIGOYEN ; J.J. ITURRI ; I.L IARENA ; S.E. MOYA.** *Soft Matter,* 2013, vol. 9, 1920-1928
- Modern Aspects of Emulsion Science. Royal Society of Chemistry, 1998, 1-48
- **A.M. BAGO RODRIGUEZ ; B.P. BINKS ; T. SEKINE.** Novel stabilisation of emulsions by soft particles: polyelectrolyte complexes. *Faraday Discuss.,* 2016, vol. 191, 255-285
- **D.E. KOPPEL.** *J. Chem. Phys.,* 1972, vol. 57, 4814-4820
- **M. SMOLUCHOWSKI.** *Handbuch der Electrizitat und des Magnetismus,* 1921, vol. 2, 366
- **W.D. HARKINS ; H.F. JORDAN.** *J. Am. Chem. Soc.,* 1930, vol. 52, 1751-1772
- **L. VITORAZI ; N. OULD-MOUSSA ; S. SEKAR ; J. FRESNAIS ; W. LOH ; J.-P. CHAPEL ; J.-H. BERRET.** Evidence of two-step process and pathway dependency in the thermodynamics of poly(diallyldimethylammonium chloride)/poly(sodium acrylate) complexation. *Soft Matter,* 2014, vol. 10, 9496-9505
- **E.A. LITMANOVICH ; E.V. CHERNIKOVA ; G.V. STOYCHEV ; S.O. ZAKHARCHENKO.** Unusual behaviour of the mixture of poly(acrylic acid) and poly(diallyldimethylammonium chloride) in acidic media. *Macromolecules,* 2010, vol. 43, 6871-6876
- **T. ALONSO ; J. IRIGOYEN ; J.J. ITURRI ; I.L. IARENA ; S.E. MOYA.** Study of the multilayer assembly and complex formation of poly(diallyldimethylammonium chloride) (PDADMAC) and poly(acrylic acid) (PAA) as a function of pH. *Soft Matter,* 2013, vol. 9, 1920-1928
- **J. KOETZ ; S. KOSMELLA.** Interactions between poly(diallyldimethylammonium chloride) and poly(acrylic acid) in dependence on polymer concentration, presented in part at the. *International Conference on Scaling concepts and complex fluids,* 1994
- **Y. ISHIMURO ; K. UEBERREITER.** The surface tension of poly(acrylic acid) in aqueous solution. *Colloid Polym. Sci.,* 1980, vol. 258, 928-931
- **A.S. PRATA ; C.R.F. GROSSO.** Influence of the oil phase on the microencapsulation by complex coacervation. *J. Am. Oil Chem. Soc.,* 2015, vol. 92, 1063-1072
- **A. GOEBEL ; K. LUNKENHEIMER.** Interfacial tension of the water/n-alkane interface. *Langmuir,* 1997, vol. 13, 369-372
- **N.R. PALLAS ; Y. HARRISON.** An automated drop shape apparatus and the surface tension of pure water. *Colloids and surfaces,* 1990, vol. 43, 169-194
- **A. MULERO ; I. CACHADINA ; M.I. PARRA.** Recommended correlations for the surface tensions of common fluids. *J. Phys. Chem. Ref. Data,* 2012, vol. 41, 043105

- **J.H. CLINT.** Adhesion and components of solid surface energies. *Curr. Opin. Colloid Interface Sci.,* 2001, vol. 6, 28-33
- **B.P. BINKS ; T. SEKINE ; A.T. TYOWUA.** Dry oil powders and oil foams stabilised by fluorinated clay platelet particles. *Soft Matter,* 2014, vol. 10, 578-589
- **J.H. CLINT ; A.C. WICKS.** Adhesion under water: surface energy considerations. *Int. J. Adhes. Adhes.,* 2001, vol. 21, 267-273
- **B. P. BINKS ; J. H. CLINT.** Solid wettability from surface energy components: Relevance to pickering emulsions. *Langmuir,* 2002, vol. 18, 1270-1273